# EUROPÄISCHE PATENTANMELDUNG

(11) **EP 0 673 930 A1**
(43) Veröffentlichungstag der Anmeldung: **27.09.1995**
(21) Anmeldenummer: 95103405.7
(22) Anmeldetag: 09.03.1995
(51) Int. Cl.: C07D 213/82, A61K 31/40

(54) **Substituierte heterocyclische Carbonsäureamidester, ihre Herstellung und ihre Verwendung als Arzneimittel**

(30) Priorität: 25.03.1994 DE 4410453
(71) Anmelder: HOECHST AKTIENGESELLSCHAFT, D-65929 Frankfurt am Main (DE)
(72) Erfinder: Weidmann, Klaus, Dr., D-61476 Kronberg (DE); Baringhaus, Karl-Heinz, Dr., D-61200 Wölfersheim (DE); Tschank, Georg, Dr., D-55270 Klein-Winternheim (DE); Bickel, Martin, Dr., D-61348 Bad Homburg (DE)

(57) **Zusammenfassung**

Die Erfindung betrifft substituierte heterocyclische Carbonsäureamidester gemäß der allgemeinen Formel 1 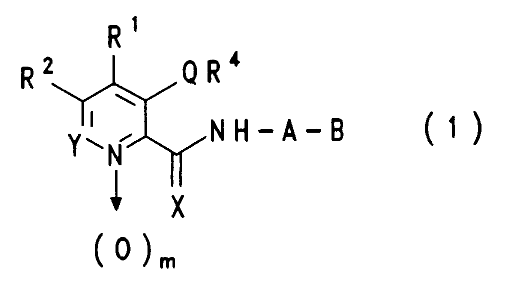
Die genannten Verbindungen finden ihre Anwendung als Arzneimittel gegen fibrotische Erkrankungen und als Inhibitoren der Prolylhydroxylase und der Kollagenbiosynthese.

## Beschreibung

Die Erfindung betrifft substituierte heterocyclische Carbonsäureamidester, ihre Herstellung und ihre Verwendung als Inhibitoren der Kollagenbiosynthese und ihre Verwendung als Arzneimittel zur Behandlung von fibrotischen Erkrankungen.

Verbindungen, die die Enzyme Prolin- und Lysinhydroxylase inhibieren, bewirken eine sehr selektive Hemmung der Kollagenbiosynthese durch Beeinflussung der kollagenspezifischen Hydroxylierungsreaktionen. In deren Verlauf wird proteingebundenes Prolin oder Lysin durch die Enzyme Prolin- bzw. Lysinhydroxylase hydroxyliert. Wird diese Reaktion durch Inhibitoren unterbunden, so entsteht ein nicht funktionsfähiges, unterhydroxyliertes Kollagenmolekül, das von den Zellen nur in geringer Menge in den extrazellulären Raum abgegeben werden kann. Das unterhydroxylierte Kollagen kann außerdem nicht in die Kollagenmatrix eingebaut werden und wird sehr leicht proteolytisch abgebaut. Als Folge dieser Effekte verringert sich insgesamt die Menge des extrazellulär abgelagerten Kollagens.

Inhibitoren der Prolylhydroxylase sind deshalb geeignete Substanzen in der Therapie von Erkrankungen, in denen die Ablagerung von Kollagenen maßgeblich zum Krankheitsbild beiträgt. Hierzu gehören u. a. Fibrosen der Lunge, Leber und Haut (Skleroderma) sowie die Atherosklerose.

Es ist bekannt, daß das Enzym Prolinhydroxylase durch Pyridin-2,4- und - 2,5-dicarbonsäure gehemmt wird (K. Majamaa et al., Eur. J. Biochem. 138 (1984) 239-245). Diese Verbindungen sind in der Zellkultur allerdings nur in sehr hohen Konzentrationen als Hemmstoffe wirksam (Tschank, G. et al., Biochem. J. 238 (1987) 625-633).
Auch Prodrugs der Pyridin-2,4(5)-dicarboxylate sind bekannt. Diese sind in den älteren deutschen Anmeldungen P 42 33 124.2, P 42 38 506.7 und P 42 09 424.0 beschrieben.

N-Oxalylglycine als Inhibitoren der Prolyl-4-hydroxylase sind aus J.Med.Chem. 1992, 35, 2652-2658 (Cunliffe et al.), und EP-A-0 457 163 (Baader et al.) bekannt.

Hydroxyisochinoline- und Hydroxycinnolincarbonsäureglycylamide sind aus Biochem. Soc. Trans. 1991, 19, 812-815 (Franklin et al.) bekannt.

Überraschenderweise wurde nun gefunden, daß heterocyclische Carbonsäureamidester mit einem Ether-, einem Thioether- oder einem Amino-Substituenten in ortho-Position und einer aciden Gruppierung in para-Position zur Amidfunktion in vivo stark wirksame Inhibitoren der Kollagenbiosynthese sind.

Die Verbindungen sind Ester-Prodrugs der entsprechenden Carbonsäuren der Formel 1, in denen B eine Carboxylgruppe bedeutet, wie sie in der gleichzeitig eingereichten Anmeldung HOE 94/F 074 beschrieben sind.

Die Verbindungen der Formel 1 werden im lebenden Organismus (in vivo) und in Zellkulturen (in vitro) zu Verbindungen der Formel 1, in denen B eine Carboxylgruppe oder deren Salze bedeutet, gespalten.

Die erfindungsgemäßen Substanzen der Formel 1 führen in vivo zu einer Inhibierung der Kollagenbiosynthese.

Nach der Applikation der Verbindungen der Formel 1 bewirken sie die in vivo und in vitro zu beobachtende Hemmung der Kollagenbiosynthese dadurch, daß sie Verbindungen der Formel 1, in denen B eine Carboxylgruppe oder deren Salze bedeutet, freisetzen. Diese Verbindungen inhibieren die Prolyl-4-hydroxylase und führen deshalb zu einer Hemmung der Kollagenbiosynthese.

Die erfindungsgemäßen Verbindungen entsprechen der allgemeinen Formel 1 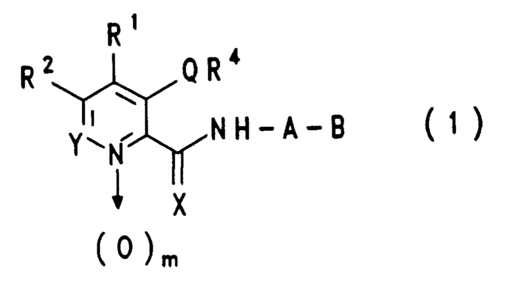
in welcher
- Q: O, S oder NR^{Y},
- X: O oder S,
- Y: C-R³ oder N,
- m: 0 und 1,
- A: (C₁-C₄)-Alkylen, das gegebenenfalls substituiert ist mit einem oder zwei Substituenten aus der Reihe Halogen, insbesondere Fluor, Chlor, Brom, Cyano, Nitro, Trifluormethyl, (C₁-C₆)-Alkyl, (C₁-C₆)-Hydroxyalkyl, (C₁-C₆)-Alkoxy, -O-[CH₂]ₓ-C_{f}H_{(2f+1-g)}Hal_{g}, vorzugsweise (C₁-C₈)-Fluoralkoxy, (C₁-C₈)-Fluoralkenyloxy, (C₁-C₈)-Fluoralkinyloxy, -OCF₂Cl oder -O-CF₂-CHFCl, (C₁-C₆)-Alkylmercapto, (C₁-C₆)-Alkylsulfinyl, (C₁-C₆)-Alkylsulfonyl, (C₁-C₆)-Alkylcarbonyl, (C₁-C₆)-Alkoxycarbonyl, Carbamoyl, N-(C₁-C₄)-Alkylcarbamoyl, N,N-Di-(C₁-C₄)-alkylcarbamoyl, (C₁-C₆)-Alkylcarbonyloxy, (C₃-C₈)-Cycloalkyl, Phenyl, Benzyl, Phenoxy, Benzyloxy, Anilino, N-Methylanilino, Phenylmercapto, Phenylsulfonyl, Phenylsulfinyl, Sulfamoyl, N-(C₁-C₄)-Alkylsulfamoyl, N,N-Di-(C₁-C₄)-alkylsulfamoyl oder
mit einem substituierten (C₆-C₁₂)-Aryloxy-, (C₇-C₁₁)-Aralkyloxy, (C₆-C₁₂)-Aryl- oder (C₇-C₁₁)-Aralkyl-Rest, der im Arylteil 1, 2, 3, 4 oder 5 gleiche oder verschiedene Substituenten aus der Reihe Halogen, Cyano, Nitro, Trifluormethyl, (C₁-C₆)-Alkyl, (C₁-C₆)-Alkoxy,
-O-[CH₂]ₓ-C_{f}H_{(2f+1-g)}Hal_{g}, -OCF₂Cl,-O-CF₂-CHFCl, (C₁-C₆)-Alkylmercapto, (C₁-C₆)-Alkylsulfinyl, (C₁-C₆)-Alkylsulfonyl, (C₁-C₆)-Alkylcarbonyl, (C₁-C₆)-Alkoxycarbonyl, Carbamoyl, N-(C₁-C₄)-Alkylcarbamoyl, N,N-Di-(C₁-C₄)-alkylcarbamoyl, (C₁-C₆)-Alkylcarbonyloxy, (C₃-C₈)-Cycloalkyl, Sulfamoyl, N-(C₁-C₄)-Alkylsulfamoyl oder N,N-Di-(C₁-C₄)-alkylsulfamoyl trägt, oder
mit einem Substituenten R^{x} des α-C-Atoms einer α-Aminosäure, zu denen die natürlichen L-Aminosäuren und ihre D-Isomeren zählen;
- B: -CO₂V bedeutet, wobei
- V: den Rest eines Alkohols VOH bedeutet, worin V
(C₁-C₁₀)-Acyloxy-(C₁-C₆)-alkyl, vorzugsweise (C₁-C₁₀)-Alkanoyloxy-(C₁-C₆)-alkyl, Benzyloxy-(C₁-C₆)-alkyl, Benzyloxycarbonyloxy-(C₁-C₆)-alkyl oder Alkoxycarbonyloxy-(C₁-C₆)-alkyl), oder
einen verzweigten, unverzweigten oder cyclischen aliphatischen (C₁-C₂₀)-Alkyl-Rest, oder
einen verzweigten, unverzweigten oder cyclischen (C₂-C₂₀)-Alkenyl-Rest, einen Retinyl-Rest, einen (C₂-C₁₆)-Alkinyl-Rest oder einen entsprechenden (C₄-C₁₆)-Alkeninyl-Rest bedeutet, wobei die Reste jeweils eine oder mehrere Mehrfachbindungen enthalten können, oder
einen (C₆-C₁₆)-Aryl-Rest, einen (C₇-C₁₆)-Aralkyl-Rest oder einen 5-oder 6-gliedrigen, vorzugsweise Stickstoff-enthaltenden Heteroaryl-oder einen 5- oder 6-gliedrigen, vorzugsweise Stickstoff-enthaltenden Heteroaralkyl-Rest bedeutet, wobei die vorstehenden Reste insbesondere einen oder mehrere Substituenten aus der Reihe
Hydroxy, Halogen, Cyano, Trifluormethyl, Nitro, Carboxy, (C₁-C₁₂)-Alkyl, (C₃-C₈)-Cycloalkyl, (C₃-C₈)-Cycloalkyl-(C₁-C₁₂)-alkyl, (C₃-C₈)-Cycloalkoxy, (C₃-C₈)-Cycloalkyl-(C₁-C₁₂)-alkoxy, (C₃-C₈)-Cycloalkyloxy-(C₁-C₁₂)-alkyl, (C₃-C₈)-Cycloalkyloxy-(C₁-C₁₂)-alkoxy, (C₃-C₈)-Cycloalkyl-(C₁-C₈)-alkyl-(C₁-C₆)-alkoxy, (C₃-C₈)-Cycloalkyl-(C₁-C₈)-alkoxy-(C₁-C₆)-alkyl, (C₃-C₈)-Cycloalkyloxy-(C₁-C₈)-alkoxy-(C₁-C₆)-alkyl, (C₃-C₈)-Cycloalkoxy-(C₁-C₈)-alkoxy-(C₁-C₈)-alkoxy, (C₆-C₁₂)-Aryl, (C₇-C₁₆)-Aralkyl, (C₂-C₁₂)-Alkenyl, (C₂-C₁₂)-Alkinyl, (C₁-C₁₂)-Alkoxy, (C₁-C₁₂)-Alkoxy-(C₁-C₁₂)alkyl, (C₁-C₁₂)-Alkoxy-(C₁-C₁₂)alkoxy, (C₁-C₁₂)-Alkoxy-(C₁-C₈)-alkoxy-(C₁-C₈)-alkyl, (C₆-C₁₂)-Aryloxy, (C₇-C₁₆)-Aralkyloxy, (C₆-C₁₂)-Aryloxy-(C₁-C₆)-alkoxy, (C₇-C₁₆)-Aralkoxy-(C₁-C₆)-alkoxy, (C₁-C₈)-Hydroxyalkyl, (C₆-C₁₆)-Aryloxy-(C₁-C₈)-alkyl, (C₇-C₁₆)-Aralkoxy-(C₁-C₈)-alkyl, (C₆-C₁₂)-Aryloxy-(C₁-C₈)-alkoxy-(C₁-C₆)-alkyl, (C₇-C₁₂)-Aralkyloxy-(C₁-C₈)-alkoxy-(C₁-C₆)-alkyl, -O-[CH₂₋]ₓC_{f}H_{(2f+1-g)}F_{g}, -OCF₂Cl, -OCF₂-CHFCl,
(C₁-C₁₂)-Alkylcarbonyl, (C₃-C₈)-Cycloalkylcarbonyl, (C₆-C₁₂)-Arylcarbonyl, (C₇-C₁₆)-Aralkylcarbonyl, Cinnamoyl, (C₂-C₁₂) Alkenylcarbonyl, (C₂-C₁₂)-Alkinylcarbonyl,
(C₁-C₁₂)-Alkoxycarbonyl, (C₁-C₁₂)-Alkoxy-(C₁-C₁₂)-alkoxycarbonyl, (C₆-C₁₂)- Aryloxycarbonyl, (C₇-C₁₆)-Aralkoxycarbonyl, (C₃-C₁₀)-Cycloalkoxycarbonyl, (C₂-C₁₂)-Alkenyloxycarbonyl, (C₂-C₁₂)-Alkinyloxycarbonyl,
(C₁-C₁₂)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy, (C₂-C₁₂)-Alkenylcarbonyloxy, (C₂-C₁₂)-Alkinylcarbonyloxy,
(C₁-C₁₂)-Alkoxycarbonyloxy, (C₁-C₁₂)-Alkoxy-(C₁-C₁₂)-alkoxycarbonyloxy, (C₆-C₁₂)-Aryloxycarbonyloxy, (C₇-C₁₆)-Aralkyloxycarbonyloxy, (C₃-C₈)-Cycloalkoxycarbonyloxy, (C₂-C₁₂)-Alkenyloxycarbonyloxy, (C₂-C₁₂)-Alkinyloxycarbonyloxy,
Carbamoyl, N-(C₁-C₁₂)-Alkylcarbamoyl, N, N-Di-(C₁-C₁₂)-alkylcarbamoyl, N-(C₃-C₈)-Cycloalkylcarbamoyl, N,N-Dicyclo-(C₃-C₈)-alkylcarbamoyl, N-(C₁-C₁₀)-Alkyl-N-(C₃-C₈)-Cycloalkylcarbamoyl, N-((C₃-C₈)-Cycloalkyl-(C₁-C₆)-alkyl)carbamoyl, N-(C₁-C₆)-Alkyl-N-((C₃-C₈)-cycloalkyl-(C₁-C₆)-alkyl)carbamoyl, N-(+)-Dehydroabietylcarbamoyl, N-(C₁-C₆)-Alkyl-N-(+)-dehydroabietylcarbamoyl, N-(C₆-C₁₂)-Arylcarbamoyl, N-(C₇-C₁₆)-Aralkylcarbamoyl, N-(C₁-C₁₀)-Alkyl-N-(C₆-C₁₆)-arylcarbamoyl, N-(C₁-C₁₀)-Alkyl-N-(C₇-C₁₆)-aralkylcarbamoyl, N-((C₁-C₁₀)-Alkoxy-(C₁-C₁₀)alkyl)carbamoyl, N-((C₆-C₁₆)-Aryloxy-(C₁-C₁₀)alkyl)carbamoyl, N-((C₇-C₁₆)-Aralkyloxy-(C₁-C₁₀)-alkyl)carbamoyl, N-(C₁-C₁₀)-Alkyl-N-((C₁-C₁₀)-alkoxy-(C₁-C₁₀)-alkyl)carbamoyl, N-(C₁-C₁₀)-Alkyl-N-((C₆-C₁₂)-aryloxy-(C₁-C₁₀)-alkyl)carbamoyl, N-(C₁-C₁₀)-Alkyl-N-((C₇-C₁₆)-aralkyloxy-(C₁-C₁₀)-alkyl)carbamoyl, CON(CH₂)ₕ, worin eine CH₂-Gruppe durch O, S, N-(C₁-C₈)-Alkylimino, N-(C₃-C₈)-Cycloalkylimino, N-(C₃-C₈)-Cycloalkyl-(C₁-C₄)-alkylimino, N-(C₆-C₁₂)-Arylimino, N-(C₇-C₁₆)-Aralkylimino oder N-(C₁-C₄)-Alkoxy-(C₁-C₆)-alkylimino ersetzt ein kann und h 3 bis 6 bedeutet, oder mit
einem Carbamoyl-Rest der allgemeinen Formel II 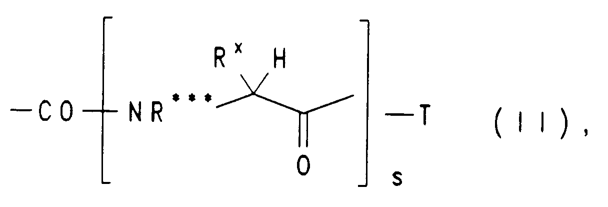 worin
- R^{x}: der Substituent einer α-Aminosäure bedeutet, zu denen die L-und D-Aminosäuren zählen,
- s: 1, 2, 3, 4 oder 5 und
- T: OH, OR oder NR*R** bedeutet, wobei
- R***: Wasserstoff oder (C₁-C₄)-Alkyl,
- R* und R**: gleich oder verschieden sind und Wasserstoff, (C₆-C₁₂)-Aryl, (C₇-C₁₁)-Aralkyl, (C₁-C₈)-Alkyl, (C₃-C₈)-Cycloalkyl, (+)-Dehydroabietyl, (C₁-C₈)-Alkoxy-(C₁-C₈)alkyl, (C₇-C₁₂)-Aralkoxy-(C₁-C₈)alkyl, (C₆-C₁₂)-Aryloxy-(C₁-C₈)-alkyl, (C₁-C₁₀)-Alkanoyl, ggf. substituiertes (C₇-C₁₆)-Aralkanoyl, ggf. substituiertes (C₆-C₁₂)-Aroyl bedeuten, oder
- R* und R**: gemeinsam für -[CH₂]ₕ stehen, worin eine CH₂ Gruppe durch O, S, SO, SO₂, N-Acylimino, N-(C₁-C₁₀)-Alkoxycarbonylimino, N-(C₁-C₈)-Alkylimino, N-(C₃-C₈)-Cycloalkylimino, N-(C₃-C₈)-Cycloalkyl-(C₁-C₄)alkylimino, N-(C₆-C₁₂)-Arylimino, N-(C₇-C₁₆)-Aralkylimino oder N-(C₁-C₄)-Alkoxy-(C₁-C₆)-alkylimino ersetzt sein kann und h 3 bis 7 bedeutet, oder mit Carbamoyloxy, N-(C₁-C₁₂)-Alkylcarbamoyloxy, N,N-Di-(C₁-C₁₂)-alkylcarbamoyloxy, N-(C₃-C₈)-Cycloalkylcarbamoyloxy, N-(C₆-C₁₂)-Arylcarbamoyloxy, N-(C₇-C₁₆)-Aralkylcarbamoyloxy, N-(C₁-C₁₀)-Alkyl-N-(C₆-C₁₂)-arylcarbamoyloxy, N-(C₁-C₁₀)-Alkyl-N-(C₇-C₁₆)-Aralkylcarbamoyloxy, N-((C₁-C₁₀)-alkyl))carbamoyloxy, N-((C₆-C₁₂)-Aryloxy-(C₁-C₁₀)-alkyl)carbamoyloxy, N-((C₇-C₁₆)-Aralkyloxy-(C₁-C₁₀)-alkyl)carbamoyloxy, N-(C₁-C₁₀)-Alkyl-N-((C₁-C₁₀)-alkoxy-(C₁-C₁₀)-alkyl)carbamoyloxy, N-(C₁-C₁₀)-Alkyl-N-((C₆-C₁₂)-aryloxy-(C₁-C₁₀)-alkyl)carbamoyloxy, N-(C₁-C₁₀)-Alkyl-N-((C₇-C₁₆)-aralkyloxy-(C₁-C₁₀)-alkyl)carbamoyloxy,
Amino, (C₁-C₁₂)-Alkylamino, Di-(C₁-C₁₂)alkylamino, (C₃-C₈)-Cycloalkylamino, (C₃-C₁₂)-Alkenylamino, (C₃-C₁₂) Alkinylamino, N-(C₆-C₁₂)-Arylamino, N-(C₇-C₁₁)-Aralkylamino, N-Alkyl-Aralkylamino, N-Alkyl-Arylamino, (C₁-C₁₂)-Alkoxyamino, (C₁-C₁₂)-Alkoxy-N-(C₁-C₁₀)-alkylamino,
(C₁-C₁₂)-Alkanoylamino, (C₃-C₈)-Cycloalkanoylamino, (C₆-C₁₂)-Aroylamino, (C₇-C₁₆)-Aralkanoylamino, (C₁-C₁₂)-Alkanoyl-N-(C₁-C₁₀)-alkylamino, (C₃-C₈)-Cycloalkanoyl-N-(C₁-C₁₀)-alkylamino, (C₆-C₁₂)-Aroyl-N-(C₁-C₁₀)alkylamino, (C₇-C₁₁)-Aralkanoyl-N-(C₁-C₁₀)-alkylamino,
(C₁-C₁₂)-Alkanoylamino-(C₁-C₈)-alkyl, (C₃-C₈)-Cycloalkanoylamino-(C₁-C₈)alkyl, (C₆-C₁₂)-Aroylamino-(C₁-C₈)-alkyl, (C₇-C₁₆)-Aralkanoylamino-(C₁-C₈)-alkyl, Amino-(C₁-C₁₀)-alkyl, N-(C₁-C₁₀)-alkylamino-(C₁-C₁₀)alkyl, N,N-Di(C₁-C₁₀)alkylamino-(C₁-C₁₀)alkyl, (C₃-C₈)-Cycloalkylamino-(C₁-C₁₀)-alkyl, (C₁-C₁₂)-Alkylmercapto, (C₁-C₁₂)-Alkylsulfinyl, (C₁-C₁₂)-Alkylsulfonyl, (C₆-C₁₂)-Arylmercapto, (C₆-C₁₂)-Arylsulfinyl,
(C₆-C₁₂)-Arylsulfonyl, (C₇-C₁₆)-Aralkylmercapto, (C₇-C₁₆)-Aralkylsulfinyl, (C₇-C₁₆)-Aralkylsulfonyl,
Sulfamoyl, N-(C₁-C₁₀)-Alkylsulfamoyl, N,N-Di-(C₁-C₁₀)-alkylsulfamoyl, (C₃-C₈)-Cycloalkylsulfamoyl, N-(C₆-(C₁₂)-Arylsulfamoyl, N-(C₇-C₁₆)-Aralkylsulfamoyl, N-(C₁-C₁₀)-Alkyl-N-(C₆-C₁₂)-arylsulfamoyl, N-(C₁-C₁₀)-Alkyl-N-(C₇-C₁₆)-aralkylsulfamoyl, (C₁-C₁₀)-Alkyl-sulfonamido, N-((C₁-C₁₀)-alkyl)-(C₁-C₁₀)-alkylsulfonamido, (C₇-C₁₆)-Aralkylsulfonamido, oder mit N-((C₁-C₁₀)-alkyl-(C₇-C₁₆)-aralkylsulfonamido substituiert sind, wobei die Reste, die einen Arylrest enthalten ihrerseits am Aryl substituiert sein können durch 1 bis 5 gleiche oder verschiedene Reste aus der Reihe:
Hydroxy, Halogen, Cyano, Trifluormethyl, Nitro, Carboxy, (C₁-C₁₂)-Alkyl, (C₃-C₈)-Cycloalkyl, (C₆-C₁₂)-Aryl, (C₇-C₁₆)-Aralkyl, (C₁-C₁₂)-Alkoxy, (C₁-C₁₂)-Alkoxy-(C₁-C₁₂)alkyl, (C₁-C₁₂)-Alkoxy-(C₁-C₁₂)alkoxy, (C₆-C₁₂)-Aryloxy, (C₇-C₁₆)-Aralkyloxy, (C₁-C₈)-Hydroxyalkyl,
(C₁-C₁₂)-Alkylcarbonyl, (C₃-C₈)-Cycloalkylcarbonyl, (C₆-C₁₂)-Arylcarbonyl, (C₇-C₁₆)-Aralkylcarbonyl,
(C₁-C₁₂)-Alkoxycarbonyl, (C₁-C₁₂)-Alkoxy-(C₁-C₁₂)-alkoxycarbonyl, (C₆-C₁₂)- Aryloxycarbonyl, (C₇-C₁₆)-Aralkoxycarbonyl, (C₃-C₈)-Cycloalkoxycarbonyl, (C₂-C₁₂)-Alkenyloxycarbonyl, (C₂-C₁₂)-Alkinyloxycarbonyl,
(C₁-C₁₂)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy, (C₂-C₁₂)-Alkenylcarbonyloxy, (C₂-C₁₂)-Alkinylcarbonyloxy,
(C₁-C₁₂)-Alkoxycarbonyloxy, (C₁-C₁₂)-Alkoxy-(C₁-C₁₂)-alkoxycarbonyloxy, (C₆-C₁₂)-Aryloxycarbonyloxy, (C₇-C₁₆)-Aralkyloxycarbonyloxy, (C₃-C₈)-Cycloalkoxycarbonyloxy, (C₂-C₁₂)-Alkenyloxycarbonyloxy, (C₂-C₁₂)-Alkinyloxycarbonyloxy,
Carbamoyl, N-(C₁-C₁₂)-Alkylcarbamoyl, N, N-Di-(C₁-C₁₂)-alkylcarbamoyl, N-(C₃-C₈)-Cycloalkylcarbamoyl, N,N-Dicyclo-(C₃-C₈)-alkylcarbamoyl, N-(C₁-C₁₀)-Alkyl-N-(C₃-C₈)-cycloalkylcarbamoyl, N-((C₃-C₈)-Cycloalkyl-(C₁-C₆)-alkyl)carbamoyl, N-(C₁-C₆)-Alkyl-N-((C₃-C₈)-cycloalkyl-(C₁-C₆)-alkyl)carbamoyl, N-(+)-Dehydroabietylcarbamoyl, N-(C₁-C₆)-Alkyl-N-(+)-dehydroabietylcarbamoyl, N-(C₆-C₁₂)-Arylcarbamoyl, N-(C₇-C₁₆)-Aralkylcarbamoyl, N-(C₁-C₁₀)-Alkyl-N-(C₆-C₁₆)-arylcarbamoyl, N-(C₁-C₁₀)-Alkyl-N-(C₇-C₁₆)-aralkylcarbamoyl, N-((C₁-C₁₀)-Alkoxy-(C₁-C₁₀)-alkyl)carbamoyl, N-((C₆-C₁₆)-Aryloxy-(C₁-C₁₀)-alkyl)carbamoyl, N-((C₇-C₁₆)-Aralkyloxy-(C₁-C₁₀)-alkyl)carbamoyl, N-(C₁-C₁₀)-Alkyl-N-((C₁-C₁₀)-alkoxy-(C₁-C₁₀)-alkyl)carbamoyl, N-(C₁-C₁₀)-Alkyl-N-((C₆-C₁₂)-aryloxy-(C₁-C₁₀)-alkyl)carbamoyl, N-(C₁-C₁₀)-Alkyl-N-((C₇-C₁₆)-aralkyloxy-(C₁-C₁₀)-alkyl)carbamoyl, CON(CH₂)ₕ worin eine CH₂-Gruppe durch O, S, N-(C₁-C₈)Alkylimino, N-(C₃-C₈)-Cycloalkylimino, N-(C₃-C₈)-Cycloalkyl-(C₁-C₂)alkylimino, N-(C₆-C₁₂)-Arylimino, N-(C₇-C₁₆)-Aralkylimino oder N-(C₁-C₄)-Alkoxy-(C₁-C₆)-alkylimino ersetzt ein kann und h 3 bis 7 bedeutet,
Carbamoyloxy, N-(C₁-C₁₂)-Alkylcarbamoyloxy, N,N-Di-(C₁-C₁₂)-alkylcarbamoyloxy, N-(C₃-C₈)-Cycloalkylcarbamoyloxy, N-(C₆-C₁₆)-Arylcarbamoyloxy, N-(C₇-C₁₆)-Aralkylcarbamoyloxy, N-(C₁-C₁₀)-Alkyl-N-(C₆-C₁₂)-arylcarbamoyloxy, N-(C₁-C₁₀)-Alkyl-N-(C₇-C₁₆)-Aralkylcarbamoyloxy, N-((C₁-C₁₀)-alkyl))carbamoyloxy, N-((C₆-C₁₂)-Aryloxy-(C₁-C₁₀)-alkyl)-carbamoyloxy, N-((C₇-C₁₆)-Aralkyloxy-(C₁-C₁₀)-alkyl)carbamoyloxy, N-(C₁-C₁₀)-Alkyl-N-((C₁-C₁₀)-alkoxy-(C₁-C₁₀)-alkyl)carbamoyloxy, N-(C₁-C₁₀)-Alkyl-N-((C₆-C₁₂)-aryloxy-(C₁-C₁₀)-alkyl)carbamoyloxy, N-(C₁-C₁₀)-Alkyl-N-((C₇-C₁₆)-aralkyloxy-(C₁-C₁₀)-alkyl)carbamoyloxy,
Amino, (C₁-C₁₂)-Alkylamino, Di-(C₁-C₁₂)alkylamino, (C₃-C₈)-Cycloalkylamino, (C₃-C₁₂)-Alkenylamino, (C₃-C₁₂) Alkinylamino, N-(C₆-C₁₂)-Arylamino, N-(C₇-C₁₁)-Aralkylamino, N-Alkyl-Aralkylamino, N-Alkyl-Arylamino, (C₁-C₁₂)-Alkoxyamino, (C₁-C₁₂)-Alkoxy-N-(C₁-C₁₀)-alkylamino,
(C₁-C₁₂)-Alkanoylamino, (C₃-C₈)-Cycloalkanoylamino, (C₆-C₁₂)-Aroylamino, (C₇-C₁₆)-Aralkanoylamino, (C₁-C₁₂)-Alkanoyl-N-(C₁-C₁₀)-alkylamino, (C₃-C₈)-Cycloalkanoyl-N-(C₁-C₁₀)-alkylamino, (C₆-C₁₂)-Aroyl-N-(C₁-C₁₀)-alkylamino, (C₇-C₁₁)-Aralkanoyl-N-(C₁-C₁₀)-alkylamino,
(C₁-C₁₂)-Alkanoylamino-(C₁-C₈)-alkyl, (C₃-C₈)-Cycloalkanoylamino-(C₁-C₈)-alkyl, (C₆-C₁₂)-Aroylamino-(C₁-C₈)-alkyl, (C₇-C₁₆)-Aralkanoylamino-(C₁-C₈)-alkyl, Amino-(C₁-C₁₀)-alkyl, N-(C₁-C₁₀)alkylamino-(C₁-C₁₀)-alkyl, N,N-Di-(C₁-C₁₀)-alkylamino-(C₁-C₁₀)-alkyl, (C₃-C₈)-Cycloalkylamino-(C₁-C₁₀)-alkyl,
(C₁-C₁₂)-Alkylmercapto, (C₁-C₁₂)-Alkylsulfinyl, (C₁-C₁₂)-Alkylsulfonyl, (C₆-C₁₆)-Arylmercapto, (C₆-C₁₆)-Arylsulfinyl, (C₆-C₁₆)-Arylsulfonyl, (C₇-C₁₆)-Aralkylmercapto, (C₇-C₁₆)-Aralkylsulfinyl, (C₇-C₁₆)-Aralkylsulfonyl,
- R¹ und R³: gleich oder verschieden sind und Wasserstoff, (C₁-C₈)-Alkyl, (C₁-C₈)-Alkoxy, Halogen, insbesondere Fluor, Chlor oder Brom, Nitril, Hydroxy, Amino, ggf. mono- oder disubstituiert mit (C₁-C₆)-Alkyl, oder Hydroxy-(C₁-C₆)-alkyl, (C₁-C₆)-Alkylcarbonyloxy bedeutet,
- R²: eine carbonsäureisostere Gruppe, vorzugsweise 2-Imidazolyl, 5-Tetrazolyl, 3-Hydroxypyrazolyl, 3-Hydroxyisothiazolyl, 3-Hydroxyisoxazolyl oder einen Rest -[CH₂]ₚ-CO-NR⁵R⁶ bedeutet, wobei
p = 0, 1, 2, 3 oder 4,
- R⁵: Wasserstoff, (C₁-C₆)-Alkyl oder eine N-Schutzgruppe wie (C₁-C₈)-Alkanoyl, (C₁-C₆)-Alkylcarbamoyl, (C₁-C₆)-Alkoxycarbonyl, Benzyloxycarbonyl, (C₁-C₁₀)-Acyloxy-(C₁-C₆)-alkyl, vorzugsweise (C₁-C₁₀)-Alkanoyloxy-(C₁-C₆)-alkyl, Benzoyloxy-(C₁-C₆)-alkyl, Benzyloxycarbonyloxy-(C₁-C₆)-alkyl oder (C₁-C₆)-Alkoxycarbonyloxy-(C₁-C₆)-alkyl, ein 1, 2, 3 oder 4-wertiges physiologisch verwendbares Kation, insbesondere Na⊕, K⊕, Mg²⊕, Ca²⊕, Al³⊕ oder ein Ammoniumion, ggf. 1-3fach substituiert mit (C₁-C₈)-Hydroxyalkyl, (C₁-C₄)-Alkoxy-(C₁-C₈)-alkyl, Phenyl, Benzyl oder (C₁-C₈)-Alkyl, welches seinerseits 1-3fach substituiert sein kann mit Hydroxy oder (C₁-C₄)-Alkoxy oder ein Kation eines basischen Aminosäurederivates bedeutet,
- R⁶: einen Rest der Formel I, ausgenommen -SO₂H, bedeutet

-G-K-[C-U]ᵣ-D-W (I)

in welchem
- G: -SO-, -SO₂- oder -CO- bedeutet,
- K: eine Bindung oder -NR⁷- bedeutet,
- C: eine Bindung oder
einen verzweigten oder unverzweigten aliphatischen (C₁-C₁₆)-Alkandiyl- oder cycloaliphatischen (C₃-C₁₀)-Alkandiylrest, einen verzweigten oder unverzweigten (C₂-C₁₆)-Alkendiyl-oder Cycloalkendiyl-Rest, einen (C₂-C₁₆)-Alkindiyl-Rest oder einen (C₂-C₁₆)-Alkenindiyl-Rest bedeutet, der jeweils eine oder mehrere C-C-Mehrfachbindungen enthalten kann,
- U: eine Bindung, Wasserstoff oder
einen Rest aus der Reihe folgender Heteroatomgruppierungen bedeutet
-CO-, -O(C)O-, -(CO)-O-, -(CO)NR-, -NR(CO)-, -O-,-SO-, - SO₂-, -NR, worin R (C₁-C₃)-Alkyl oder Wasserstoff bedeutet,
- r: 1, 2, 3 oder 4 ist,
- D: eine Bindung, Wasserstoff oder
einen verzweigten oder unverzweigten aliphatischen (C₁-C₁₀)-Alkandiyl-Rest,
einen verzweigten oder unverzweigten (C₁-C₁₀)-Alkendiyl-Rest, einen (C₂-C₁₀)-Alkindiyl-Rest oder einen (C₂-C₁₀)-Alkenindiyl-Rest bedeutet, der jeweils eine oder mehrere C-C-Mehrfachbindungen enthalten kann,
- W: eine Bindung, Wasserstoff oder
einen (C₃-C₁₀)-cycloaliphatischen Alkyl-, Alkenyl-, Alkinyl-oder Alkeninyl-Rest,
einen (C₆-C₁₆)-Arylrest oder einen 5- oder 6-gliedrigen Heteroarylrest bedeutet, wobei mindestens eine der Variablen C, D oder W nicht eine Bindung bedeuten und U nur dann in der Bedeutung einer Heteratomgruppierung steht, wenn C nicht eine Bindung bedeutet oder wenn D und/oder W nicht eine Bindung bedeuten und
- C,D und/oder W,: sofern diese keine Bindung oder Wasserstoff bedeuten, vorzugsweise ihrerseits substituiert sind durch eine Kombination von bis zu 5 gleichen oder verschiedenen Substituenten aus der Reihe
Hydroxy, Halogen, Cyano, Trifluoromethyl, Nitro, Carboxy, (C₁-C₁₂)-Alkyl, (C₃-C₈)-Cycloalkyl, (C₃-C₈)-Cycloalkyl-(C₁-C₁₂)-alkyl, (C₃-C₈)-Cycloalkoxy, (C₃-C₈)-Cycloalkyl-(C₁-C₁₂)-alkoxy, (C₃-C₈)-Cycloalkyloxy-(C₁-C₁₂)-alkyl, (C₃-C₈)-Cycloalkyloxy-(C₁-C₁₂)-alkoxy, (C₃-C₈)-Cycloalkyl-(C₁-C₈)-alkyl-(C₁-C₆)-alkoxy, (C₃-C₈)-Cycloalkyl-(C₁-C₈)-alkoxy-(C₁-C₆)-alkyl, (C₃-C₈)-Cycloalkyloxy-(C₁-C₈)-alkoxy-(C₁-C₆)-alkyl, (C₃-C₈)-Cycloalkoxy-(C₁-C₈)-alkoxy-(C₁-C₈)-alkoxy, (C₆-C₁₂)-Aryl, (C₇-C₁₆)-Aralkyl, (C₂-C₁₂)-Alkenyl, (C₂-C₁₂)-Alkinyl, (C₁-C₁₂)-Alkoxy, (C₁-C₁₂)-Alkoxy-(C₁-C₁₂)alkyl, (C₁-C₁₂)-Alkoxy-(C₁-C₁₂)alkoxy, (C₁-C₁₂)-Alkoxy-(C₁-C₈)-alkoxy-(C₁-C₈)-alkyl, (C₆-C₁₂)-Aryloxy, (C₇-C₁₆)-Aralkyloxy, (C₆-C₁₂)-Aryloxy-(C₁-C₆)-alkoxy, (C₇-C₁₆)-Aralkoxy-(C₁-C₆)-alkoxy, (C₁-C₈)-Hydroxyalkyl, (C₆-C₁₆)-Aryloxy-(C₁-C₈)-alkyl, (C₇-C₁₆)-Aralkoxy-(C₁-C₈)-alkyl, (C₆-C₁₂)-Aryloxy-(C₁-C₈)-alkoxy-(C₁-C₆)-alkyl, (C₇-C₁₂)-Aralkyloxy-(C₁-C₈)-alkoxy-(C₁-C₆)-alkyl, -O-[CH₂₋]ₓC_{f}H_{(2f+1-g)}F_{g}, -OCF₂Cl, -OCF₂-CHFCl,
(C₁-C₁₂)-Alkylcarbonyl, (C₃-C₈)-Cycloalkylcarboxyl, (C₆-C₁₂)-Arylcarbonyl, (C₇-C₁₆)-Aralkylcarbonyl, Cinnamoyl, (C₂-C₁₂) Alkenylcarbonyl, (C₂-C₁₂)-Alkinylcarbonyl,
(C₁-C₁₂)-Alkoxycarbonyl, (C₁-C₁₂)-Akoxy-(C₁-C₁₂)-alkoxycarbonyl, (C₆-C₁₂)- Aryloxycarbonyl, (C₇-C₁₆)-Aralkoxycarbonyl, (C₃-C₁₀)-Cycloalkoxycarbonyl, (C₂-C₁₂)-Alkenyloxycarbonyl, (C₂-C₁₂)-Alkinyloxycarbonyl,
(C₁-C₁₂)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy, (C₂-C₁₂)-Alkenylcarboflyloxy, (C₂-C₁₂)-Alkinylcarbonyloxy,
(C₁-C₁₂)-Alkoxycarbonyloxy, (C₁-C₁₂)-Alkoxy-(C₁-C₁₂)-alkoxycarbonyloxy, (C₆-C₁₂)-Aryloxycarbonyloxy, (C₇-C₁₆)-Aralkyloxycarbonyloxy, (C₃-C₈)-Cycloalkoxycarbonyloxy, (C₂-C₁₂)-Alkenyloxycarbonyloxy, (C₂-C₁₂)-Alkinyloxycarbonyloxy,
Carbamoyl, N-(C₁-C₁₂)-Alkylcarbamoyl, N, N-Di-(C₁-C₁₂)-alkylcarbamoyl, N-(C₃-C₈)-Cycloalkylcarbamoyl, N,N-Dicyclo-(C₃-C₈)-alkylcarbamoyl, N-(C₁-C₁₀)-Alkyl-N-(C₃-C₈)-Cycloalkylcarbamoyl, N-((C₃-C₈)-Cycloalkyl-(C₁-C₆)-alkyl)carbamoyl, N-(C₁-C₆)-Alkyl-N-((C₃-C₈)-cycloalkyl-(C₁-C₆)-alkyl)carbamoyl, N-(+)-Dehydroabietylcarbamoyl, N-(C₁-C₆)-Alkyl-N-(+)-dehydroabietylcarbamoyl, N-(C₆-C₁₂)-Arylcarbamoyl, N-(C₇-C₁₆)-Aralkylcarbamoyl, N-(C₁-C₁₀)-Alkyl-N-(C₆-C₁₆)-arylcarbamoyl, N-(C₁-C₁₀)-Alkyl-N-(C₇-C₁₆)-aralkylcarbamoyl, N-((C₁-C₁₀)-Alkoxy-(C₁-C₁₀)alkyl)carbamoyl, N-((C₆-C₁₆)-Aryloxy-(C₁-C₁₀)alkyl)carbamoyl,
N-((C₇-C₁₆)-Aralkyloxy-(C₁-C₁₀)-alkyl)carbamoyl, N-(C₁-C₁₀)-Alkyl-N-((C₁-C₁₀)-alkoxy-(C₁-C₁₀)-alkyl)carbamoyl, N-(C₁-C₁₀)-Alkyl-N-((C₆-C₁₂)-aryloxy-(C₁-C₁₀)-alkyl)carbamoyl, N-(C₁-C₁₀)-Alkyl-N-((C₇-C₁₆)-aralkyloxy-(C₁-C₁₀)-alkyl)carbamoyl, CON(CH₂)ₕ, worin eine CH₂-Gruppe durch O, S, N-(C₁-C₈)-Alkylimino, N-(C₃-C₈)-Cycloalkylimino, N-(C₃-C₈)-Cycloalkyl-(C₁-C₄)-alkylimino, N-(C₆-C₁₂)-Arylimino, N-(C₇-C₁₆)-Aralkylimino oder N-(C₁-C₄)-Alkoxy-(C₁-C₆)-alkylimino ersetzt ein kann und h 3 bis 6 bedeutet, oder mit einem Carbamoyl-Rest der allgemeinen Formel II 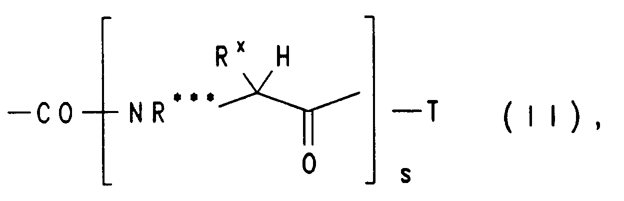 worin
- R^{x}: der Substituent einer α-Aminosäure bedeutet, zu denen die L-und D-Aminosäuren zählen,
- s: 1, 2, 3, 4 oder 5 und
- T: OH, OR oder NR*R** bedeutet, wobei
- R***: Wasserstoff oder (C₁-C₄)-Alkyl,
- R* und R**: gleich oder verschieden sind und Wasserstoff, (C₆-C₁₂)-Aryl, (C₇-C₁₁)-Aralkyl, (C₁-C₈)-Alkyl, (C₃-C₈)-Cycloalkyl, (+)-Dehydroabietyl, (C₁-C₈)-Alkoxy-(C₁-C₈)-alkyl, (C₇-C₁₂)-Aralkoxy-(C₁-C₈)-alkyl, (C₆-C₁₂)-Aryloxy-(C₁-C₈)-alkyl, (C₁-C₁₀)-Alkanoyl, ggf. substituiertes (C₇-C₁₆)-Aralkanoyl, ggf. substituiertes (C₆-C₁₂)-Aroyl bedeuten, oder
- R* und R**: gemeinsam für -[CH₂]ₕ stehen, worin eine CH₂ Gruppe durch O, S, SO, SO₂, N-Acylimino, N-(C₁-C₁₀)-Alkoxycarbonylimino, N-(C₁-C₈)-Alkylimino, N-(C₃-C₈)-Cycloalkylimino, N-(C₃-C₈)-Cycloalkyl-(C₁-C₄)alkylimino, N-(C₆-C₁₂)-Arylimino, N-(C₇-C₁₆)-Aralkylimino oder N-(C₁-C₄)-Alkoxy-(C₁-C₆)-alkylimino ersetzt sein kann und h 3 bis 7 bedeutet, oder mit
Carbamoyloxy, N-(C₁-C₁₂)-Alkylcarbamoyloxy, N,N-Di-(C₁-C₁₂)-alkylcarbamoyloxy, N-(C₃-C₈)-Cycloalkylcarbamoyloxy, N-(C₆-C₁₂)-Arylcarbamoyloxy, N-(C₇-C₁₆)-Aralkylcarbamoyloxy, N-(C₁-C₁₀)-Alkyl-N-(C₆-C₁₂)-arylcarbamoyloxy, N-(C₁-C₁₀)-Alkyl-N-(C₇-C₁₆)-Aralkylcarbamoyloxy, N-((C₁-C₁₀)-alkyl))carbamoyloxy, N-((C₆-C₁₂)-Aryloxy-(C₁-C₁₀)-alkyl)carbamoyloxy, N-((C₇-C₁₆)-Aralkyloxy-(C₁-C₁₀)-alkyl)carbamoyloxy, N-(C₁-C₁₀)-Alkyl-N-((C₁-C₁₀)-alkoxy-(C₁-C₁₀)-alkyl)carbamoyloxy, N-(C₁-C₁₀)-Alkyl-N-((C₆-C₁₂)-aryloxy-(C₁-C₁₀)-alkyl)carbamoyloxy, N-(C₁-C₁₀)-Alkyl-N-((C₇-C₁₆)-aralkyloxy-(C₁-C₁₀)-alkyl)carbamoyloxy,
Amino, (C₁-C₁₂)-Alkylamino, Di-(C₁-C₁₂)alkylamino, (C₃-C₈)-Cycloalkylamino, (C₃-C₁₂)-Alkenylamino, (C₃-C₁₂) Alkinylamino, N-(C₆-C₁₂)-Arylamino, N-(C₇-C₁₁)-Aralkylamino, N-Alkyl-Aralkylamino, N-Alkyl-Arylamino, (C₁-C₁₂)-Alkoxyamino, (C₁-C₁₂)-Alkoxy-N-(C₁-C₁₀)-alkylamino,
(C₁-C₁₂)-Alkanoylamino, (C₃-C₈)-Cycloalkanoylamino, (C₆-C₁₂)-Aroylamino, (C₇-C₁₆)-Aralkanoylamino, (C₁-C₁₂)-Alkanoyl-N-(C₁-C₁₀)-alkylamino, (C₃-C₈)-Cycloalkanoyl-N-(C₁-C₁₀)-alkylamino, (C₆-C₁₂)-Aroyl-N-(C₁-C₁₀)alkylamino, (C₇-C₁₁)-Aralkanoyl-N-(C₁-C₁₀)-alkylamino,
(C₁-C₁₂)-Alkanoylamino-(C₁-C₈)-alkyl, (C₃-C₈)-Cycloalkanoylamino-(C₁-C₈)alkyl, (C₆-C₁₂)-Aroylamino-(C₁-C₈)-alkyl, (C₇-C₁₆)-Aralkanoylamino-(C₁-C₈)-alkyl, Amino-(C₁-C₁₀)-alkyl, N-(C₁-C₁₀)-alkylamino-(C₁-C₁₀)alkyl, N,N-Di(C₁-C₁₀)alkylamino-(C₁-C₁₀)alkyl, (C₃-C₈)-Cycloalkylamino-(C₁-C₁₀)-alkyl, (C₁-C₁₂)-Alkylmercapto, (C₁-C₁₂)-Alkylsulfinyl, (C₁-C₁₂)-Alkylsulfonyl, (C₆-C₁₂)-Arylmercapto, (C₆-C₁₂)-Arylsulfinyl,
(C₆-C₁₂)-Arylsulfonyl, (C₇-C₁₆)-Aralkylmercapto, (C₇-C₁₆)-Aralkylsulfinyl, (C₇-C₁₆)-Aralkylsulfonyl,
Sulfamoyl, N-(C₁-C₁₀)-Alkylsulfamoyl, N,N-Di-(C₁-C₁₀)-alkylsulfamoyl, (C₃-C₈)-Cycloalkylsulfamoyl, N-(C₆-(C₁₂)-Arylsulfamoyl, N-(C₇-C₁₆)-Aralkylsulfamoyl, N-(C₁-C₁₀)-Alkyl-N-(C₆-C₁₂)-arylsulfamoyl, N-(C₁-C₁₀)-Alkyl-N-(C₇-C₁₆)-aralkylsulfamoyl, (C₁-C₁₀)-Alkyl-sulfonamido, N-((C₁-C₁₀)-alkyl)-(C₁-C₁₀)-alkylsulfonamido, (C₇-C₁₆)-Aralkylsulfonamido, oder mit N-((C₁-C₁₀)-alkyl-(C₇-C₁₆)-aralkylsulfonamido substituiert sind, wobei die Reste, die einen Arylrest enthalten ihrerseits am Aryl substituiert sein können durch 1 bis 5 gleiche oder verschiedene Reste aus der Reihe:
Hydroxy, Halogen, Cyano, Trifluormethyl, Nitro, Carboxy, (C₁-C₁₂)-Alkyl, (C₃-C₈)-Cycloalkyl, (C₆-C₁₂)-Aryl, (C₇-C₁₆)-Aralkyl, (C₁-C₁₂)-Alkoxy, (C₁-C₁₂)-Alkoxy-(C₁-C₁₂)alkyl, (C₁-C₁₂)-Alkoxy-(C₁-C₁₂)alkoxy, (C₆-C₁₂)-Aryloxy, (C₇-C₁₆)-Aralkyloxy, (C₁-C₈)-Hydroxyalkyl,
(C₁-C₁₂)-Alkylcarbonyl, (C₃-C₈)-Cycloalkylcarbonyl, (C₆-C₁₂)-Arylcarbonyl, (C₇-C₁₆)-Aralkylcarbonyl,
(C₁-C₁₂)-Alkoxycarbonyl, (C₁-C₁₂)-Alkoxy-(C₁-C₁₂)-alkoxycarbonyl, (C₆-C₁₂)- Aryloxycarbonyl, (C₇-C₁₆)-Aralkoxycarbonyl, (C₃-C₈)-Cycloalkoxycarbonyl, (C₂-C₁₂)-Alkenyloxycarbonyl, (C₂-C₁₂)-Alkinyloxycarbonyl,
(C₁-C₁₂)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy, (C₂-C₁₂)-Alkenylcarbonyloxy, (C₂-C₁₂)-Alkinylcarbonyloxy,
(C₁-C₁₂)-Alkoxycarbonyloxy, (C₁-C₁₂)-Alkoxy-(C₁-C₁₂)-alkoxycarbonyloxy, (C₆-C₁₂)-Aryloxycarbonyloxy, (C₇-C₁₆)-Aralkyloxycarbonyloxy, (C₃-C₈)-Cycloalkoxycarbonyloxy, (C₂-C₁₂)-Alkenyloxycarbonyloxy, (C₂-C₁₂)-Alkinyloxycarbonyloxy,
Carbamoyl, N-(C₁-C₁₂)-Alkylcarbamoyl, N, N-Di-(C₁-C₁₂)-alkylcarbamoyl, N-(C₃-C₈)-Cycloalkylcarbamoyl, N,N-Dicyclo-(C₃-C₈)-alkylcarbamoyl, N-(C₁-C₁₀)-Alkyl-N-(C₃-C₈)-cycloalkylcarbamoyl, N-((C₃-C₈)-Cycloalkyl-(C₁-C₆)-alkyl)carbamoyl, N-(C₁-C₆)-Alkyl-N-((C₃-C₈)-cycloalkyl-(C₁-C₆)-alkyl)carbamoyl, N-(+)-Dehydroabietylcarbamoyl, N-(C₁-C₆)-Alkyl-N-(+)-dehydroabietylcarbamoyl, N-(C₆-C₁₂)-Arylcarbamoyl, N-(C₇-C₁₆)-Aralkylcarbamoyl, N-(C₁-C₁₀)-Alkyl-N-(C₆-C₁₆)-arylcarbamoyl, N-(C₁-C₁₀)-Alkyl-N-(C₇-C₁₆)-aralkylcarbamoyl, N-((C₁-C₁₀)-Alkoxy-(C₁-C₁₀)-alkyl)carbamoyl, N-((C₆-C₁₆)-Aryloxy-(C₁-C₁₀)-alkyl)carbamoyl, N-((C₇-C₁₆)-Aralkyloxy-(C₁-C₁₀)-alkyl)carbamoyl, N-(C₁-C₁₀)-Alkyl-N-((C₁-C₁₀)-alkoxy-(C₁-C₁₀)-alkyl)carbamoyl, N-(C₁-C₁₀)-Alkyl-N-((C₆-C₁₂)-aryloxy-(C₁-C₁₀)-alkyl)carbamoyl, N-(C₁-C₁₀)-Alkyl-N-((C₇-C₁₆)-aralkyloxy-(C₁-C₁₀)-alkyl)carbamoyl, CON(CH₂)ₕ worin eine CH₂-Gruppe durch O, S, N-(C₁-C₈)Alkylimino, N-(C₃-C₈)-Cycloalkylimino, N-(C₃-C₈)-Cycloalkyl-(C₁-C₄)alkylimino, N-(C₆-C₁₂)-Arylimino, N-(C₇-C₁₆)-Aralkylimino oder N-(C₁-C₄)-Alkoxy-(C₁-C₆)-alkylimino ersetzt ein kann und h 3 bis 7 bedeutet,
Carbamoyloxy, N-(C₁-C₁₂)-Alkylcarbamoyloxy, N,N-Di-(C₁-C₁₂)-alkylcarbamoyloxy, N-(C₃-C₈)-Cycloalkylcarbamoyloxy, N-(C₆-C₁₆)-Arylcarbamoyloxy, N-(C₇-C₁₆)-Aralkylcarbamoyloxy, N-(C₁-C₁₀)-Alkyl-N-(C₆-C₁₂)-arylcarbamoyloxy, N-(C₁-C₁₀)-Alkyl-N-(C₇-C₁₆)-Aralkylcarbamoyloxy, N-((C₁-C₁₀)-alkyl))carbamoyloxy, N-((C₆-C₁₂)-Aryloxy-(C₁-C₁₀)-alkyl)-carbamoyloxy, N-((C₇-C₁₆)-Aralkyloxy-(C₁-C₁₀)-alkyl)carbamoyloxy, N-(C₁-C₁₀)-Alkyl-N-((C₁-C₁₀)-alkoxy-(C₁-C₁₀)-alkyl)carbamoyloxy, N-(C₁-C₁₀)-Alkyl-N-((C₆-C₁₂)-aryloxy-(C₁-C₁₀)-alkyl)carbamoyloxy, N-(C₁-C₁₀)-Alkyl-N-((C₇-C₁₆)-aralkyloxy-(C₁-C₁₀)-alkyl)carbamoyloxy,
Amino, (C₁-C₁₂)-Alkylamino, Di-(C₁-C₁₂)alkylamino, (C₃-C₈)-Cycloalkylamino, (C₃-C₁₂)-Alkenylamino, (C₃-C₁₂) Alkinylamino, N-(C₆-C₁₂)-Arylamino, N-(C₇-C₁₁)-Aralkylamino, N-Alkyl-Aralkylamino, N-Alkyl-Arylamino, (C₁-C₁₂)-Alkoxyamino, (C₁-C₁₂)-Alkoxy-N-(C₁-C₁₀)-alkylamino,
(C₁-C₁₂)-Alkanoylamino, (C₃-C₈)-Cycloalkanoylamino, (C₆-C₁₂)-Aroylamino, (C₇-C₁₆)-Aralkanoylamino, (C₁-C₁₂)-Alkanoyl-N-(C₁-C₁₀)-alkylamino, (C₃-C₈)-Cycloalkanoyl-N-(C₁-C₁₀)-alkylamino, (C₆-C₁₂)-Aroyl-N-(C₁-C₁₀)-alkylamino, (C₇-C₁₁)-Aralkanoyl-N-(C₁-C₁₀)-alkylamino,
(C₁-C₁₂)-Alkanoylamino-(C₁-C₈)-alkyl, (C₃-C₈)-Cycloalkanoylamino-(C₁-C₈)-alkyl, (C₆-C₁₂)-Aroylamino-(C₁-C₈)-alkyl, (C₇-C₁₆)-Aralkanoylamino-(C₁-C₈)-alkyl, Amino-(C₁-C₁₀)-alkyl, N-(C₁-C₁₀)alkylamino-(C₁-C₁₀)-alkyl, N,N-Di-(C₁-C₁₀)-alkylamino-(C₁-C₁₀)-alkyl, (C₃-C₈)-Cycloalkylamino-(C₁-C₁₀)-alkyl,
(C₁-C₁₂)-Alkylmercapto, (C₁-C₁₂)-Alkylsulfinyl, (C₁-C₁₂)-Alkylsulfonyl, (C₆-C₁₆)-Arylmercapto, (C₆-C₁₆)-Arylsulfinyl, (C₆-C₁₆)-Arylsulfonyl, (C₇-C₁₆)-Aralkylmercapto, (C₇-C₁₆)-Aralkylsulfinyl, (C₇-C₁₆)-Aralkylsulfonyl,
- R⁷: Wasserstoff, (C₁-C₈)-Alkyl, (C₆-C₁₂)-Aryl, (C₇-C₁₁)-Aralkyl, Heteroaryl, bedeutet,
wobei die vorstehenden Reste 1, 2 oder 3 gleiche oder verschiedene Substituenten aus der Reihe
Fluor, Chlor, Trifluormethyl, Nitril, (C₁-C₆)-Alkyl, (C₁-C₆)-Alkoxy, (C₁-C₆)-Alkylcarbamoyl, Di-(C₁-C₆)-alkylcarbamoyl, (C₃-C₆)-Cycloalkylcarbamoyl, (C₁-C₆)-Alkoxycarbonyl tragen können,
- R⁴: einen verzweigten oder unverzweigten (C₇-C₂₀)-Alkylrest, einen (C₆-C₁₆)-Arylrest, einen (C₇-C₁₆)-Aralkylrest, einen Heteroarylrest oder einen Heteroaralkylrest bedeutet,
wobei diese Reste substituiert sind mit einem oder mehreren Resten aus der Reihe
(C₁-C₁₂)-Alkylcarbonyl, (C₃-C₈)-Cycloalkylcarbonyl, (C₆-C₁₂)-Arylcarbonyl, (C₇-C₁₆)-Aralkylcarbonyl, Cinnamoy, (C₂-C₁₂) Alkenylcarbonyl, (C₂-C₁₂)-Alkinylcarbonyl,
Hydroxy, Halogen, Cyano, Trifluormethyl, Nitro, Carboxy, (C₁-C₁₂)-Alkyl, (C₃-C₈)-Cycloalkyl, (C₃-C₈)-Cycloalkyl-(C₁-C₁₂)-alkyl, (C₃-C₈)-Cycloalkoxy, (C₃-C₈)-Cycloalkyl-(C₁-C₁₂)-alkoxy, (C₃-C₈)-Cycloalkyloxy-(C₁-C₁₂)-alkyl, (C₃-C₈)-Cycloalkyloxy-(C₁-C₁₂)-alkoxy, (C₃-C₈)-Cycloalkyl-(C₁-C₈)-alkyl-(C₁-C₆)-alkoxy, (C₃-C₈)-Cycloalkyl-(C₁-C₈)-alkoxy-(C₁-C₆)-alkyl, (C₃-C₈)-Cycloalkyloxy-(C₁-C₈)-alkoxy-(C₁-C₆)-alkyl, (C₃-C₈)-Cycloalkoxy-(C₁-C₈)-alkoxy-(C₁-C₈)-alkoxy, (C₆-C₁₂)-Aryl, (C₇-C₁₆)-Aralkyl, (C₂-C₁₂)-Alkenyl, (C₂-C₁₂)-Alkinyl, (C₁-C₁₂)-Alkoxy, (C₁-C₁₂)-Alkoxy-(C₁-C₁₂)alkyl, (C₁-C₁₂)-Alkoxy-(C₁-C₁₂)alkoxy, (C₁-C₁₂)-Alkoxy-(C₁-C₈)-alkoxy-(C₁-C₈)-alkyl, (C₆-C₁₂)-Aryloxy, (C₇-C₁₆)-Aralkyloxy, (C₆-C₁₂)-Aryloxy-(C₁-C₆)-alkoxy, (C₇-C₁₆)-Aralkoxy-(C₁-C₆)-alkoxy, (C₁-C₈)-Hydroxyalkyl, (C₆-C₁₆)-Aryloxy-(C₁-C₈)-alkyl, (C₇-C₁₆)-Aralkoxy-(C₁-C₈)-alkyl, (C₆-C₁₂)-Aryloxy-(C₁-C₈)-alkoxy-(C₁-C₆)-alkyl, (C₇-C₁₂)-Aralkyloxy-(C₁-C₈)-alkoxy-(C₁-C₆)-alkyl, -O-[CH₂₋]ₓC_{f}H_{(2f+1-g)}F_{g}, -OCF₂Cl, -OCF₂-CHFCl, (C₁-C₁₂)-Alkylcarbonyl, (C₃-C₈)-Cycloalkylcarbonyl, (C₆-C₁₂)-Arylcarbonyl, (C₇-C₁₆)-Aralkylcarbonyl, Cinnamoyl, (C₂-C₁₂) Alkenylcarbonyl, (C₂-C₁₂)-Alkinylcarbonyl,
(C₁-C₁₂)-Alkoxycarbonyl, (C₁-C₁₂)-Alkoxy-(C₁-C₁₂)-alkoxycarbonyl, (C₆-C₁₂)-Aryloxycarbonyl, (C₇-C₁₆)-Aralkoxycarbonyl, (C₃-C₁₀)-Cycloalkoxycarbonyl, (C₂-C₁₂)-Alkenyloxycarbonyl, (C₂-C₁₂)-Alkinyloxycarbonyl,
(C₁-C₁₂)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy, (C₂-C₁₂)-Alkenylcarbonyloxy, (C₂-C₁₂)-Alkinylcarbonyloxy,
(C₁-C₁₂)-Alkoxycarbonyloxy, (C₁-C₁₂)-Alkoxy-(C₁-C₁₂)-alkoxycarbonyloxy, (C₆-C₁₂)-Aryloxycarbonyloxy, (C₇-C₁₆)-Aralkyloxycarbonyloxy, (C₃-C₈)-Cycloalkoxycarbonyloxy, (C₂-C₁₂)-Alkenyloxycarbonyloxy, (C₂-C₁₂)-Alkinyloxycarbonyloxy,
Carbamoyl, N-(C₁-C₁₂)-Alkylcarbamoyl, N, N-Di-(C₁-C₁₂)-alkylcarbamoyl, N-(C₃-C₈)-Cycloalkylcarbamoyl, N,N-Dicyclo-(C₃-C₈)-alkylcarbamoyl, N-(C₁-C₁₀)-Alkyl-N-(C₃-C₈)-Cycloalkylcarbamoyl, N-((C₃-C₈)-Cycloalkyl-(C₁-C₆)-alkyl)carbamoyl, N-(C₁-C₆)-Alkyl-N-((C₃-C₈)-cycloalkyl-(C₁-C₆)-alkyl)carbamoyl, N-(+)-Dehydroabietylcarbamoyl, N-(C₁-C₆)-Alkyl-N-(+)-dehydroabietylcarbamoyl, N-(C₆-C₁₂)-Arylcarbamoyl, N-(C₇-C₁₆)-Aralkylcarbamoyl, N-(C₁-C₁₀)-Alkyl-N-(C₆-C₁₆)-arylcarbamoyl, N-(C₁-C₁₀)-Alkyl-N-(C₇-C₁₆)-aralkylcarbamoyl, N-((C₁-C₁₀)-Alkoxy-(C₁-C₁₀)alkyl)carbamoyl, N-((C₆-C₁₆)-Aryloxy-(C₁-C₁₀)alkyl)carbamoyl,
N-((C₇-C₁₆)-Aralkyloxy-(C₁-C₁₀)-alkyl)carbamoyl,
N-(C₁-C₁₀)-Alkyl-N-((C₁-C₁₀)-alkoxy-(C₁-C₁₀)-alkyl)carbamoyl,
N-(C₁-C₁₀)-Alkyl-N-((C₆-C₁₂)-aryloxy-(C₁-C₁₀)-alkyl)carbamoyl,
N-(C₁-C₁₀)-Alkyl-N-((C₇-C₁₆)-aralkyloxy-(C₁-C₁₀)-alkyl)carbamoyl, CON(CH₂)ₕ, worin eine CH₂-Gruppe durch O, S, N-(C₁-C₈)-Alkylimino, N-(C₃-C₈)-Cycloalkylimino, N-(C₃-C₈)-Cycloalkyl-(C₁-C₄)-alkylimino, N-(C₆-C₁₂)-Arylimino, N-(C₇-C₁₆)-Aralkylimino oder N-(C₁-C₄)-Alkoxy-(C₁-C₆)-alkylimino ersetzt ein kann und h 3 bis 6 bedeutet, oder mit
einem Carbamoyl-Rest der allgemeinen Formel II 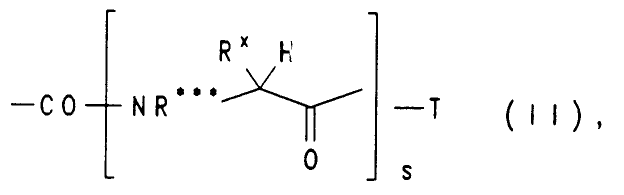
worin
- R^{x}: der Substituent einer α-Aminosäure bedeutet, zu denen die L-und D-Aminosäuren zählen,
- s: 1, 2, 3, 4 oder 5 und
- T: OH, OR oder NR*R** bedeutet, wobei
- R***: Wasserstoff oder (C₁-C₄)-Alkyl,
- R* und R**: gleich oder verschieden sind und Wasserstoff, (C₆-C₁₂)-Aryl, (C₇-C₁₁)-Aralkyl, (C₁-C₈)-Alkyl, (C₃-C₈)-Cycloalkyl, (+)-Dehydroabietyl, (C₁-C₈)-Alkoxy-(C₁-C₈)alkyl, (C₇-C₁₂)-Aralkoxy-(C₁-C₈)alkyl, (C₆-C₁₂)-Aryloxy-(C₁-C₈)-alkyl, (C₁-C₁₀)-Alkanoyl, ggf. substituiertes (C₇-C₁₆)-Aralkanoyl, ggf. substituiertes (C₆-C₁₂)-Aroyl bedeuten, oder
- R* und R**: gemeinsam für -[CH₂]ₕ stehen, worin eine CH₂ Gruppe durch O, S, SO, SO₂, N-Acylimino, N-(C₁-C₁₀)-Alkoxycarbonylimino, N-(C₁-C₈)-Alkylimino, N-(C₃-C₈)-Cycloalkylimino, N-(C₃-C₈)-Cycloalkyl-(C₁-C₄)alkylimino, N-(C₆-C₁₂)-Arylimino, N-(C₇-C₁₆)-Aralkylimino oder N-(C₁-C₄)-Alkoxy-(C₁-C₆)-alkylimino ersetzt sein kann und h 3 bis 7 bedeutet, oder mit
Carbamoyloxy, N-(C₁-C₁₂)-Alkylcarbamoyloxy, N,N-Di-(C₁-C₁₂)-alkylcarbamoyloxy, N-(C₃-C₈)-Cycloalkylcarbamoyloxy, N-(C₆-C₁₂)-Arylcarbamoyloxy, N-(C₇-C₁₆)-Aralkylcarbamoyloxy, N-(C₁-C₁₀)-Alkyl-N-(C₆-C₁₂)-arylcarbamoyloxy, N-(C₁-C₁₀)-Alkyl-N-(C₇-C₁₆)-Aralkylcarbamoyloxy, N-((C₁-C₁₀)-alkyl))carbamoyloxy, N-((C₆-C₁₂)-Aryloxy-(C₁-C₁₀)-alkyl)carbamoyloxy, N-((C₇-C₁₆)-Aralkyloxy-(C₁-C₁₀)-alkyl)carbamoyloxy, N-(C₁-C₁₀)-Alkyl-N-((C₁-C₁₀)-alkoxy-(C₁-C₁₀)-alkyl)carbamoyloxy, N-(C₁-C₁₀)-Alkyl-N-((C₆-C₁₂)-aryloxy-(C₁-C₁₀)-alkyl)carbamoyloxy, N-(C₁-C₁₀)-Alkyl-N-((C₇-C₁₆)-aralkyloxy-(C₁-C₁₀)-alkyl)carbamoyloxy,
Amino, (C₁-C₁₂)-Alkylamino, Di-(C₁-C₁₂)alkylamino, (C₃-C₈)-Cycloalkylamino, (C₃-C₁₂)-Alkenylamino, (C₃-C₁₂) Alkinylamino, N-(C₆-C₁₂)-Arylamino, N-(C₇-C₁₁)-Aralkylamino, N-Alkyl-Aralkylamino, N-Alkyl-Arylamino, (C₁-C₁₂)-Alkoxyamino, (C₁-C₁₂)-Alkoxy-N-(C₁-C₁₀)-alkylamino,
(C₁-C₁₂)-Alkanoylamino, (C₃-C₈)-Cycloalkanoylamino, (C₆-C₁₂)-Aroylamino, (C₇-C₁₆)-Aralkanoylamino, (C₁-C₁₂)-Alkanoyl-N-(C₁-C₁₀)-alkylamino, (C₃-C₈)-Cycloalkanoyl-N-(C₁-C₁₀)-alkylamino, (C₆-C₁₂)-Aroyl-N-(C₁-C₁₀)alkylamino, (C₇-C₁₁)-Aralkanoyl-N-(C₁-C₁₀)-alkylamino,
(C₁-C₁₂)-Alkanoylamino-(C₁-C₈)-alkyl, (C₃-C₈)-Cycloalkanoylamino-(C₁-C₈)alkyl, (C₆-C₁₂)-Aroylamino-(C₁-C₈)-alkyl, (C₇-C₁₆)-Aralkanoylamino-(C₁-C₈)-alkyl, Amino-(C₁-C₁₀)-alkyl, N-(C₁-C₁₀)-alkylamino-(C₁-C₁₀)alkyl, N,N-Di(C₁-C₁₀)alkylamino-(C₁-C₁₀)alkyl, (C₃-C₈)-Cycloalkylamino-(C₁-C₁₀)-alkyl, (C₁-C₁₂)-Alkylmercapto, (C₁-C₁₂)-Alkylsulfinyl, (C₁-C₁₂)-Alkylsulfonyl, (C₆-C₁₂)-Arylmercapto, (C₆-C₁₂)-Arylsulfinyl,
(C₆-C₁₂)-Arylsulfonyl, (C₇-C₁₆)-Aralkylmercapto, (C₇-C₁₆)-Aralkylsulfinyl, (C₇-C₁₆)-Aralkylsulfonyl,
Sulfamoyl, N-(C₁-C₁₀)-Alkylsulfamoyl, N,N-Di-(C₁-C₁₀)-alkylsulfamoyl, (C₃-C₈)-Cycloalkylsulfamoyl, N-(C₆-(C₁₂)-Arylsulfamoyl, N-(C₇-C₁₆)-Aralkylsulfamoyl, N-(C₁-C₁₀)-Alkyl-N-(C₆-C₁₂)-arylsulfamoyl, N-(C₁-C₁₀)-Alkyl-N-(C₇-C₁₆)-aralkylsulfamoyl, (C₁-C₁₀)-Alkyl-sulfonamido, N-((C₁-C₁₀)-alkyl)-(C₁-C₁₀)-alkylsulfonamido, (C₇-C₁₆)-Aralkylsulfonamido, oder mit N-((C₁-C₁₀)-alkyl-(C₇-C₁₆)-aralkylsulfonamido substituiert sind, wobei die Reste, die einen Arylrest enthalten ihrerseits am Aryl substituiert sein können durch 1 bis 5 gleiche oder verschiedene Reste aus der Reihe:
Hydroxy, Halogen, Cyano, Trifluormethyl, Nitro, Carboxy, (C₁-C₁₂)-Alkyl, (C₃-C₈)-Cycloalkyl, (C₆-C₁₂)-Aryl, (C₇-C₁₆)-Aralkyl, (C₁-C₁₂)-Alkoxy, (C₁-C₁₂)-Alkoxy-(C₁-C₁₂)alkyl, (C₁-C₁₂)-Alkoxy-(C₁-C₁₂)alkoxy, (C₆-C₁₂)-Aryloxy, (C₇-C₁₆)-Aralkyloxy, (C₁-C₈)-Hydroxyalkyl,
(C₁-C₁₂)-Alkylcarbonyl, (C₃-C₈)-Cycloalkylcarbonyl, (C₆-C₁₂)-Arylcarbonyl, (C₇-C₁₆)-Aralkylcarbonyl,
(C₁-C₁₂)-Alkoxycarbonyl, (C₁-C₁₂)-Alkoxy-(C₁-C₁₂)-alkoxycarbonyl, (C₆-C₁₂)- Aryloxycarbonyl, (C₇-C₁₆)-Aralkoxycarbonyl, (C₃-C₈)-Cycloalkoxycarbonyl, (C₂-C₁₂)-Alkenyloxycarbonyl, (C₂-C₁₂)-Alkinyloxycarbonyl,
(C₁-C₁₂)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy, (C₂-C₁₂)-Alkenylcarbonyloxy, (C₂-C₁₂)-Alkinylcarbonyloxy,
(C₁-C₁₂)-Alkoxycarbonyloxy, (C₁-C₁₂)-Alkoxy-(C₁-C₁₂)-alkoxycarbonyloxy, (C₆-C₁₂)-Aryloxycarbonyloxy, (C₇-C₁₆)-Aralkyloxycarbonyloxy, (C₃-C₈)-Cycloalkoxycarbonyloxy, (C₂-C₁₂)-Alkenyloxycarbonyloxy, (C₂-C₁₂)-Alkinyloxycarbonyloxy,
Carbamoyl, N-(C₁-C₁₂)-Alkylcarbamoyl, N, N-Di-(C₁-C₁₂)-alkylcarbamoyl, N-(C₃-C₈)-Cycloalkylcarbamoyl, N,N-Dicyclo-(C₃-C₈)-alkylcarbamoyl, N-(C₁-C₁₀)-Alkyl-N-(C₃-C₈)-cycloalkylcarbamoyl, N-((C₃-C₈)-Cycloalkyl-(C₁-C₆)-alkyl)carbamoyl, N-(C₁-C₆)-Alkyl-N-((C₃-C₈)-cycloalkyl-(C₁-C₆)-alkyl)carbamoyl, N-(+)-Dehydroabietylcarbamoyl, N-(C₁-C₆)-Alkyl-N-(+)-dehydroabietylcarbamoyl, N-(C₆-C₁₂)-Arylcarbamoyl, N-(C₇-C₁₆)-Aralkylcarbamoyl, N-(C₁-C₁₀)-Alkyl-N-(C₆-C₁₆)-arylcarbamoyl, N-(C₁-C₁₀)-Alkyl-N-(C₇-C₁₆)-aralkylcarbamoyl, N-((C₁-C₁₀)-Alkoxy-(C₁-C₁₀)-alkyl)carbamoyl, N-((C₆-C₁₆)-Aryloxy-(C₁-C₁₀)-alkyl)carbamoyl, N-((C₇-C₁₆)-Aralkyloxy-(C₁-C₁₀)-alkyl)carbamoyl, N-(C₁-C₁₀)-Alkyl-N-((C₁-C₁₀)-alkoxy-(C₁-C₁₀)-alkyl)carbamoyl, N-(C₁-C₁₀)-Alkyl-N-((C₆-C₁₂)-aryloxy-(C₁-C₁₀)-alkyl)carbamoyl, N-(C₁-C₁₀)-Alkyl-N-((C₇-C₁₆)-aralkyloxy-(C₁-C₁₀)-alkyl)carbamoyl, CON(CH₂)ₕ worin eine CH₂-Gruppe durch O, S, N-(C₁-C₈)Alkylimino, N-(C₃-C₈)-Cycloalkylimino, N-(C₃-C₈)-Cycloalkyl-(C₁-C₄)alkylimino, N-(C₆-C₁₂)-Arylimino, N-(C₇-C₁₆)-Aralkylimino oder N-(C₁-C₄)-Alkoxy-(C₁-C₆)-alkylimino ersetzt ein kann und h 3 bis 7 bedeutet,
Carbamoyloxy, N-(C₁-C₁₂)-Alkylcarbamoyloxy, N,N-Di-(C₁-C₁₂)-alkylcarbamoyloxy, N-(C₃-C₈)-Cycloalkylcarbamoyloxy, N-(C₆-C₁₆)-Arylcarbamoyloxy, N-(C₇-C₁₆)-Aralkylcarbamoyloxy, N-(C₁-C₁₀)-Alkyl-N-(C₆-C₁₂)-arylcarbamoyloxy, N-(C₁-C₁₀)-Alkyl-N-(C₇-C₁₆)-Aralkylcarbamoyloxy, N-((C₁-C₁₀)-alkyl))carbamoyloxy, N-((C₆-C₁₂)-Aryloxy-(C₁-C₁₀)-alkyl)-carbamoyloxy, N-((C₇-C₁₆)-Aralkyloxy-(C₁-C₁₀)-alkyl)carbamoyloxy, N-(C₁-C₁₀)-Alkyl-N-((C₁-C₁₀)-alkoxy-(C₁-C₁₀)-alkyl)carbamoyloxy, N-(C₁-C₁₀)-Alkyl-N-((C₆-C₁₂)-aryloxy-(C₁-C₁₀)-alkyl)carbamoyloxy, N-(C₁-C₁₀)-Alkyl-N-((C₇-C₁₆)-aralkyloxy-(C₁-C₁₀)-alkyl)carbamoyloxy,
Amino, (C₁-C₁₂)-Alkylamino, Di-(C₁-C₁₂)alkylamino, (C₃-C₈)-Cycloalkylamino, (C₃-C₁₂)-Alkenylamino, (C₃-C₁₂) Alkinylamino, N-(C₆-C₁₂)-Arylamino, N-(C₇-C₁₁)-Aralkylamino, N-Alkyl-Aralkylamino, N-Alkyl-Arylamino, (C₁-C₁₂)-Alkoxyamino, (C₁-C₁₂)-Alkoxy-N-(C₁-C₁₀)-alkylamino,
(C₁-C₁₂)-Alkanoylamino, (C₃-C₈)-Cycloalkanoylamino, (C₆-C₁₂)-Aroylamino, (C₇-C₁₆)-Aralkanoylamino, (C₁-C₁₂)-Alkanoyl-N-(C₁-C₁₀)-alkylamino, (C₃-C₈)-Cycloalkanoyl-N-(C₁-C₁₀)-alkylamino, (C₆-C₁₂)-Aroyl-N-(C₁-C₁₀)-alkylamino, (C₇-C₁₁)-Aralkanoyl-N-(C₁-C₁₀)-alkylamino,
(C₁-C₁₂)-Alkanoylamino-(C₁-C₈)-alkyl, (C₃-C₈)-Cycloalkanoylamino-(C₁-C₈)-alkyl, (C₆-C₁₂)-Aroylamino-(C₁-C₈)-alkyl, (C₇-C₁₆)-Aralkanoylamino-(C₁-C₈)-alkyl, Amno-(C₁-C₁₀)-alkyl, N-(C₁-C₁₀)alkylamino-(C₁-C₁₀)-alkyl, N,N-Di-(C₁-C₁₀)-alkylamino-(C₁-C₁₀)-alkyl, (C₃-C₈)-Cycloalkylamino-(C₁-C₁₀)-alkyl,
(C₁-C₁₂)-Alkylmercapto, (C₁-C₁₂)-Alkylsulfinyl, (C₁-C₁₂)-Alkylsulfonyl, (C₆-C₁₆)-Arylmercapto, (C₆-C₁₆)-Arylsulfinyl, (C₆-C₁₆)-Arylsulfonyl, (C₇-C₁₆)-Aralkylmercapto, (C₇-C₁₆)-Aralkylsulfinyl, (C₇-C₁₆)-Aralkylsulfonyl,
- R¹ und R⁴: eine Kette [CH₂]ₒ bilden können, in welcher eine oder zwei CH₂-Gruppen der gesättigten oder mit einer C = C-Doppelbindung ungesättigten Kette gegebenenfalls durch O, S, SO, SO₂ oder NR' ersetzt sind, o = 3, 4 oder 5 bedeutet und
- R^{y}: (C₆-C₁₂)-Aryl, (C₅-C₈)-Alkyl, (C₁-C₈)-Alkoxy-(C₁-C₈)alkyl, (C₇-C₁₂)-Aralkoxy-(C₁-C₈)alkyl, (C₆-C₁₂)Aryloxy-(C₁-C₈)-alkyl, (C₁-C₁₀)-Alkanoyl, ggf. substituiertes (C₇-C₁₆)-Aralkanoyl, ggf. substituiertes (C₆-C₁₂)-Aroyl bedeuten.
Steht Q in der Bedeutung von NR^{y}, so steht R⁴ in der Bedeutung von R^{z}, wobei R^{y} und R^{z} gleich oder verschieden sind und (C₆-C₁₂)-Aryl, (C₁-C₈)-Alkyl, (C₁-C₈)-Alkoxy-(C₁-C₈)-alkyl, (C₇-C₁₂)-Aralkoxy-(C₁-C₈)-alkyl, (C₆-C₁₂)-Aryloxy-(C₁-C₈)-alkyl, (C₁-C₁₀)-Alkanoyl, ggf. substituiertes (C₇-C₁₆)-Aralkanoyl, ggf. substituiertes (C₆-C₁₂)-Aroyl bedeuten, oder
R^{y} und R^{z} gemeinsam für -[CH₂]ₕ stehen, worin eine CH₂-Gruppe durch O, S, N-Acylimino oder N-(C₁-C₁₀)-Alkoxycarbonylimino ersetzt sein kann, und
f = 1 bis 8,
g = 0,1 bis (2f+1),
x = 0 bis 3,
h = 3 bis 6 bedeuten.

Unter Aryl-, Aryloxy-, Heteroaryl- bzw. Heteroaryloxyverbindungen versteht man insbesondere Phenyl-, Biphenyl- oder Naphthyl- bzw. unsubstituierte 5- und 6-gliedrige heteroaromatische Ringe mit 1, 2 oder 3 Stickstoff- und/oder Sauerstoff und/oder Schwefelatomen, wie Pyridyl-, Pyridazyl-, Pyrimidyl-, Pyrazyl-, Imidazolyl-, Triazolyl-, Thienyl-, Oxazolyl-, und Thiazolyl-Derivate, und deren benzoannellierte Derivate, unter Halogen Fluor, Chlor, Brom und Iod, insbesondere Fluor, Chlor und Brom.

Die Erfindung umfaßt weiterhin Salze der Verbindungen der allgemeinen Formel 1.

Die Salzbildung mit basischen Reagenzien kann ein-, zwei- oder dreifach an den aciden Gruppen der Verbindungen der Formel 1 erfolgen, insbesondere an den Resten B, R² und R⁴.

Zur Anwendung kommende Reagenzien sind beispielsweise Alkoholate, Hydroxide, Carbonate, Hydrogencarbonate, Hydrogenphosphate, Metallorganyle der Alkali- und Erdalkalielemente, der Elemente der 3. und 4. Hauptgruppe des Periodensystems und der Elemente der Übergangsmetalle.

Amine, ggf. 1 - bis 3-fach substituiert mit (C₁-C₈)-Hydroxyalkyl, (C₁-C₄)-Alkoxy-(C₁-C₈)-alkyl, Phenyl, Benzyl oder (C₁-C₈)-Alkyl, welches seinerseits 1- bis 3-fach substituiert sein kann mit Hydroxy oder (C₁-C₄)-Alkoxy,
beispielsweise Tromethan (Tris-Puffer), 2-Aminoethanol, 3-Aminopropanol, Hydroxylamin, Dimethylhydroxylamin, 2-Methoxyethylamin, 3-Ethoxypropylamin, und
basische Aminosäuren und -derivate, wie Aminosäureester, Histidin, Arginin und Lysin und deren Derivate, sowie
Arzneimittel, die eine basische Gruppe enthalten, wie beispielsweise ®Amilorid, ®Verapamil und Betablocker.

Die Erfindung betrifft weiterhin die Verbindungen gemäß Formel 1, zur Anwendung der Arzneimittel.

Bevorzugt sind Verbindungen der Formel 1, in der
Q = O,
X = O,
Y = N oder CR³,
m = 0 und 1
- A: (C₁-C₃)-Alkylen, das gegebenenfalls einfach oder zweifach substituiert ist mit Halogen, Cyano, Trifluormethyl, (C₁-C₆)-Alkyl, (C₁-C₆)-Hydroxyalkyl, (C₁-C₆)-Alkoxy, -O-[CH₂]ₓ-C_{f}H_{(2f+1-g)}F_{g} oder
- A: -CHR^{x}- bedeutet, wobei R^{x} einen der Substituenten des α-C-Atoms einer α-Aminosäure bedeutet, insbesondere einer natürlichen L-Aminosäure und ihres D-Isomeren,
- B: -CO₂V bedeutet, wobei
- V: den Rest eines Alkohols VOH bedeutet, worin V
(C₁-C₁₀)-Acyloxy-(C₁-C₆)-alkyl, vorzugsweise (C₁-C₁₀)-Alkanoyloxy-(C₁-C₆)-alkyl, Benzoyloxy-(C₁-C₆)-alkyl, Benzyloxycarbonyloxy-(C₁-C₆)-alkyl oder (C₁-C₆)-Alkoxycarbonyloxy-(C₁-C₆)-alkyl, oder
einen verzweigten, unverzweigten oder cyclischen aliphatischen (C₁-C₂₀)-Alkyl-Rest, oder
einen verzweigten oder unverzweigten (C₂-C₂₀)-Alkenyl-Rest, einen Retinyl-Rest, oder
einen (C₂-C₁₂)-Alkinyl-Rest, der jeweils eine oder mehrere C-C-Mehrfachbindungen enthalten kann, oder
einen (C₆-C₁₂)-Aryl-Rest, einen (C₇-C₁₁)-Aralkyl-Rest oder einen Heteroaryl- oder Heteroaralkyl-Rest bedeutet,
wobei die vorstehenden Reste einen oder zwei Substituenten aus der Reihe (C₁-C₈)-Alkyl, (C₃-C₈)-Cycloalkyl, Fluor, Chlor, Hydroxy, (C₁-C₆)-Alkoxy, (C₁-C₆)-Alkoxy-(C₁-C₆)alkoxy, (C₆-C₁₂)-Aryloxy, (C₇-C₁₂)-Aralkyloxy,
(C₁-C₈)-Alkylcarbonyl, (C₃-C₈)-Cycloalkylcarbonyl, (C₆-C₁₂)-Arylcarbonyl, (C₇-C₁₂)-Aralkylcarbonyl,
(C₁-C₈)-Alkoxycarbonyl, (C₁-C₆)-Alkoxy-(C₁-C₆)-alkoxycarbonyl, (C₆-C₁₂)-Aryloxycarbonyl, (C₇-C₁₂)-Aralkoxycarbonyl, (C₃-C₈)-Cycloalkoxycarbonyl,
(C₁-C₈)-Alkylcarbonyloxy, (C₃-C₈)-Cycloalkylcarbonyloxy, (C₆-C₁₂)-Arylcarbonyloxy, (C₇-C₁₂)-Aralkylcarbonyloxy,
(C₁-C₈)-Alkoxycarbonyloxy, (C₁-C₆)-Alkoxy-(C₁-C₆)-alkoxycarbonyloxy, (C₆-C₁₂)-Aryloxycarbonyloxy, (C₇-C₁₂)-Aralkyloxycarbonyloxy, (C₃-C₈)-Cycloalkoxycarbonyloxy,
Carbamoyl, N-(C₁-C₈)-Alkylcarbamoyl, N,N-Di-(C₁-C₈)-alkylcarbamoyl, N-(C₃-C₈)-Cycloalkylcarbamoyl,
N-((C₁-C₆)-Alkoxy-(C₁-C₆)-alkyl)carbamoyl,
Amino, (C₁-C₆)-Alkylamino, Di-(C₁-C₆)-alkylamino, (C₃-C₈)-Cycloalkylamino,N-(C₆-C₁₂)-Arylamino, N-(C₇-C₁₁)-Aralkylamino, N-(C₁-C₅)-Alkyl-(C₆-C₁₂)-arylamino,
(C₁-C₈)-Alkanoylamino, (C₃-C₈)-Cycloalkanoylamino, (C₆-C₁₂)-Aroylamino, (C₇-C₁₂)-Aralkanoylamino, (C₁-C₈)-Alkanoyl-N-(C₁-C₆)-alkylamino, (C₃-C₈)-Cycloalkanoyl-N-(C₁-C₆)-alkylamino, (C₆-C₁₂)-Aroyl-N-(C₁-C₆)-alkylamino, (C₇-C₁₁)-Aralkanoyl-N-(C₁-C₆)-alkylamino tragen können, und
wobei die Reste, die einen Arylrest enthalten, insbesondere substituiert sind mit bis zu 3 Substituenten aus der Reihe
Hydroxy, Fluor, Chlor, Cyano, Trifluormethyl, (C₁-C₆)-Alkyl, (C₃-C₈)-Cycloalkyl, (C₁-C₈)-Alkoxy,
(C₁-C₆)-Alkylcarbonyl, (C₃-C₈)-Cycloalkylcarbonyl,
(C₁-C₆)-Alkoxycarbonyl, (C₃-C₈)-Cycloalkoxycarbonyl,
(C₁-C₆)-Alkylcarbonyloxy, (C₃-C₈)-Cycloalkylcarbonyloxy,
(C₁-C₆)-Alkoxycarbonyloxy, (C₃-C₈)-Cycloalkoxycarbamoyloxy,
Carbamoyl, N-(C₁-C₆)-Alkylcarbamoyl, N,N-Di-(C₁-C₆)-alkylcarbamoyl, N-(C₃-C₈)-Cycloalkylcarbamoyl,
N-((C₁-C₆)-Alkoxy-(C₁-C₆)-alkyl)carbamoyl,
N-(C₁-C₆)-Alkyl-N-((C₁-C₆)-alkoxy-(C₁-C₆)-alkyl)carbamoyl,
Carbamoyloxy, N-(C₁-C₆)-Alkylcarbamoyloxy, N,N-Di-(C₁-C₆)-alkylcarbamoyloxy, N-(C₃-C₈)-Cycloalkylcarbamoyloxy,
(C₁-C₆)-Alkanoylamino, (C₃-C₈)-Cycloalkanoylamino,
(C₁-C₆)Alkylmercapto, (C₁-C₆)-Alkylsulfinyl, (C₁-C₆)-Alkylsulfonyl und
- R¹ und R³: Wasserstoff bedeuten,
- R²: einen Rest -CO-NR⁵R⁶ bedeutet, wobei
- R⁵: Wasserstoff, (C₁-C₆)-Alkyl oder eine N-Schutzgruppe wie (C₁-C₈)-Alkanoyl, (C₁-C₆)-Alkylcarbamoyl, (C₁-C₆)-Alkoxycarbonyl, Benzyloxycarbonyl, (C₁-C₁₀)-Acyloxy-(C₁-C₆)-alkyl, vorzugsweise (C₁-C₁₀)-Alkanoyloxy-(C₁-C₆)-alkyl, Benzoyloxy-(C₁-C₆)-alkyl, Benzyloxycarbonyloxy-(C₁-C₆)-alkyl oder (C₁-C₆)-Alkoxycarbonyloxy-(C₁-C₆)-alkyl, ein 1, 2, 3 oder 4-wertiges physiologisch verwendbares Kation, insbesondere Na⊕, K⊕, Mg²⊕, Ca²⊕, Al³⊕ oder ein Ammoniumion, ggf. 1-3fach substituiert mit (C₁-C₈)-Hydroxyalkyl, (C₁-C₄)-Alkoxy-(C₁-C₈)-alkyl, Phenyl, Benzyl oder (C₁-C₈)-Alkyl, welches seinerseits 1-3fach substituiert sein kann mit Hydroxy oder (C₁-C₄)-Alkoxy, oder ein Kation eines basischen Aminosäurederivates bedeutet,
- R⁶: einen Rest der Formel I, ausgenommen -SO₂H, bedeutet

-G-K-[CU]ᵣ-D-W (I)

in welchem
- G: -SO₂- oder -CO- bedeutet,
- K: eine Bindung,
- C: eine Bindung oder
einen verzweigten oder unverzweigten aliphatischen (C₁-C₁₂)-Alkandiyl-rest, oder
einen verzweigten oder unverzweigten (C₂-C₁₂)-Alkendiyl-Rest, einen (C₂-C₁₂)-Alkindiyl-Rest oder einen (C₂-C₁₂)-Alkenindiyl-Rest bedeutet, der eine oder mehrere C-C-Mehrfachbindungen enthalten kann,
- U: eine Bindung, Wasserstoff oder einen Rest aus der Reihe folgender Heteroatomgruppierungen bedeutet
-(CO)NR-, -NR(CO)-, -O-,-SO-, -SO₂-, worin R (C₂-C₃)-Alkyl oder Wasserstoff bedeutet,
- r: 1 oder 2 ist,
- D: eine Bindung, Wasserstoff oder
einen verzweigten oder unverzweigten aliphatischen (C₁-C₈)-Alkandiyl-Rest,
einen verzweigten oder unverzweigten (C₂-C₈)-Alkendiyl-Rest oder (C₂-C₈)-Alkindiyl-Rest bedeutet und
- W: eine Bindung, Wasserstoff oder
einen (C₃-C₁₀) cycloaliphatischen Alkyl-, Alkenyl-, Alkinyl-oder Alkeninyl-Rest,
einen (C₆-C₁₆)-Arylrest oder einen 5- oder 6-gliedrigen Heteroarylrest bedeutet, wobei mindestens eine der Variablen C, D oder W nicht eine Bindung bedeuten und U nur dann in der Bedeutung einer Heteratomgruppierung steht, wenn C nicht eine Bindung bedeutet oder wenn D und/oder W nicht eine Bindung bedeuten und
- C, D und/oder W,: sofern diese keine Bindung oder Wasserstoff bedeuten, vorzugsweise ihrerseits substituiert sind durch eine Kombination von bis zu 5 gleichen oder verschiedenen Substituenten aus der Reihe
Hydroxy, Halogen, Cyano, Trifluormethyl, Nitro, Carboxy, (C₁-C₁₂)-Alkyl, (C₃-C₈)-Cycloalkyl, (C₃-C₈)-Cycloalkyl-(C₁-C₁₂)-alkyl, (C₃-C₈)-Cycloalkoxy, (C₃-C₈)-Cycloalkyl-(C₁-C₁₂)-alkoxy, (C₃-C₈)-Cycloalkyloxy-(C₁-C₁₂)-alkyl, (C₃-C₈)-Cycloalkyloxy-(C₁-C₁₂)-alkoxy, (C₃-C₈)-Cycloalkyl-(C₁-C₈)-alkyl-(C₁-C₆)-alkoxy, (C₃-C₈)-Cycloalkyl-(C₁-C₈)-alkoxy-(C₁-C₆)-alkyl, (C₃-C₈)-Cycloalkyloxy-(C₁-C₈)-alkoxy-(C₁-C₆)-alkyl, (C₃-C₈)-Cycloalkoxy-(C₁-C₈)-alkoxy-(C₁-C₈)-alkoxy, (C₆-C₁₂)-Aryl, (C₇-C₁₆)-Aralkyl, (C₂-C₁₂)-Alkenyl, (C₂-C₁₂)-Alkinyl, (C₁-C₁₂)-Alkoxy, (C₁-C₁₂)-Alkoxy-(C₁-C₁₂)alkyl, (C₁-C₁₂)-Alkoxy-(C₁-C₁₂)alkoxy, (C₁-C₁₂)-Alkoxy-(C₁-C₈)-alkoxy-(C₁-C₈)-alkyl, (C₆-C₁₂)-Aryloxy, (C₇-C₁₆)-Aralkyloxy, (C₆-C₁₂)-Aryloxy-(C₁-C₆)-alkoxy, (C₇-C₁₆)-Aralkoxy-(C₁-C₆)-alkoxy, (C₁-C₈)-Hydroxyalkyl, (C₆-C₁₆)-Aryloxy-(C₁-C₈)-alkyl, (C₇-C₁₆)-Aralkoxy-(C₁-C₈)-alkyl, (C₆-C₁₂)-Aryloxy-(C₁-C₈)-alkoxy-(C₁-C₆)-alkyl, (C₇-C₁₂)-Aralkyloxy-(C₁-C₈)-alkoxy-(C₁-C₆)-alkyl, -O-[CH₂₋]ₓC_{f}H_{(2f+1-g)}F_{g}, -OCF₂Cl, -OCF₂-CHFCl,
(C₁-C₁₂)-Alkylcarbonyl, (C₃-C₈)-Cycloalkylcarbonyl, (C₆-C₁₂)-Arylcarbonyl, (C₇-C₁₆)-Aralkylcarbonyl, Cinnamoyl, (C₂-C₁₂) Alkenylcarbonyl, (C₂-C₁₂)-Alkinylcarbonyl,
(C₁-C₁₂)-Alkoxycarbonyl, (C₁-C₁₂)-Alkoxy-(C₁-C₁₂)-alkoxycarbonyl, (C₆-C₁₂)- Aryloxycarbonyl, (C₇-C₁₆)-Aralkoxycarbonyl, (C₃-C₁₀)-Cycloalkoxycarbonyl, (C₂-C₁₂)-Alkenyloxycarbonyl, (C₂-C₁₂)-Alkinyloxycarbonyl,
(C₁-C₁₂)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy, (C₂-C₁₂)-Alkenylcarbonyloxy, (C₂-C₁₂)-Alkinylcarbonyloxy,
Carbamoyl, N-(C₁-C₁₂)-Alkylcarbamoyl, N, N-Di-(C₁-C₁₂)-alkylcarbamoyl, N-(C₃-C₈)-Cycloalkylcarbamoyl, N,N-Dicyclo-(C₃-C₈)-alkylcarbamoyl, N-(C₁-C₁₀)-Alkyl-N-(C₃-C₈)-cycloalkylcarbamoyl, N-((C₃-C₈)-Cycloalkyl-(C₁-C₆)-alkyl)carbamoyl, N-(C₁-C₆)-Alkyl-N-((C₃-C₈)-cycloalkyl-(C₁-C₆)-alkyl)carbamoyl, N-(+)-Dehydroabietylcarbamoyl, N-(C₁-C₆)-Alkyl-N-(+)-dehydroabietylcarbamoyl, N-(C₆-C₁₂)-Arylcarbamoyl, N-(C₇-C₁₆)-Aralkylcarbamoyl, N-(C₁-C₁₀)-Alkyl-N-(C₆-C₁₆)-arylcarbamoyl, N-(C₁-C₁₀)-Alkyl-N-(C₇-C₁₆)-aralkylcarbamoyl, N-((C₁-C₁₀)-Alkoxy-(C₁-C₁₀)alkyl)carbamoyl, N-((C₆-C₁₆)-Aryloxy-(C₁-₁₀)-alkyl)carbamoyl, N-((C₇-C₁₆)-Aralkyloxy-(C₁-C₁₀)-alkyl)carbamoyl, N-(C₁-C₁₀)-Alkyl-N-((C₁-C₁₀)-alkoxy-(C₁-C₁₀)-alkyl)carbamoyl, N-(C₁-C₁₀)-Alkyl-N-((C₆-C₁₂)-aryloxy-(C₁-C₁₀)-alkyl)carbamoyl, N-(C₁-C₁₀)-Alkyl-N-((C₇-C₁₆)-aralkyloxy-(C₁-C₁₀)alkyl)carbamoyl, CON(CH₂)ₕ, worin eine CH₂-Gruppe durch O, S, N-(C₁-C₈)Alkylimino, N-(C₃-C₈)-Cycloalkylimino, N-(C₃-C₈)-Cycloalkyl-(C₁-C₄)alkylimino, N-(C₆-C₁₂)-Arylimino, N-(C₇-C₁₆)-Aralkylimino oder N-(C₁-C₄)-Alkoxy-(C₁-C₆)-alkylimino ersetzt ein kann und h 3 bis 6 bedeutet, oder mit
einem Carbamoyl-Rest der allgemeinen Formel II 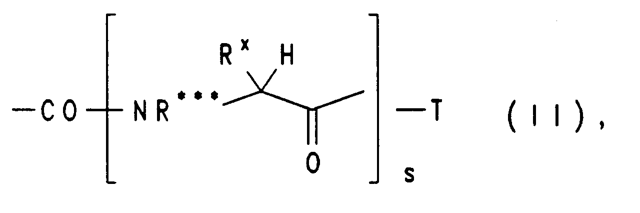 worin
- R^{x}: der Substituent einer α-Aminosäure bedeutet, zu denen die L-und D-Aminosäuren zählen,
- s: 1, 2, 3, 4 oder 5 und
- T: OH, OR oder NR*R** bedeutet, wobei
- R***: Wasserstoff oder (C₁-C₄)-Alkyl,
- R* und R**: gleich oder verschieden sind und Wasserstoff, (C₆-C₁₂)-Aryl, (C₇-C₁₁)-Aralkyl, (C₁-C₈)-Alkyl, (C₃-C₈)-Cycloalkyl, (+)-Dehydroabietyl, (C₁-C₈)-Alkoxy-(C₁-C₈)alkyl, (C₇-C₁₂)-Aralkoxy-(C₁-C₈)alkyl, (C₆-C₁₂)-Aryloxy-(C₁-C₈)-alkyl, (C₁-C₁₀)-Alkanoyl, ggf. substituiertes (C₇-C₁₆)-Aralkanoyl, ggf. substituiertes (C₆-C₁₂)-Aroyl bedeuten, oder
- R* und R**: gemeinsam für -[CH₂]ₕ stehen, worin eine CH₂ Gruppe durch O, S, SO, SO₂, N-Acylimino, N-(C₁-C₁₀)-Alkoxycarbonylimino, N-(C₁-C₈)-Alkylimino, N-(C₃-C₈)-Cycloalkylimino, N-(C₃-C₈)-Cycloalkyl-(C₁-C₄)alkylimino, N-(C₆-C₁₂)-Arylimino, N-(C₇-C₁₆)-Aralkylimino oder N-(C₁-C₄)-Alkoxy-(C₁-C₆)-alkylimino ersetzt sein kann und h 3 bis 7 bedeutet, oder mit
Carbamoyloxy, N-(C₁-C₁₂)-Alkylcarbamoyloxy, N,N-Di-(C₁-C₁₂)-alkylcarbamoyloxy, N-(C₃-C₈)-Cycloalkylcarbamoyloxy, N-(C₆-C₁₂)-Arylcarbamoyloxy, N-(C₇-C₁₆)-Aralkylcarbamoyloxy, N-(C₁-C₁₀)-Alkyl-N-(C₆-C₁₂)arylcarbamoyloxy, N-(C₁-C₁₀)-Alkyl-N-(C₇-C₁₆)-Aralkylcarbamoyloxy,N-((C₁-C₁₀)-alkyl))carbamoyloxy, N-((C₆-C₁₂)-Aryloxy-(C₁-C₁₀)alkyl)carbamoyloxy, N-((C₇-C₁₆)-Aralkyloxy-(C₁-C₁₀)alkyl)carbamoyloxy, N-(C₁-C₁₀)-Alkyl-N-((C₁-C₁₀)-alkoxy-(C₁-C₁₀)-alkyl)carbamoyloxy, N-(C₁-C₁₀)-Alkyl-N-((C₆-C₁₂)-aryloxy-(C₁-C₁₀)-alkyl)carbamoyloxy, N-(C₁-C₁₀)-Alkyl-N-((C₇-C₁₆)-aralkyloxy-(C₁-C₁₀)alkyl)carbamoyloxy,
Amino, (C₁-C₁₂)-Alkylamino, Di-(C₁-C₁₂)alkylamino, (C₃-C₈)-Cycloalkylamino, (C₃-C₁₂)-Alkenylamino, (C₃-C₁₂)-Alkinylamino, N-(C₆-C₁₂)-Arylamino, N-(C₇-C₁₁)-Aralkylamino, N-Alkyl-Aralkylamino, N-Alkyl-Arylamino, (C₁-C₁₂)-Alkoxyamino, (C₁-C₁₂)-Alkoxy-N-(C₁-C₁₀)-alkylamino,
(C₁-C₁₂)-Alkanoylamino, (C₃-C₈)-Cycloalkanoylamino, (C₆-C₁₂)-Aroylamino, (C₇-C₁₆)-Aralkanoylamino, (C₁-C₁₂)-Alkanoyl-N-(C₁-C₁₀)-alkylamino, (C₃-C₈)-Cycloalkanoyl-N-(C₁-C₁₀)-alkylamino, (C₆-C₁₂)-Aroyl-N-(C₁-C₁₀)alkylamino, (C₇-C₁₁)-Aralkanoyl-N-(C₁-C₁₀)-alkylamino,
(C₁-C₁₂)-Alkanoylamino-(C₁-C₈)-alkyl, (C₃-C₈)-Cycloalkanoylamino-(C₁-C₈)-alkyl, (C₆-C₁₂)-Aroylamino-(C₁-C₈)-alkyl, (C₇-C₁₆)-Aralkanoylamino-(C₁-C₈)-alkyl, Amino-(C₁-C₁₀)-alkyl, N-(C₁-C₁₀)-alkylamino-(C₁-C₁₀)-alkyl, N,N-Di(C₁-C₁₀)-alkylamino-(C₁-C₁₀)alkyl, (C₃-C₈)-Cycloalkylamino-(C₁-C₁₀)-alkyl, (C₁-C₁₂)-Alkylmercapto, (C₁-C₁₂)-Alkylsulfinyl, (C₁-C₁₂)-Alkylsulfonyl, (C₆-C₁₂)-Arylmercapto, (C₆-C₁₂)-Arylsulfinyl, (C₆-C₁₂)-Arylsulfonyl, (C₇-C₁₆)-Aralkylmercapto, (C₇-C₁₆)-Aralkylsulfinyl, (C₇-C₁₆)-Aralkylsulfonyl, bedeuten, wobei die Reste, die einen Arylrest enthalten ihrerseits am Aryl substituiert sein können durch 1 bis 5 gleiche oder verschiedene Reste aus der Reihe:
Hydroxy, Halogen, Cyano, Trifluormethyl, Nitro, Carboxy, (C₁-C₁₂)-Alkyl, (C₃-C₈)-Cycloalkyl, (C₆-C₁₂)-Aryl, (C₇-C₁₆)-Aralkyl, (C₁-C₁₂)-Alkoxy, (C₁-C₁₂)-Alkoxy-(C₁-C₁₂)alkyl, (C₁-C₁₂)-Alkoxy-(C₁-C₁₂)alkoxy, (C₆-C₁₂)-Aryloxy, (C₇-C₁₆)-Aralkyloxy, (C₁-C₈)-Hydroxyalkyl, -O-[CH₂]ₓC_{f}H_{(2f+1-g)}F_{g}, OCF₂Cl, OCF₂-CHFCl,
(C₁-C₁₂)-Alkylcarbonyl, (C₃-C₈)-Cycloalkylcarbonyl, (C₆-C₁₂)-Arylcarbonyl, (C₇-C₁₆)-Aralkylcarbonyl,
(C₁-C₁₂)-Alkoxycarbonyl, (C₁-C₁₂)-Alkoxy-(C₁-C₁₂)-alkoxycarbonyl, (C₆-C₁₂)- Aryloxycarbonyl, (C₇-C₁₆)-Aralkoxycarbonyl, (C₃-C₈)-Cycloalkoxycarbonyl, (C₂-C₁₂)-Alkenyloxycarbonyl, (C₂-C₁₂)-Alkinyloxycarbonyl,
(C₁-C₁₂)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy, (C₂-C₁₂)-Alkenylcarbonyloxy, (C₂-C₁₂)-Alkinylcarbonyloxy,
Carbamoyl, N-(C₁-C₁₂)-Alkylcarbamoyl, N, N-Di-(C₁-C₁₂)-alkylcarbamoyl, N-(C₃-C₈)-Cycloalkylcarbamoyl, N,N-Dicyclo-(C₃-C₈)-alkylcarbamoyl, N-(C₁-C₁₀)-Alkyl-N-(C₃-C₈)-cycloalkylcarbamoyl, N-((C₃-C₈)-Cycloalkyl-(C₁-C₆)-alkyl)carbamoyl, N-(C₁-C₆)-Alkyl-N-((C₃-C₈)-cycloalkyl-(C₁-C₆)-alkyl)carbamoyl, N-(+)-Dehydroabietylcarbamoyl, N-(C₁-C₆)-Alkyl-N-(+)-dehydroabietylcarbamoyl, N-(C₆-C₁₂)-Arylcarbamoyl, N-(C₇-C₁₆)-Aralkylcarbamoyl, N-(C₁-C₁₀)-Alkyl-N-(C₆-C₁₆)-arylcarbamoyl, N-(C₁-C₁₀)-Alkyl-N-(C₇-C₁₆)-aralkylcarbamoyl, N-((C₁-C₁₀)-Alkoxy-(C₁-C₁₀)-alkyl)carbamoyl, N-((C₆-C₁₆)-Aryloxy-(C₁-C₁₀)-alkyl)carbamoyl, N-((C₇-C₁₆)-Aralkyloxy-(C₁-C₁₀)alkyl)carbamoyl,
N-(C₁-C₁₀)-Alkyl-N-((C₁-C₁₀)-alkoxy-(C₁-C₁₀)-alkyl)carbamoyl, N-(C₁-C₁₀)-Alkyl-N-((C₆-C₁₂)-aryloxy-(C₁-C₁₀)-alkyl)carbamoyl, N-(C₁-C₁₀)-Alkyl-N-((C₇-C₁₆)-aralkyloxy-(C₁-C₁₀)-alkyl)carbamoyl, CON(CH₂)ₕ worin eine CH₂-Gruppe durch O, S, N-(C₁-C₈)-Alkylimino, N-(C₃-C₈)-Cycloalkylimino, N-(C₃-C₈)-Cycloalkyl-(C₁-C₄)-alkylimino, N-(C₆-C₁₂)-Arylimino, N-(C₇-C₁₆)-Aralkylimino oder N-(C₁-C₄)-Alkoxy-(C₁-C₆)-alkylimino ersetzt ein kann und h 3 bis 6 bedeutet,
Amino, (C₁-C₁₂)-Alkylamino, Di-(C₁-C₁₂)alkylamino, (C₃-C₈)-Cycloalkylamino, (C₃-C₁₂)-Alkenylamino, (C₃-C₁₂) Alkinylamino, N-(C₆-C₁₂)-Arylamino, N-(C₇-C₁₁)-Aralkylamino, N-Alkyl-Aralkylamino, N-Alkyl-Arylamino, (C₁-C₁₂)-Alkoxyamino, (C₁-C₁₂)-Alkoxy-N-(C₁-C₁₀)-alkylamino,
(C₁-C₁₂)-Alkanoylamino, (C₃-C₈)-Cycloalkanoylamino, (C₆-C₁₂)-Aroylamino, (C₇-C₁₆)-Aralkanoylamino, (C₁-C₁₂)-Alkanoyl-N-(C₁-C₁₀)-alkylamino, (C₃-C₈)-Cycloalkanoyl-N-(C₁-C₁₀)-alkylamino, (C₆-C₁₂)-Aroyl-N-(C₁-C₁₀)alkylamino, (C₇-C₁₁)-Aralkanoyl-N-(C₁-C₁₀)-alkylamino,
(C₁-C₁₂)-Alkanoylamino-(C₁-C₈)-alkyl, (C₃-C₈)-Cycloalkanoylamino-(C₁-C₈)-alkyl, (C₆-C₁₂)-Aroylamino-(C₁-C₈)-alkyl, (C₇-C₁₆)-Aralkanoylamino-(C₁-C₈)-alkyl, Amino-(C₁-C₁₀)-alkyl, N-(C₁-C₁₀)-alkylamino-(C₁-C₁₀)alkyl, N,N-Di-(C₁-C₁₀)-alkylamino-(C₁-C₁₀)alkyl, (C₃-C₈)-Cycloalkylamino-(C₁-C₁₀)alkyl,
(C₁-C₁₂)-Alkylmercapto, (C₁-C₁₂)-Alkylsulfinyl, (C₁-C₁₂)-Alkylsulfonyl, (C₆-C₁₆)-Arylmercapto, (C₆-C₁₆)-Arylsulfinyl, (C₆-C₁₆)-Arylsulfonyl, (C₇-C₁₆)-Aralkylmercapto, (C₇-C₁₆)-Aralkylsulfinyl, (C₇-C₁₆)-Aralkylsulfonyl,
- R⁴: einen verzweigten oder unverzweigten (C₇-C₂₀)-Alkylrest, der bis zu 3 C-C-Mehrfachbindungen enthalten kann, oder (C₁-C₁₂)-Alkoxy-(C₁-C₈)-alkyl, (C₁-C₁₂)-Alkoxy-(C₁-C₈)-alkoxy-(C₁-C₈)-alkyl, [CH₂]ₓ C_{f}H_{(2f+1-g)}F_{g}, oder (C₆-C₁₂)-Aryl, (C₇-C₁₁)-Aralkyl, Heteroaryl, Heteroaralkyl, (C₃-C₈)-Cycloalkyl, (C₃-C₈)-Cycloalkyl-(C₁-C₆)-alkyl, (C₆-C₁₄)-Aryloxy-(C₁-C₈)-alkyl, Heteroaryloxy-(C₁-C₈)-alkyl, (C₃-C₈)-Cycloalkyloxy-(C₁-C₈)-alkyl,(C₇-C₁₆)-Aralkyloxy-(C₁-C₈)-alkyl, Heteroaralkyloxy-(C₁-C₈)-alkyl,
(C₆-C₁₄)-Aryloxy-(C₁-C₈)-alkoxy-(C₁-C₈)-alkyl, Heteroalkyl-(C₁-C₈)-alkoxy-(C₁-C₈)-alkyl, (C₃-C₈)-Cycloalkoxy-(C₁-C₈)-alkoxy-(C₁-C₆)-alkyl,
(C₇-C₁₆)-Aralkyloxy-(C₁-C₈)-alkoxy-(C₁-C₈)alkyl, Heteroaralkyloxy-(C₁-C₈)-alkoxy-(C₁-C₈)-alkyl, (C₃-C₈)-Cycloalkyl-(C₁-C₆)-alkoxy-(C₁-C₆)-alkyl bedeutet, oder
einen Rest der Formel Z bedeutet,

[CH₂]ᵥ-[O]_{w}-[CH₂]ₜ-E (Z),

worin E einen substituierten Phenylrest der Formel F 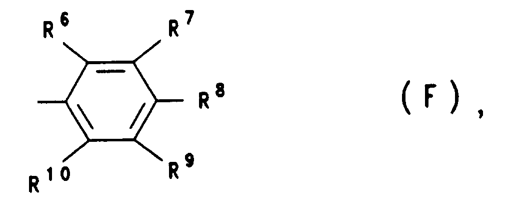
oder einen substituierten Heteroaryl-Rest, oder einen substituierten (C₃-C₈)-Cycloalkylrest bedeutet und
wobei
v = 0, 1, 2, 3, 4, 5, 6, w = 0,1 und t = 0, 1, 2, 3, mit der Einschränkung, daß v ungleich 0 ist, falls w = 1 ist, bedeutet und R⁶, R⁷, R⁸, R⁹ und R¹⁰ gleich oder verschieden sind und Wasserstoff, Halogen, Cyano, Nitro, Trifluormethyl, (C₁-C₆)-Alkyl, (C₃-C₈-Cycloalkyl, (C₁-C₆)-Alkoxy, -O-[CH₂₋]ₓC_{f}H_{(2f+1-g)}F_{g}, -OCF₂Cl, -O-CF₂-CHFCl, (C₁-C₆)-Alkylmercapto, (C₁-C₆)-Hydroxyalkyl, (C₁-C₆)-Alkoxy-(C₁-C₆)-alkoxy, (C₁-C₆)-Alkoxy-(C₁-C₆)-alkyl, (C₁-C₆)-Alkylsulfinyl, (C₁-C₆)-Alkylsulfonyl, (C₁-C₆)-Alkylcarbonyl, (C₁-C₈)-Alkoxycarbonyl, Carbamoyl, N-(C₁-C₈)-Alkylcarbamoyl, N,N-Di-(C₁-C₈)-alkylcarbamoyl, gegebenenfalls mit Fluor, Chlor, Brom, Trifluormethyl und (C₁-C₆)-Alkoxy-substituiertes (C₇-C₁₁)-Aralkylcarbamoyl, N-(C₃-C₈)-Cycloalkylcarbamoyl, N-(C₃-C₈)-Cycloalkyl-(C₁-C₄)-alkylcarbamoyl, (C₁-C₆)-Alkylcarbonyloxy, Phenyl, Benzyl, Phenoxy, Benzyloxy, NR'R'', wie Amino, Anilino, N-Methylanilino, Phenylmercapto, Phenylsulfonyl, Phenylsulfinyl, Sulfamoyl, N-(C₁-C₈)-Alkylsulfamoyl oder N,N-Di-(C₁-C₈)-alkylsulfamoyl bedeuten, oder zwei benachbarte Substituenten gemeinsam eine Kette -[CH₂₋]ₙ oder -CH = CH-CH = CH- bedeuten, wobei eine CH₂-Gruppe der Kette gegebenenfalls durch O, S, SO, SO₂ oder NR' ersetzt ist wobei
- R' und R'': gemeinsam für -[CH₂]ₕ₋ stehen, worin eine CH₂-Gruppe durch O, S, N-(C₁-C₄)-Alkanoylimino oder N-(C₁-C₄)-Alkoxycarbonylimino ersetzt sein kann, und
f = 1 bis 8,
g = 0, 1 bis (2f+1),
h = 3 bis 6,
x = 0 bis 3, und
n = 3 oder 4 ist.

Besonders bevorzugt sind Verbindungen der Formel 1, in der
Q = O,
X = O,
Y = CR³,
m = O,
- A: -CHR^{x}-, wobei R^{x} den Substituenten des α-C-Atoms einer α-Aminosäure bedeutet, insbesondere einer natürlichen L-Aminosäure oder ihr D-Isomeres,
- B =: CO₂V bedeutet, wobei
- V: den Rest eines Alkohols VOH bedeutet und für einen verzweigten, unverzweigten oder cyclischen aliphatischen (C₁-C₉)-Alkyl-Rest, einen (C₃-C₈)-Cycloalkyl-(C₁-C₄)-alkylrest,
einen verzweigten oder unverzweigten (C₂-C₈)-Alkenylrest oder (C₂-C₈)-Alkinylrest, einen Phenyl-, Benzyl-, Phenethyl-, Phenylpropyl- oder Phenylbutylrest steht,
wobei die vorstehenden Reste einen Substituenten aus der Reihe Fluor, Chlor, Hydroxy, (C₁-C₄)-Alkoxy, (C₁-C₆)-Alkylcarbonyloxy, (C₃-C₈)-Cycloalkylcarbonyloxy, Benzoyloxy, Phenylalkylcarbonyloxy enthalten,
- R¹ und R³: Wasserstoff bedeuten,
- R²: einen Rest -CONR⁵R⁶ bedeutet, wobei
- R⁵: Wasserstoff, (C₁-C₃)-Alkyl, (C₁-C₄)-Alkanoyl oder ein 1, 2 oder 3-wertiges physiologisch verwendbares Kation, insbesondere Na⊕, K⊕, Mg²⊕, Ca²⊕ oder ein Ammoniumion bedeutet, ggf. 1-3fach substituiert mit (C₁-C₈)-Hydroxyalkyl, (C₁-C₄)-Alkoxy-(C₁-C₈)-alkyl, Phenyl, Benzyl oder (C₁-C₈)-Alkyl, welches seinerseits 1-3fach substituiert sein kann mit Hydroxy und/oder (C₁-C₄)-Alkoxy,
- R⁶: einen Rest der Formel I, ausgenommen -SO₂H, bedeutet,

-G-K-[C-U]ᵣ-D-W (I)

in welchem
- G: -SO₂- bedeutet,
- K: eine Bindung,
- C: eine Bindung oder
einen (C₁-C₆)-Alkandiyl-Rest bedeutet,
- U: eine Bindung, Wasserstoff oder -O- bedeutet,
- r: 1 ist,
- D: eine Bindung, Wasserstoff oder
einen unverzweigten aliphatischen (C₁-C₈)-Alkandiyl-Rest bedeutet, und
- W: eine Bindung, Wasserstoff, einen (C₆-C₁₂)-Arylrest oder einen 5-oder 6-gliedrigen Heteroarylrest bedeutet, wobei mindestens eine der Variablen C oder D oder W nicht eine Bindung bedeuten und U nur dann in der Bedeutung einer Heteratomgruppierung steht, wenn C nicht eine Bindung bedeutet oder wenn D und/oder W nicht eine Bindung bedeuten und
- C, D und/oder W,: sofern diese keine Bindung oder Wasserstoff bedeuten, vorzugsweise ihrerseits substituiert sind mit bis zu 3 gleichen oder verschiedenen Substituenten aus der Reihe
Hydroxy, Halogen, Cyano, Trifluormethyl, Nitro, Carboxy, (C₁-C₁₂)-Alkyl, (C₃-C₈)-Cycloalkyl, (C₃-C₈)-Cycloalkyl-(C₁-C₁₂)-alkyl, (C₃-C₈)-Cycloalkoxy, (C₃-C₈)-Cycloalkyl-(C₁-C₁₂)-alkoxy, (C₃-C₈)-Cycloalkyloxy-(C₁-C₁₂)-alkyl, (C₃-C₈)-Cycloalkyloxy-(C₁-C₁₂)-alkoxy, (C₃-C₈)-Cycloalkyl-(C₁-C₈)-alkyl-(C₁-C₆)-alkoxy, (C₃-C₈)-Cycloalkyl-(C₁-C₈)-alkoxy-(C₁-C₆)-alkyl, (C₃-C₈)-Cycloalkyloxy-(C₁-C₈)-alkoxy-(C₁-C₆)-alkyl, (C₃-C₈)-Cycloalkoxy-(C₁-C₈)-alkoxy-(C₁-C₈)-alkoxy, (C₆-C₁₂)-Aryl, (C₇-C₁₆)-Aralkyl, (C₂-C₁₂)-Alkenyl, (C₂-C₁₂)-Alkinyl, (C₁-C₁₂)-Alkoxy, (C₁-C₁₂)-Alkoxy-(C₁-C₁₂)alkyl, (C₁-C₁₂)-Alkoxy-(C₁-C₁₂)alkoxy, (C₁-C₁₂)-Alkoxy-(C₁-C₈)-alkoxy-(C₁-C₈)-alkyl, (C₆-C₁₂)-Aryloxy, (C₇-C₁₆)-Aralkyloxy, (C₆-C₁₂)-Aryloxy-(C₁-C₆)-alkoxy, (C₇-C₁₆)-Aralkoxy-(C₁-C₆)-alkoxy, (C₁-C₈)-Hydroxyalkyl, (C₆-C₁₆)-Aryloxy-(C₁-C₈)-alkyl, (C₇-C₁₆)-Aralkoxy-(C₁-C₈)-alkyl, (C₆-C₁₂)-Aryloxy-(C₁-C₈)-alkoxy-(C₁-C₆)-alkyl, (C₇-C₁₂)-Aralkyloxy-(C₁-C₈)-alkoxy-(C₁-C₆)-alkyl, -O-[CH₂₋]ₓC_{f}H_{(2f+1-g)}F_{g}, (C₁-C₁₂)-Alkoxycarbonyl, (C₁-C₁₂)-Alkoxy-(C₁-C₁₂)-alkoxycarbonyl, (C₆-C₁₂)- Aryloxycarbonyl, (C₇-C₁₆)-Aralkoxycarbonyl, (C₃-C₈)-Cycloalkoxycarbonyl,
(C₁-C₁₂)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy,
Carbamoyl, N-(C₁-C₁₂)-Alkylcarbamoyl, N, N-Di-(C₁-C₁₂)-alkylcarbamoyl, N-(C₃-C₈)-Cycloalkylcarbamoyl, N,N-Dicyclo-(C₃-C₈)-alkylcarbamoyl, N-(C₁-C₁₀)-Alkyl-N-(C₃-C₈)-cycloalkylcarbamoyl, N-((C₃-C₈)-Cycloalkyl-(C₁-C₆)-alkyl)carbamoyl, N-(C₁-C₆)-Alkyl-N-((C₃-C₈)-cycloalkyl-(C₁-C₆)-alkyl)carbamoyl, N-(+)-Dehydroabietylcarbamoyl, N-(C₁-C₆)-Alkyl-N-(+)-dehydroabietylcarbamoyl, N-(C₆-C₁₂)-Arylcarbamoyl, N-(C₇-C₁₆)-Aralkylcarbamoyl, N-(C₁-C₁₀)-Alkyl-N-(C₆-C₁₆)-arylcarbamoyl, N-(C₁-C₁₀)-Alkyl-N-(C₇-C₁₆)-aralkylcarbamoyl, N-((C₁-C₁₀)-Alkoxy-(C₁-C₁₀)alkyl)carbamoyl, N-((C₆-C₁₆)-Aryloxy-(C₁-C₁₀)alkyl)carbamoyl, N-((C₇-C₁₆)-Aralkyloxy-(C₁-C₁₀)-alkyl)carbamoyl, N-(C₁-C₁₀)-Alkyl-N-((C₁-C₁₀)-alkoxy-(C₁-C₁₀)-alkyl)carbamoyl, N-(C₁-C₁₀)-Alkyl-N-((C₆-C₁₂)-aryloxy-(C₁-C₁₀)-alkyl)carbamoyl, N-(C₁-C₁₀)-Alkyl-N-((C₇-C₁₆)-aralkyloxy-(C₁-C₁₀)-alkyl)carbamoyl, CON(CH₂)ₕ, worin eine CH₂-Gruppe durch O, S, N-(C₁-C₈)Alkylimino, N-(C₃-C₈)-Cycloalkylimino, N-(C₃-C₈)-Cycloalkyl-(C₁-C₄)-alkylimino, N-(C₆-C₁₂)-Arylimino, N-(C₇-C₁₆)-Aralkylimino oder N-(C₁-C₄)-Alkoxy-(C₁-C₆)-alkylimino ersetzt ein kann und h 3 bis 7 bedeutet,
einem Carbamoyl-Rest der allgemeinen Formel II 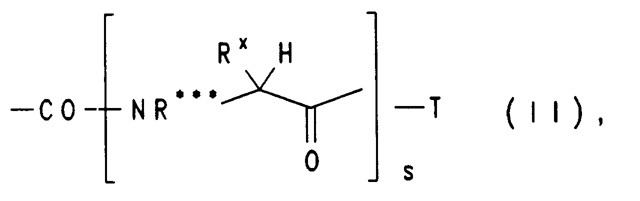 worin
- R^{x}: der Substituent einer α-Aminosäure bedeutet, zu denen die L-und D-Aminosäuren zählen,
- s: 1, 2, 3, 4 oder 5 und
- T: OH, OR oder NR*R** bedeutet, wobei
- R***: Wasserstoff oder (C₁-C₄)-Alkyl,
- R* und R**: gleich oder verschieden sind und Wasserstoff, Phenyl, Benzyl Phenethyl, (C₁-C₈)-Alkyl, (C₃-C₆)-Cycloalkyl, (+)-Dehydroabietyl, (C₁-C₈)-Alkoxy-(C₁-C₈)-alkyl, Phenyl-(C₁-C₆)-alkoxy-(C₁-C₈)-alkyl, (C₆-C₁₂)-Phenoxy-(C₁-C₈)-alkyl bedeuten, oder
- R* und R**: gemeinsam für -[CH₂]ₕ stehen, worin eine CH₂ Gruppe durch O, S, SO, SO₂, N-Acylimino, N-(C₁-C₁₀)-Alkoxycarbonylimino, N-(C₁-C₈)-Alkylimino, N-(C₃-C₈)-Cycloalkylimino, N-(C₃-C₈)-Cycloalkyl-(C₁-C₄)-alkylimino, N-Phenylimino, N-Benzylimino oder N-(C₁-C₄)-Alkoxy-(C₁-C₆)-alkylimino ersetzt sein kann und h 3 bis 7 bedeutet, oder mit
Amino, (C₁-C₁₂)-Alkylamino, Di-(C₁-C₁₂)-alkylamino, (C₃-C₈)-Cycloalkylamino, N-(C₆-C₁₂)-Arylamino, N-(C₇-C₁₁)-Aralkylamino,
(C₁-C₁₂)-Alkanoylamino, (C₃-C₈)-Cycloalkanoylamino, (C₆-C₁₂)-Aroylamino, (C₇-C₁₆)-Aralkanoylamino, (C₁-C₁₂)-Alkanoyl-N-(C₁-C₁₀)-alkylamino, (C₃-C₈)-Cycloalkanoyl-N-(C₁-C₁₀)-alkylamino, (C₆-C₁₂)-Aroyl-N-(C₁-C₁₀)-alkylamino, (C₇-C₁₁)-Aralkanoyl-N-(C₁-C₁₀)-alkylamino,
(C₁-C₁₂)-Alkanoylamino-(C₁-C₆)-alkyl, (C₃-C₈)-Cycloalkanoylamino-(C₁-C₈)-alkyl, (C₆-C₁₂)-Aroylamino-(C₁-C₆)-alkyl, (C₇-C₁₆)-Aralkanoylamino-(C₁-C₆)-alkyl, Amino-(C₁-C₁₀)-alkyl, N-(C₁-C₁₀)-alkylamino-(C₁-C₁₀)alkyl, N,N-Di(C₁-C₁₀)-alkylamino-(C₁-C₁₀)-alkyl, (C₃-C₈)-Cycloalkylamino-(C₁-C₁₀)-alkyl, (C₁-C₁₂)-Alkylmercapto, (C₁-C₁₂)-Alkylsulfinyl, (C₁-C₁₂)-Alkylsulfonyl, (C₆-C₁₂)-Arylmercapto, (C₆-C₁₂)-Arylsulfinyl, (C₆-C₁₂)-Arylsulfonyl, (C₇-C₁₂)-Aralkylmercapto, (C₇-C₁₂)-Aralkylsulfinyl, (C₇-C₁₂)-Aralkylsulfonyl,
wobei die Reste, die einen Arylrest enthalten ihrerseits am Aryl substituiert sein können durch 1 bis 5 gleiche oder verschiedene Reste aus der Reihe:
Wasserstoff, Hydroxy, Halogen, Cyano, Trifluormethyl, Nitro, Carboxyl, (C₁-C₈)-Alkyl, (C₃-C₈)-Cycloalkyl, (C₆-C₁₂)-Aryl, (C₇-C₁₆)-Aralkyl, (C₂-C₁₂)-Alkenyl, (C₂-C₁₂)-Alkinyl, (C₁-C₈)-Alkoxy, (C₁-C₆)-Alkoxy-(C₁-C₆)-alkyl, (C₆-C₁₂)-Aryloxy, (C₇-C₁₆)-Aralkyloxy, -O-[CH₂₋]_{X}C_{f}H_{(2f+1-g)}F_{g},
(C₁-C₁₂)-Alkoxycarbonyl, (C₃-C₈)-Cycloalkoxycarbonyl,
(C₁-C₁₂)-Alkylcarbonyloxy, (C₃-C₈)-Cycloalkylcarbonyloxy,
Carbamoyl, N-(C₁-C₁₂)-Alkylcarbamoyl, N, N-Di-(C₁-C₁₂)-alkylcarbamoyl, N-(C₃-C₈)-Cycloalkylcarbamoyl, N,N-Dicyclo-(C₃-C₈)-alkylcarbamoyl, N-(C₁-C₁₀)-Alkyl-N-(C₃-C₈)-cycloalkylcarbamoyl, N-((C₃-C₈)-Cycloalkyl-(C₁-C₆)-alkyl)carbamoyl, N-(C₁-C₆)-Alkyl-N-((C₃-C₈)-cycloalkyl-(C₁-C₆)-alkyl)carbamoyl, N-(+)-Dehydroabietylcarbamoyl, N-(C₁-C₆)-Alkyl-N-(+)-dehydroabietylcarbamoyl, N-(C₆-C₁₂)-Arylcarbamoyl, N-(C₇-C₁₆)-Aralkylcarbamoyl, N-(C₁-C₁₀)-Alkyl-N-(C₆-C₁₆)-arylcarbamoyl, N-(C₁-C₁₀)-Alkyl-N-(C₇-C₁₆)-aralkylcarbamoyl, N-((C₁-C₁₀)-Alkoxy-(C₁-C₁₀)-alkyl)carbamoyl, N-((C₆-C₁₆)-Aryloxy-(C₁-C₁₀)-alkyl)carbamoyl,
N-((C₇-C₁₆)-Aralkyloxy-(C₁-C₁₀)-alkyl)carbamoyl,
N-(C₁-C₁₀)-Alkyl-N-((C₁-C₁₀)-alkoxy-(C₁-C₁₀)-alkyl)carbamoyl,
N-(C₁-C₁₀)-Alkyl-N-((C₆-C₁₂)-aryloxy-(C₁-C₁₀)-alkyl)carbamoyl,
N-(C₁-C₁₀)-Alkyl-N-((C₇-C₁₆)-aralkyloxy-(C₁-C₁₀)-alkyl)carbamoyl, CON(CH₂)ₕ worin eine CH₂-Gruppe durch O, S, N-(C₁-C₈)-Alkylimino, N-(C₃-C₈)-Cycloalkylimino, N-(C₃-C₈)-Cycloalkyl-(C₁-C₄)-alkylimino, N-(C₆-C₁₂)-Arylimino, N-(C₇-C₁₆)-Aralkylimino oder N-(C₁-C₄)-Alkoxy-(C₁-C₆)-alkylimino ersetzt ein kann und h 3 bis 7 bedeutet,
Amino, (C₁-C₆)-Alkylamino, Di-(C₁-C₆)-alkylamino, (C₃-C₈)-Cycloalkylamino, N-(C₆-C₁₂)-Arylamino, N-(C₇-C₁₁)-Aralkylamino, N-(C₁-C₃)-Alkyl-(C₇-C₁₁)-aralkylamino, N-(C₁-C₃)-Alkyl-(C₆-C₁₂)-arylamino, (C₁-C₈)-Alkoxyamino,
(C₁-C₈)-Alkanoylamino, (C₃-C₈)-Cycloalkanoylamino, (C₆-C₁₂)-Aroylamino, (C₇-C₁₂)-Aralkanoylamino, (C₁-C₈)-Alkanoyl-N-(C₁-C₆)-alkylamino, (C₃-C₈)-Cycloalkanoyl-N-(C₁-C₆)-alkylamino, (C₆-C₁₂)-Aroyl-N-(C₁-C₁₀)-alkylamino, (C₇-C₁₁)-Aralkanoyl-N-(C₁-C₆)-alkylamino,
(C₁-C₈)-Alkanoylamino-(C₁-C₄)-alkyl, (C₃-C₈)-Cycloalkanoylamino-(C₁-C₄)-alkyl, (C₆-C₁₂)-Aroylamino-(C₁-C₄)-alkyl und (C₇-C₁₂)-Aralkanoylamino-(C₁-C₄)-alkyl,
- R⁴: einen verzweigten oder unverzweigten (C₇-C₂₀)-Alkylrest, der eine oder zwei C-C-Mehrfachbindungen enthalten kann, oder
(C₁-C₈)-Alkoxy-(C₁-C₆)-alkyl, (C₁-C₆)-Alkoxy-(C₁-C₄)-alkoxy-(C₁-C₄)-alkyl oder einen Rest der Formel Z bedeutet,

-[CH₂]ᵥ-[O]_{w}-[CH₂]ₜ-E (Z),

wobei E einen substituierten Phenylrest der Formel F 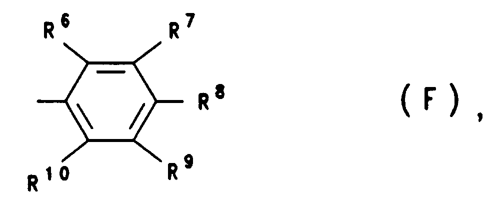 oder einen (C₃-C₈)-Cycloalkylrest bedeutet, wobei
v = 0, 1, 2, 3, w = 0, 1 und t = 0,1 sein kann, mit der Einschränkung, daß v ungleich 0 ist, falls w = 1 ist, und
worin R⁶, R⁷, R⁸, R⁹ und R¹⁰ gleich oder verschieden sind und Wasserstoff, Fluor, Chlor, Cyano, Trifluormethyl, (C₁-C₆)-Alkyl, (C₁-C₆)-Alkoxy, -O-[CH₂₋]ₓC_{f}H_{(2f+1-g)}F_{g}, N-(C₁-C₈)-Alkylcarbamoyl, N,N-Di-(C₁-C₈)-alkylcarbamoyl, N-(C₃-C₈)-Cycloalkylcarbamoyl, gegebenenfalls mit Fluor, Chlor, Trifluormethyl und (C₁-C₆)-Alkoxy substituiertes (C₇-C₁₁)-Phenylalkylcarbamoyl bedeuten.
Ganz besonders bevorzugt sind Verbindungen der Formel 1, in der
Q = O,
X = O,
Y = CR³,
m = O,
- A: -CHR^{x}, wobei R^{x} den Substituenten des α-C-Atoms einer α-Aminosäure bedeutet, insbesondere Wasserstoff
- B =: CO₂V bedeutet, wobei
- V: den Rest eines Alkohols VOH bedeutet und für einen verzweigten oder unverzweigten oder cyclischen aliphatischen (C₁-C₉)-Alkyl-Rest, einen (C₅-C₆)-Cycloalkyl-(C₁-C₄)-alkylrest,
einen verzweigten oder unverzweigten (C₂-C₈)-Alkenylrest oder einen Phenyl-, Benzyl-, Phenethyl-, Phenylpropyl-oder Phenylbutylrest steht, wobei die vorstehenden Reste einen Substituenten aus der Reihe Hydroxy, (C₁-C₄)-Alkoxy, (C₁-C₆)-Alkylcarbonyloxy, (C₅-C₆)-Cycloalkylcarbonyloxy, Benzoyloxy, Phenylalkylcarbonyloxy enthalten,
- R¹ und R³: Wasserstoff,
- R²: einen Rest -CONR⁵R⁶ bedeutet, wobei
- R⁵: Wasserstoff oder ein 1, 2 oder 3-wertiges physiologisch verwendbares Kation bedeutet, insbesondere Na⊕, K⊕, Mg²⊕, Ca²⊕ oder H₃N^{⊕}C(CH₂OH)₃ (Tris-Salz),
- R⁶: einen Rest der Formel I, ausgenommen -SO₂H, bedeutet

-G-K-[C-U]ᵣ-D-W (I)

in welchem
- G: -SO₂- bedeutet,
- K: eine Bindung,
- C: eine Bindung,
- U: eine Bindung,
- r: 1 ist,
- D: eine Bindung oder
(C₁-C₄)-Alkandiyl,
- W: Wasserstoff oder
einen Phenylrest bedeutet,
wobei dieser durch 1, 2, 3, 4 oder 5 Substituenten aus der Reihe Fluor, Chlor, Nitril, Trifluormethyl, (C₁-C₆)-Alkyl, Phenyl, (C₁-C₆)-Alkoxy, Phenoxy, -O-[CH₂₋]_{X}C_{f}H_{(2f+1-g)}F_{g},
Carbamoyl, N-(C₁-C₈)-Alkylcarbamoyl, N, N-Di-(C₁-C₈)-alkylcarbamoyl, N-(C₃-C₆)-Cycloalkylcarbamoyl, N-(C₁-C₄)-Alkyl-N-(C₃-C₆)-cycloalkylcarbamoyl, N-((C₃-C₆)-Cycloalkyl-(C₁-C₄)-alkyl)carbamoyl, N-(C₁-C₆)-Alkyl-N-((C₃-C₆)-cycloalkyl-(C₁-C₄)-alkyl)carbamoyl, N-(+)-Dehydroabietylcarbamoyl, N-(C₆-C₁₂)-Phenylcarbamoyl, N-Phenyl-(C₁-C₄)-alkylcarbamoyl, N-(C₁-C₆)-Alkyl-N-phenylcarbamoyl, N-(C₁-C₆)-Alkyl-N-phenyl-(C₁-C₄)-alkylcarbamoyl, N-((C₁-C₆)-Alkoxy-(C₁-C₆)-alkyl)carbamoyl, N-Phenoxy-(C₁-C₆)-alkyl)carbamoyl, CON(CH₂)ₕ, worin eine CH₂-Gruppe durch O, N-(C₁-C₆)-Alkylimino, ersetzt ein kann und h 3 bis 5 bedeutet, oder
einen Carbamoyl-Rest der allgemeinen Formel II 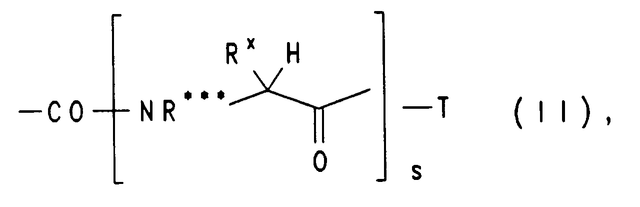 worin
- R^{x}: der Substituent einer α-Aminosäure bedeutet, zu denen die L-und D-Aminosäuren zählen,
- s: 1, 2, 3, 4 oder 5 und
- T: OH, OR oder NR*R** bedeutet, wobei
- R***: Wasserstoff oder (C₁-C₄)-Alkyl,
- R* und R**: gleich oder verschieden sind und Wasserstoff, Phenyl, Benzyl Phenethyl, (C₁-C₈)-Alkyl, (C₃-C₆)-Cycloalkyl, (+)-Dehydroabietyl, (C₁-C₈)-Alkoxy-(C₁-C₈)-alkyl, (C₇-C₁₂)-Phenylalkoxy-(C₁-C₈)-alkyl, (C₆-C₁₂)-Phenoxy-(C₁-C₈)-alkyl bedeuten, oder
- R* und R**: gemeinsam für -[CH₂]ₕ stehen, worin eine CH₂ Gruppe durch O, S, SO, SO₂, N-Acylimino, N-(C₁-C₁₀)-Alkoxycarbonylimino, N-(C₁-C₈)-Alkylimino, N-(C₃-C₆)-Cycloalkylimino, N-(C₃-C₆)-Cycloalkyl-(C₁-C₄)-alkylimino, N-Phenylimino, N-Benzylimino oder N-(C₁-C₄)-Alkoxy-(C₁-C₆)-alkylimino ersetzt sein kann und h 3 bis 5 bedeutet,
(C₁-C₈)-Alkanoylamino, (C₃-C₈)-Cycloalkanoylamino, (C₆-C₁₂)-Phenyl-amino, (C₇-C₁₁)-Phenylalkanoylamino, (C₁-C₈)-Alkanoyl-N-(C₁-C₁₀)-alkylamino, (C₃-C₆)-Cycloalkanoyl-N-(C₁-C₆)-alkylamino, Benzoyl-N-(C₁-C₁₀)-alkylamino, (C₇-C₁₁)-Phenylalkanoyl-N-(C₁-C₆)-alkylamino,
(C₁-C₁₀)-Alkanoylamino-(C₁-C₂)-alkyl, (C₃-C₈)-Cycloalkanoylamino-(C₁-C₂)-alkyl, Benzoylamino-(C₁-C₂)-alkyl, (C₇-C₁₄)-Phenylalkanoylamino-(C₁-C₂)-alkyl substituiert ist und wobei die Reste, die einen Arylrest enthalten, ihrerseits mit einem Substituenten aus der Reihe Wasserstoff, Hydroxy, Fluor, Chlor, Trifluormethyl, (C₁-C₆)-Alkyl, (C₁-C₈)-Alkoxy substituiert sein können und
- R⁴: einen verzweigten oder unverzweigten (C₇-C₁₂)-Alkylrest, der eine oder zwei C-C-Mehrfachbindungen enthalten kann, oder
(C₁-C₄)-Alkoxy-(C₁-C₆)-alkyl, (C₁-C₄)-Alkoxy-(C₁-C₄)-alkoxy-(C₁-C₄)alkyl oder einen Rest der Formel Z bedeutet,

-[CH₂]ᵥ-[O]_{w}-[CH₂]ₜ-E (Z),

wobei E einen substituierten Phenylrest der Formel F 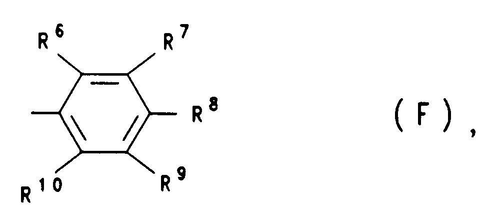 oder einen (C₃-C₈)-Cycloalkylrest bedeutet, wobei
v = 0, 1, 2 oder 3, w = 0 und t = 0 ist, und
worin R⁶, R⁷, R⁸, R⁹ und R¹⁰ gleich oder verschieden sind und Wasserstoff, Fluor, Chlor, Cyano, Trifluormethyl, (C₁-C₆)-Alkyl, (C₁-C₆)-Alkoxy, -O-[CH₂₋]ₓC_{f}H_{(2f+1-g)}F_{g}, N-(C₁-C₈)-Alkylcarbamoyl, N,N-Di-(C₁-C₈)-alkylcarbamoyl, N-(C₃-C₈)-Cycloalkylcarbamoyl, gegebenenfalls mit Fluor, Chlor, Trifluormethyl und (C₁-C₆)-Alkoxy substituiertes (C₇-C₁₁)-Phenylalkylcarbamoyl bedeuten und
- n: 0,
- f: 1 bis 5,
- g: 0, 1 bis (2f+1) und
- x: 0 oder 1 bedeuten.

Die Erfindung betrifft die Verwendung von Verbindungen der allgemeinen Formel 1 sowie die physiologisch verträglichen Salze zur Inhibierung der Kollagenbiosynthese.

Weiterhin betrifft die Erfindung die Verwendung von Verbindungen der allgemeinen Formel 1 sowie die physiologisch verträglichen Salze zur Herstellung eines Arzneimittels gegen fibrotische Erkrankungen, insbesondere in der Leber, in der Lunge und der Haut (Skleroderma).

Insbesondere betrifft die Erfindung die Verbindungen der Formel 1 zur Anwendung als Inhibitoren der Prolyl-4-hydroxylase.

Schließlich betrifft die Erfindung die Verbindungen der allgemeinen Formel 1 zur Verwendung als Arzneimittel.

Die Erfindung umfaßt weiterhin Prodrugs zu den Verbindungen der Formel 1 die eine Hemmung der Kollagenbiosynthese in vivo durch Freisetzung von Verbindungen der Formel 1 oder deren Salzen bewirken.

Schließlich umfaßt die Erfindung auch Prodrugs, die in vivo durch Freisetzung von Verbindungen der Formel 1 odet deren Salzen eine inhibitorische Wirkung auf die Prolyl-4-hydroxylase bewirken.

Prodrug-Gruppierungen sind chemische Gruppen, die in vivo
- zur Carboxylatgruppe der Verbindungen der Formel 1 umgewandelt werden und/oder
- vom Amid-N-Atom abgespalten werden können und/oder
- zu einem Pyridinring umgewandelt werden können.

Die in Betracht kommenden Prodrug-Gruppen sind dem Fachmann bekannt.

Insbesondere sind folgende Prodrug-Gruppierungen genannt:
für die Carboxylatgruppe Ester-, Amid-, Hydroxymethylgruppen und deren Derivate und Aldehydgruppen und deren Abkömmlinge für das Pyridin-N-Atom N-Oxide und N-Alkylderivate und für den Pyridinring 1,4-Dihydropyridin-Derivate.

Weiterhin betrifft die Erfindung ein Verfahren zur Herstellung von Verbindungen der allgemeinen Formel 1.

Die Herstellung der Verbindungen der Formel 1, in denen A einen substituierten Alkylen-Teil, B = CO₂H, Y = CR³ und m = 0 und 1 bedeuten, erfolgt, indem die in den Schemata 1, 2 und 3 beschriebenen Reaktionen durchgeführt werden.

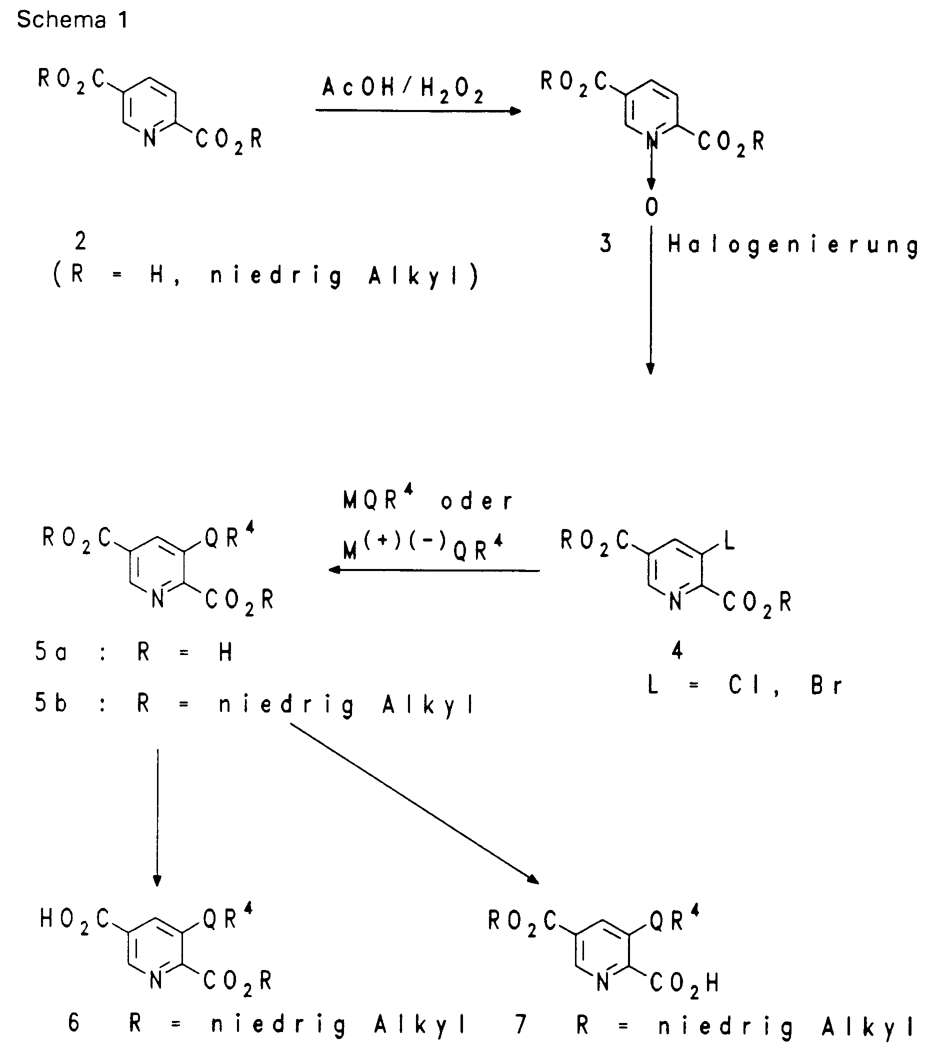

### zu Schema 1

Die 3-substituierten 5-Carboxypyridin-2-carbonsäureester der Formel 6 werden aus den Pyridin-2,5-dicarbonsäurediestern der Formel 2 hergestellt.

Die Oxidation der Pyridin-2,5-dicarboxylate der Formel 2 ist in J. Chem. Soc. Perkin Trans. 2, 1978, 34-38 und in J. Org. Chem. 25 (1960) 565-568 (M.L. Peterson) beschrieben.

Die Halogenierung (Chlorierung) der Pyridin-N-oxide der Formel 3 erfolgt mit Thionylchlorid, bevorzugt in einem unpolaren Lösungsmittel und ggf. mit einem Katalysator, und die Reaktion des 3-Chlorpyridin-2,5-dicarbonsäurediesters (Formel 4) mit Alkoholaten MQR⁴ (M = Metall, Q = O) in dem Alkohol R⁴OH oder in einem dipolar aprotischen Lösungsmittel.

Aus substituierten Pyridin-2,5-dicarbonsäuren (siehe CA: Vol. 68, 1968, 68840 h) können unter Veresterungsbedingungen die Pyridin-2-carbonsäureester-5-carboxylate der Formel 6 hergestellt werden. Geeignete Bedingungen sind z.B. die Veresterung mit Methanol in Gegenwart von Schwefelsäure, wobei die Reaktionszeit so zu wählen ist, daß die vollständige Veresterung zum Diesterprodukt nur untergeordnet stattfindet, bzw. die Diesterprodukte als Nebenprodukte abgetrennt werden können. 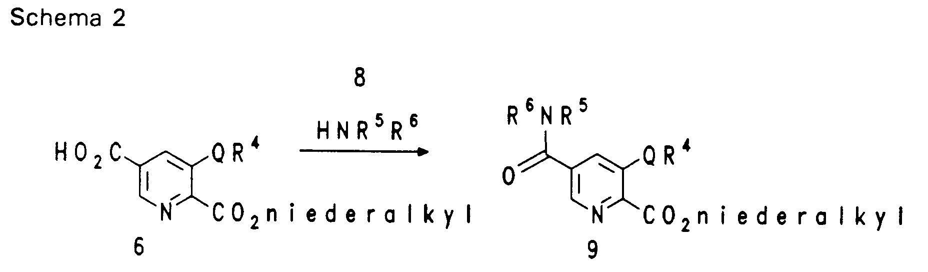
Die Herstellung der Verbindungen der Formel 9 erfolgt aus den Verbindungen der Formel 6 und den Amidderivaten der Formel 8, wobei es zweckmäßig sein kann, beide Reaktanden mit Hilfsreagenzien zu aktivieren (Houben-Weyl: Methoden der Organischen Chemie, Band IX, Kapitel 19, Seiten 636 bis 637); vgl. Schema 2.

An Reagenzien zur Carbonsäurereaktivierung können die dem Fachmann bekannten Substanzen, wie Thionylchlorid, Oxalylchlorid, Pivaloylchlorid oder Chlorameisensäureester-Derivate Verwendung finden. Es ist nicht immer notwendig, diese aktivierten Derivate der Verbindungen der Formel 7 zu isolieren. Meist ist es zweckmäßig, sie nach Herstellung in situ oder als Rohprodukte mit den Sulfonamidderivaten der Formel 8 umzusetzen.

Zweckmäßigerweise werden die Verbindungen der Formel 8 zunächst mit einer anorganischen oder organischen Base, wie z. B. Natrium- oder Kaliumhydroxid, -carbonat, -alkoxid, -hydrid, -amid, Ammoniak, Triethylamin, Tributylamin, Pyridin bei -20° bis + 150°C, vorzugsweise bei 0° bis -80°C zur Reaktion gebracht und dieses Reaktionsgemisch mit einer Verbindung der Formel 6 oder dessen aktivierter Form umgesetzt. Die Umsetzung erfolgt in einem inerten Lösungsmittel, wie z. B. Methylenchlorid, Methanol, Ethanol, Aceton, Essigsäureethylester, Toluol, Tetrahydrofuran, Acetonitril, N,N-Dimethylformamid, N,N-Dimethylacetamid, Nitromethan, Dimethylsulfoxid oder Gemischen dieser Lösungsmittel. 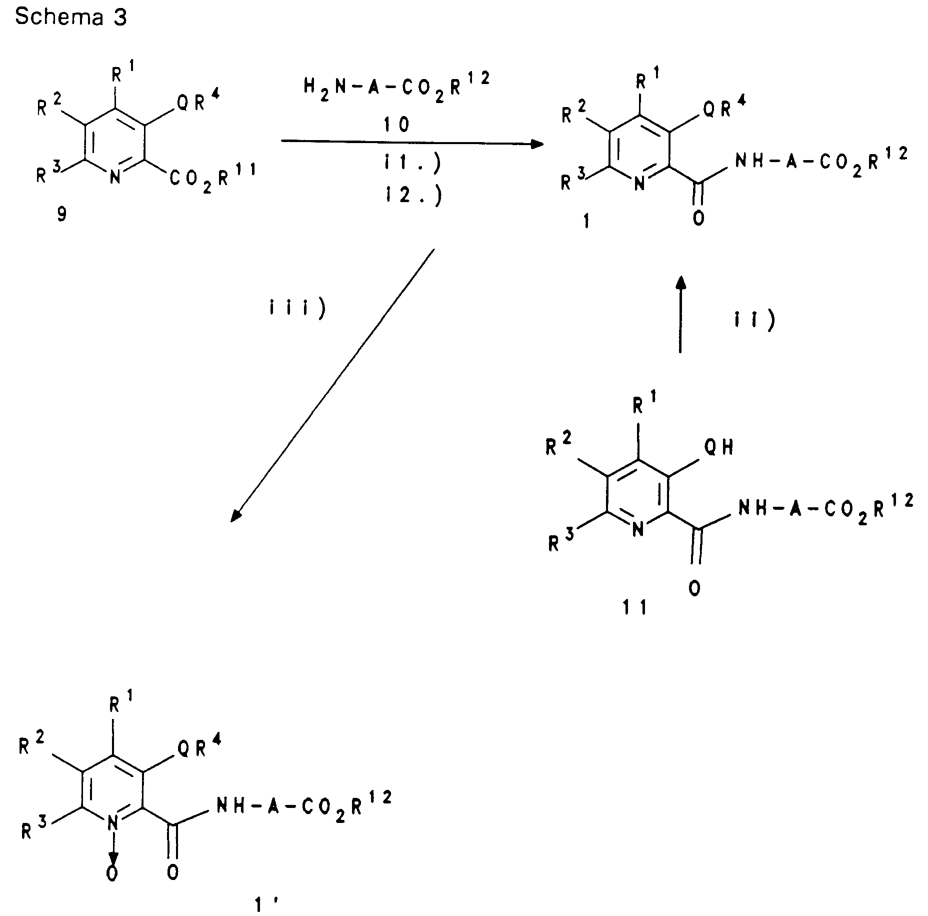
R¹¹ = H, (C₁-C₃)-Alkyl, PG
R¹² = H, (C₁-C₈)-Alkyl, Benzyl
X = Abgangsgruppe, insbesondere Halogen, OSO₂Me, OSO₂Phenyl

### zu Schema 3

i1.) Pyridin-2-carbonsäuren der Formel 9 (R¹¹ = H) mit den Aminoestern der Formel 10 zu den Amidestern der Formel 1 umgesetzt werden, oder
i2.) Pyridin-2-carbonsäureester der Formel 9 (R¹¹ = niedrig Alkyl) unter den Bedingungen der Aminolyse mit den Verbindungen der Formel 10 zu den Verbindungen der Formel 1 umgesetzt werden; oder
ii) die Verbindungen der Formel 1 durch Alkylierung von Verbindungen der Formel 11 mit R⁴X hergestellt sind und ggf.
iii) die Verbindungen der Formel 1 sofern Q = O und NR^{y} gilt, in ihre Pyridin-N-oxide übergeführt werden.

Geeignete Verfahren zur Amidbildung (Umsetzung i1) sind die Methoden der Carbonylaktivierung und die aus der Peptidchemie bekannten Kondensationsreaktionen.

An Reagenzien zur Carbonsäureaktivierung können die dem Fachmann bekannten Substanzen, wie Thionylchlorid, Oxalylchlorid, Pivaloylchlorid, Chlorameisensäureester-Derivate oder N,N'-Carbonyldimidazol Verwendung finden. Die aktivierten Derivate der Verbindungen der Formel 9 werden nach Herstellung in situ mit den Amidderivaten der Formel 10 umgesetzt.

Ein geeignetes Kondensationsmittel ist beispielsweise die Kombination von N,N'-Dicyclohexylcarbodiimid/N-Hydroxy-1H-benzotriazol und N-Ethylmorpholin.

Geeignete Lösungsmittel sind Dichlormethan, Tetrachlormethan, Butylacetat, Ethylacetat, Toluol, Tetrahydrofuran, Dimethoxyethan, 1,4-Dioxan, Acetonitril, N,N-Dimethylformamid, N,N-Dimethylacetamid, Dimethylsulfoxid, Nitromethan und/oder Pyridin.

Die Verbindungen der Formel I sind Inhibitoren der Prolyl-4-hydroxylase. Die Hemmung dieses Enzyms wurde, wie von Kaule und Günzler in Anal. Biochem. 184, 291-297 (1990) beschrieben, bestimmt.

Die erfindungsgemäßen Verbindungen der Formel 1 besitzen wertvolle pharmakologische Eigenschaften und zeigen insbesondere antifibrotische Wirksamkeit, insbesondere inder Leber, in der Lunge und auf der Haut (Skleroderma).

Die antifibrotische Wirkung kann im Modell der Tetrachlorkohlenstoff-induzierten Leberfibrose bestimmt werden. Dazu werden Ratten mit CCl₄ (1 ml/kg) - gelöst in Olivenöl - zweimal wöchentlich behandelt. Die Prüfsubstanz wird täglich, gegebenenfalls sogar zweimal täglich per os oder intraperitoneal - gelöst in einem geeigneten verträglichen Lösungsmittel - verabreicht. Das Ausmaß der Leberfibrose wird histologisch bestimmt und der Anteil Kollagen in der Leber per Hydroxyprolinbestimmung - wie bei Kivirikko et al. (Anal. Biochem. 19, 249 f. (1967)) beschrieben - analysiert. Die Aktivität der Fibrogenese kann durch radioimmunologische Bestimmung von Kollagenfragmenten und Prokollagenpeptiden im Serum bestimmt werden. Die erfindungsgemäßen Verbindungen sind in diesem Modell in Konzentration 1 bis 100 mg/kg wirksam.

Die Aktivität der Fibrogenese kann durch radioimmunologische Bestimmung des N-terminalen Propeptids des Kollagen Typ-III oder der N- bzw. C-terminalen Quervernetzungsdomäne des Kollagen-Typ-IV (7s-Kollagen bzw. Typ-IV-Kollagen NC₁) im Serum bestimmt werden.

Zu diesem Zweck wurden die Hydroxyprolin-, Prokollagen-III-Peptid-, 7s-Kollagen- und Typ-IV-Kollagen-NC-Konzentrationen in der Leber von
a) unbehandelten Ratten (Kontrolle)
b) Ratten, denen Tetrachlorkohlenstoff verabreicht wurden (CCl₄-Kontrolle)
c) Ratten, denen zunächst CCl₄ und anschließend eine erfindungsgemäße Verbindung verabreicht wurde
gemessen (diese Testmethode wird beschrieben von Rouiller, C., experimental toxic injury of the liver; in The Liver, C. Rouiller, Vol. 2, 5. 335 bis 476, New York, Academic Press, 1964).

Die Verbindungen der Formel 1 können als Medikamente in Form von pharmazeutischer Präparate Verwendung finden, welche sie gegebenenfalls mit verträglichen pharmazeutischen Trägern enthalten. Die Verbindungen können als Heilmittel, z. B. in Form pharmazeutischer Präparate Verwendung finden, welche diese Verbindungen in Mischung mit einem für die enterale, perkutane oder parenterale Applikation geeigneten pharmazeutischen, organischen oder anorganischen Träger, wie z. B. Wasser, Gummi arabicum, Gelatine, Milchzucker, Stärke, Magnesiumstearat, Talk, pflanzliche Öle, Polyalkylenglykole, Vaseline usw. enthalten.

Sie können zu diesem Zweck oral in Dosen von 0,1 bis 25 mg/kg/Tag, vorzugsweise 1 bis 5 mg/kg/Tag oder parenteral in Dosen von 0,01 bis 5 mg/kg/Tag, vorzugsweise 0,01 bis 2,5 mg/kg/Tag, insbesondere 0,5 bis 1,0 mg/kg/Tag, appliziert werden. Die Dosierung kann in schweren Fällen auch erhöht werden. In vielen Fällen genügen jedoch auch geringere Dosen. Diese Angaben beziehen sich auf einen Erwachsenen von etwa 75 kg Gewicht.

### Beispiel 1

3-Benzyloxy-5-[((4-((((+)-dehydroabietylamino)-L-valinyl)carbonyl)-phenylsulfonyl)amino)carbonyl]pyridin-2-carbonsäure(glycylethylester)amid

### Beispiel 2

3-Benzyloxy-5-[((4-(((2-(4-fluorphenyl)ethyl)amino)carbonyl)phenylsulfonyl)-amino)carbonyl]pyridin-2-carbonsäure(glycylethylester)amid

### Beispiel 3

3-Benzyloxy-5-[((4-((cylcohexylamino)carbonyl)phenylsulfonyl)amino)carbonyl]-pyridin-2-carbonsäure(glycylethylester)amid

### Beispiel 4

3-Benzyloxy-5-[((4-(((Diethylamino)-L-valinyl)carbonyl)phenylsulfonyl)-amino)carbonyl]pyridin-2-carbonsäure(glycyethyester)amid

### Beispiel 5

3-Benzyloxy-5-[((4-(N,N-diethylaminocarbonyl)phenylsulfonyl)amino)carbonyl]-pyridin-2-carbonsäure(glycylethylester)amid

### Beispiel 6

3-Benzyloxy-5-[((4-(2-((2-chlor-5-methoxybenzoyl)amino)ethyl)phenylsulfonyl)-amino)carbonyl]pyridin-2-carbonsäure(glycylethylester)amid

### Beispiel 7

3-Benzyloxy-5-[((4-(2-propyloxy)phenyl)sulfonyl)amino)carbonyl]pyridin-2-carbonsäure(glycylethylester)amid

### Beispiel 8

3-(4-Fluorbenzyloxy)-5-[((4-(2-propyloxy)phenyl)sulfonyl)amino)-carbonyl]pyridin-2-carbonsäure(glycylethylester)amid

### Beispiel 9

3-Benzyloxy-5-[((n-butylsulfonyl)amino)carbonyl]pyridin-2-carbonsäure-(glycylethylester)amid

### Beispiel 10

5-[((n-Butylsulfonyl)amino)carbonyl]-3-(4-fluorbenzyloxy)pyridin-2-carbonsäure-(glycylethylester)amid

### Beispiel 11

5-[((n-Butylsulfonyl)amino)carbonyl]-3-(4-chlorbenzyloxy)pyridin-2-carbonsäure-(glycylethylester)amid

### Beispiel 12

5-[((n-Butylsulfonyl)amino)carbonyl]-3-(3-methoxybenzyloxy)pyridin-2-carbonsäure(glycylethylester)amid

### Beispiel 13

3-Benzyloxy-5-[((4-((((+)-dehydroabietylamino)-L-valinyl)carbonyl)-phenylsulfonyl)amino)carbonyl]pyridin-2-carbonsäure(glycyl-(2-propyl)ester)amid

### Beispiel 14

3-Benzyloxy-5-[((4-(((2-(4-fluorphenyl)ethyl)amino)carbonyl)phenylsulfonyl)-amino)carbonyl]pyridin-2-carbonsäure(glycyl-(2-propyl)ester)amid

### Beispiel 15

3-Benzyloxy-5-[((4-((cylcohexylamino)carbonyl)phenylsulfonyl)amino)carbonyl]-pyridin-2-carbonsäure(glycyl-(2-propyl)ester)amid

### Beispiel 16

3-Benzyloxy-5-[((4-(((diethylamino)-L-valinyl)carbonyl)phenylsulfonyl)-amino)carbonyl]pyridin-2-carbonsäure(glycyl-(2-propyl)ester)amid

### Beispiel 17

3-Benzyloxy-5-[((4-(N,N-diethylaminocarbonyl)phenylsulfonyl)amino)carbonyl]-pyridin-2-carbonsäure(glycyl-(2-propyl)ester)amid

### Beispiel 18

3-Benzyloxy-5-[((4-(2-((2-chlor-5-methoxybenzoyl)amino)ethyl)phenylsulfonyl)-amino)carbonyl]pyridin-2-carbonsäure(glycyl-(2-propyl)ester)amid

### Beispiel 19

3-Benzyloxy-5-[((4-((((+)-dehydroabietylamino)-L-valinyl)carbonyl)-phenylsulfonyl)amino)carbonyl]pyridin-2-carbonsäure(glycyl-(3-pentyl)ester)amid

### Beispiel 20

3-Benzyloxy-5-[((4-(((2-(4-fluorphenyl)ethyl)amino)carbonyl)phenylsulfonyl)-amino)carbonyl]pyridin-2-carbonsäure(glycyl-(3-pentyl)ester)amid

### Beispiel 21

3-Benzyloxy-5-[((4-((cylcohexylamino)carbonyl)phenylsulfonyl)amino)carbonyl]-pyridin-2-carbonsäure(glycyl-(3-pentyl)ester)amid

### Beispiel 22

3-Benzyloxy-5-[((4-(((diethylamino)-L-valinyl)carbonyl)phenylsulfonyl)-amino)carbonyl]pyridin-2-carbonsäure(glycyl-(3-pentyl)ester)amid

### Beispiel 23

3-Benzyloxy-5-[((4-(N,N-diethylaminocarbonyl)phenylsulfonyl)amino)carbonyl]-pyridin-2-carbonsäure(glycyl-(3-pentyl)ester)amid

### Beispiel 24

3-Benzyloxy-5-[((4-(2-((2-chlor-5-methoxybenzoyl)amino)ethyl)phenylsulfonyl)-amino)carbonyl]pyridin-2-carbonsäure(glycyl-(3-pentyl)ester)amid

### Beispiel 25

3-Benzyloxy-5-[((n-butylsulfonyl)amino)carbonyl]pyrimidin-2-carbonsäure-(glycyl-(3-pentyl)ester)amid

### Beispiel 26

5-[((n-Butylsulfonyl)amino)carbonyl]-3-(4-fluorbenzyloxy)pyridin-2-carbonsäure-(glycyl-(3-pentyl)ester)amid

### Beispiel 27

5-[((n-Butylsulfonyl)amino)carbonyl]-3-(4-chlorbenzyloxy)pyridin-2-carbonsäure-(glycyl-(3-pentyl)ester)amid

### Beispiel 28

5-[((n-Butylsulfonyl)amino)carbonyl]-3-(3-methoxybenzyloxy)pyridin-2-carbonsäure(glycyl(3-pentyl)ester)amid

### Beispiel 29

3-Benzyloxy-5-[((4-((((+)-dehydroabietylamino)-L-valinyl)carbonyl)-phenylsulfonyl)amino)carbonyl]pyridin-2-carbonsäure(glycyl-(1-butyl)ester)amid

### Beispiel 30

3-Benzyloxy-5-[((4-(((2-(4-fluorphenyl)ethyl)amino)carbonyl)phenylsulfonyl)-amino)carbonyl]pyridin-2-carbonsäure(glycyl-(1-butyl)ester)amid

### Beispiel 31

3-Benzyloxy-5-[((4-((cylcohexylamino)carbonyl)phenylsulfonyl)amino)carbonyl]-pyridin-2-carbonsäure(glycyl-(1-butyl)ester)amid

### Beispiel 32

3-Benzyloxy-5-[((4-(((2-(4-fluorphenyl)ethyl)amino)carbonyl)phenylsulfonyl)-amino)carbonyl]pyridin-2-carbonsäure(glycyl-(4-heptyl)ester)amid

### Beispiel 33

3-Benzyloxy-5-[((4-((cylcohexylamino)carbonyl)phenylsulfonyl)amino)carbonyl]-pyridin-2-carbonsäure(glycylretinylester)amid

## Patentansprüche

1. Verbindungen der allgemeinen Formel 1 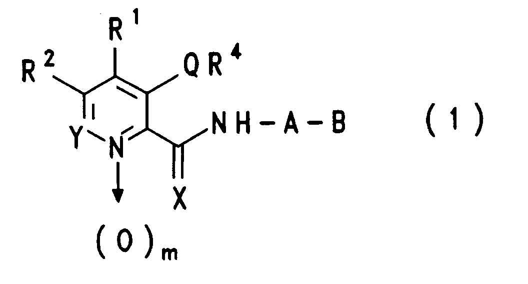 in welcher
Q O, S oder NR^{Y},
X O oder S,
Y C-R³ oder N,
m 0 und 1,
A (C₁-C₄)-Alkylen, das gegebenenfalls substituiert ist mit einem oder zwei Substituenten aus der Reihe Halogen, Cyano, Nitro, Trifluormethyl, (C₁-C₆)-Alkyl, (C₁-C₆)-Hydroxyalkyl, (C₁-C₆)-Alkoxy, -O-[CH₂]ₓ-C_{f}H_{(2f+1-g)}Hal_{g}, vorzugsweise (C₁-C₈)-Fluoralkoxy, (C₁-C₈)-Fluoralkenyloxy, (C₁-C₈)-Fluoralkinyloxy, -OCF₂Cl oder -O-CF₂-CHFCl, (C₁-C₆)-Alkylmercapto, (C₁-C₆)-Alkylsulfinyl, (C₁-C₆)-Alkylsulfonyl, (C₁-C₆)-Alkylcarbonyl, (C₁-C₆)-Alkoxycarbonyl, Carbamoyl, N-(C₁-C₄)-Alkylcarbamoyl, N,N-Di-(C₁-C₄)-alkylcarbamoyl, (C₁-C₆)-Alkylcarbonyloxy, (C₃-C₈)-Cycloalkyl, Phenyl, Benzyl, Phenoxy, Benzyloxy, Anilino, N-Methylanilino, Phenylmercapto, Phenylsulfonyl, Phenylsulfinyl, Sulfamoyl, N-(C₁-C₄)-Alkylsulfamoyl, N,N-Di-(C₁-C₄)-alkylsulfamoyl oder
mit einem substituierten (C₆-C₁₂)-Aryloxy-, (C₇-C₁₁)-Aralkyloxy, (C₆-C₁₂)-Aryl- oder (C₇-C₁₁)-Aralkyl-Rest, der im Arylteil 1, 2, 3, 4 oder 5 gleiche oder verschiedene Substituenten aus der Reihe Halogen, Cyano, Nitro, Trifluormethyl, (C₁-C₆)-Alkyl, (C₁-C₆)-Alkoxy,
-O-[CH₂]ₓ-C_{f}H_{(2f+1-g)}Hal_{g}, -OCF₂Cl,-O-CF₂-CHFCl, (C₁-C₆)-Alkylmercapto, (C₁-C₆)-Alkylsulfinyl, (C₁-C₆)-Alkylsulfonyl, (C₁-C₆)-Alkylcarbonyl, (C₁-C₆)-Alkoxycarbonyl, Carbamoyl, N-(C₁-C₄)-Alkylcarbamoyl, N,N-Di-(C₁-C₄)-alkylcarbamoyl, (C₁-C₆)-Alkylcarbonyloxy, (C₃-C₈)-Cycloalkyl, Sulfamoyl, N-(C₁-C₄)-Alkylsulfamoyl oder N,N-Di-(C₁-C₄)-alkylsulfamoyl trägt, oder
mit einem Substituenten R^{x} des α-C-Atoms einer α-Aminosäure, zu denen die natürlichen L-Aminosäuren und ihre D-Isomeren zählen;
B -CO₂V bedeutet, wobei
V den Rest eines Alkohols VOH bedeutet, worin V
(C₁-C₁₀)-Acyloxy-(C₁-C₆)-alkyl, vorzugsweise (C₁-C₁₀)-Alkanoyloxy-(C₁-C₆)-alkyl, Benzyloxy-(C₁-C₆)-alkyl, Benzyloxycarbonyloxy-(C₁-C₆)-alkyl oder Alkoxycarbonyloxy-(C₁-C₆)-alkyl), oder
einen verzweigten, unverzweigten oder cyclischen aliphatischen (C₁-C₂₀)-Alkyl-Rest, oder
einen verzweigten, unverzweigten oder cyclischen (C₂-C₂₀)-Alkenyl-Rest, einen Retinyl-Rest, einen (C₂-C₁₆)-Alkinyl-Rest oder einen entsprechenden (C₄-C₁₆)-Alkeninyl-Rest bedeutet, wobei die Reste jeweils eine oder mehrere Mehrfachbindungen enthalten können, oder
einen (C₆-C₁₆)-Aryl-Rest, einen (C₇-C₁₆)-Aralkyl-Rest oder einen 5- oder 6-gliedrigen, Heteroaryl- oder einen 5- oder 6-gliedrigen, Heteroaralkyl-Rest bedeutet, wobei die vorstehenden Reste insbesondere einen oder mehrere Substituenten aus der Reihe
Hydroxy, Halogen, Cyano, Trifluormethyl, Nitro, Carboxy, (C₁-C₁₂)-Alkyl, (C₃-C₈)-Cycloalkyl, (C₃-C₈)-Cycloalkyl-(C₁-C₁₂)-alkyl, (C₃-C₈)-Cycloalkoxy, (C₃-C₈)-Cycloalkyl-(C₁-C₁₂)-alkoxy, (C₃-C₈)-Cycloalkyloxy-(C₁-C₁₂)-alkyl, (C₃-C₈)-Cycloalkyloxy-(C₁-C₁₂)-alkoxy, (C₃-C₈)-Cycloalkyl-(C₁-C₈)-alkyl-(C₁-C₆)-alkoxy, (C₃-C₈)-Cycloalkyl-(C₁-C₈)-alkoxy-(C₁-C₆)-alkyl, (C₃-C₈)-Cycloalkyloxy-(C₁-C₈)-alkoxy-(C₁-C₆)-alkyl, (C₃-C₈)-Cycloalkoxy-(C₁-C₈)-alkoxy-(C₁-C₈)-alkoxy, (C₆-C₁₂)-Aryl, (C₇-C₁₆)-Aralkyl, (C₂-C₁₂)-Alkenyl, (C₂-C₁₂)-Alkinyl, (C₁-C₁₂)-Alkoxy, (C₁-C₁₂)-Alkoxy-(C₁-C₁₂)alkyl, (C₁-C₁₂)-Alkoxy-(C₁-C₁₂)alkoxy, (C₁-C₁₂)-Alkoxy-(C₁-C₈)-alkoxy-(C₁-C₈)-alkyl, (C₆-C₁₂)-Aryloxy, (C₇-C₁₆)-Aralkyloxy, (C₆-C₁₂)-Aryloxy-(C₁-C₆)-alkoxy, (C₇-C₁₆)-Aralkoxy-(C₁-C₆)-alkoxy, (C₁-C₈)-Hydroxyalkyl, (C₆-C₁₆)-Aryloxy-(C₁-C₈)-alkyl, (C₇-C₁₆)-Aralkoxy-(C₁-C₈)-alkyl, (C₆-C₁₂)-Aryloxy-(C₁-C₈)-alkoxy-(C₁-C₆)-alkyl, (C₇-C₁₂)-Aralkyloxy-(C₁-C₈)-alkoxy-(C₁-C₆)-alkyl, -O-[CH₂₋]ₓC_{f}H_{(2f+1-g)} F_{g}, -OCF₂Cl, -OCF₂-CHFCl,
(C₁-C₁₂)-Alkylcarbonyl, (C₃-C₈)-Cycloalkylcarbonyl, (C₆-C₁₂)-Arylcarbonyl, (C₇-C₁₆)-Aralkylcarbonyl, Cinnamoyl, (C₂-C₁₂) Alkenylcarbonyl, (C₂-C₁₂)-Alkinylcarbonyl,
(C₁-C₁₂)-Alkoxycarbonyl, (C₁-C₁₂)-Alkoxy-(C₁-C₁₂)-alkoxycarbonyl, (C₆-C₁₂)- Aryloxycarbonyl, (C₇-C₁₆)-Aralkoxycarbonyl, (C₃-C₁₀)-Cycloalkoxycarbonyl, (C₂-C₁₂)-Alkenyloxycarbonyl, (C₂-C₁₂)-Alkinyloxycarbonyl,
(C₁-C₁₂)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy, (C₂-C₁₂)-Alkenylcarbonyloxy, (C₂-C₁₂)-Alkinylcarbonyloxy,
(C₁-C₁₂)-Alkoxycarbonyloxy, (C₁-C₁₂)-Alkoxy-(C₁-C₁₂)-alkoxycarbonyloxy, (C₆-C₁₂)-Aryloxycarbonyloxy, (C₇-C₁₆)-Aralkyloxycarbonyloxy, (C₃-C₈)-Cycloalkoxycarbonyloxy, (C₂-C₁₂)-Alkenyloxycarbonyloxy, (C₂-C₁₂)-Alkinyloxycarbonyloxy,
Carbamoyl, N-(C₁-C₁₂)-Alkylcarbamoyl, N, N-Di-(C₁-C₁₂)-alkylcarbamoyl, N-(C₃-C₈)-Cycloalkylcarbamoyl, N,N-Dicyclo-(C₃-C₈)-alkylcarbamoyl, N-(C₁-C₁₀)-Alkyl-N-(C₃-C₈)-Cycloalkylcarbamoyl, N-((C₃-C₈)-Cycloalkyl-(C₁-C₆)-alkyl)carbamoyl, N-(C₁-C₆)-Alkyl-N-((C₃-C₈)-cycloalkyl-(C₁-C₆)-alkyl)carbamoyl, N-(+)-Dehydroabietylcarbamoyl, N-(C₁-C₆)-Alkyl-N-(+)-dehydroabietylcarbamoyl, N-(C₆-C₁₂)-Arylcarbamoyl, N-(C₇-C₁₆)-Aralkylcarbamoyl, N-(C₁-C₁₀)-Alkyl-N-(C₆-C₁₆)-arylcarbamoyl, N-(C₁-C₁₀)-Alkyl-N-(C₇-C₁₆)-aralkylcarbamoyl, N-((C₁-C₁₀)-Alkoxy-(C₁-C₁₀)alkyl)carbamoyl, N-((C₆-C₁₆)-Aryloxy-(C₁-C₁₀)alkyl)carbamoyl, N-((C₇-C₁₆)-Aralkyloxy-(C₁-C₁₀)-alkyl)carbamoyl,
N-(C₁-C₁₀)-Alkyl-N-((C₁-C₁₀)-alkoxy-(C₁-C₁₀)-alkyl)carbamoyl,
N-(C₁-C₁₀)-Alkyl-N-((C₆-C₁₂)-aryloxy-(C₁-C₁₀)-alkyl)carbamoyl,
N-(C₁-C₁₀)-Alkyl-N-((C₇-C₁₆)-aralkyloxy-(C₁-C₁₀)-alkyl)carbamoyl,
CON(CH₂)ₕ, worin eine CH₂-Gruppe durch O, S, N-(C₁-C₈)-Alkylimino, N-(C₃-C₈)-Cycloalkylimino, N-(C₃-C₈)-Cycloalkyl-(C₁-C₄)-alkylimino, N-(C₆-C₁₂)-Arylimino, N-(C₇-C₁₆)-Aralkylimino oder N-(C₁-C₄)-Alkoxy-(C₁-C₆)-alkylimino ersetzt ein kann und h 3 bis 6 bedeutet, oder mit
einem Carbamoyl-Rest der allgemeinen Formel II 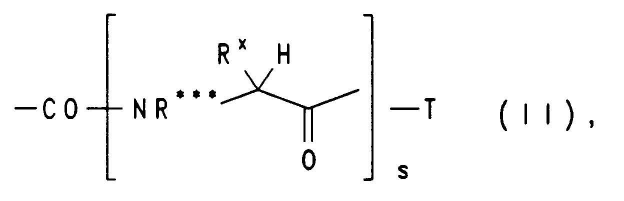 worin
R^{x} der Substituent einer α-Aminosäure bedeutet, zu denen die L-und D-Aminosäuren zählen,
s 1, 2, 3, 4 oder 5 und
T OH, OR oder NR*R** bedeutet, wobei
R*** Wasserstoff oder (C₁-C₄)-Alkyl,
R* und R** gleich oder verschieden sind und Wasserstoff, (C₆-C₁₂)-Aryl, (C₇-C₁₁)-Aralkyl, (C₁-C₈)-Alkyl, (C₃-C₈)-Cycloalkyl, (+)-Dehydroabietyl, (C₁-C₈)-Alkoxy-(C₁-C₈)alkyl, (C₇-C₁₂)-Aralkoxy-(C₁-C₈)alkyl, (C₆-C₁₂)-Aryloxy-(C₁-C₈)-alkyl, (C₁-C₁₀)-Alkanoyl, ggf. substituiertes (C₇-C₁₆)-Aralkanoyl, ggf. substituiertes (C₆-C₁₂)-Aroyl bedeuten, oder
R* und R** gemeinsam für -[CH₂]ₕ stehen, worin eine CH₂ Gruppe durch O, S, SO, SO₂, N-Acylimino, N-(C₁-C₁₀)-Alkoxycarbonylimino, N-(C₁-C₈)-Alkylimino, N-(C₃-C₈)-Cycloalkylimino, N-(C₃-C₈)-Cycloalkyl-(C₁-C₄)alkylimino, N-(C₆-C₁₂)-Arylimino, N-(C₇-C₁₆)-Aralkylimino oder N-(C₁-C₄)-Alkoxy-(C₁-C₆)-alkylimino ersetzt sein kann und h 3 bis 7 bedeutet, oder mit
Carbamoyloxy, N-(C₁-C₁₂)-Alkylcarbamoyloxy, N,N-Di-(C₁-C₁₂)-alkylcarbamoyloxy, N-(C₃-C₈)-Cycloalkylcarbamoyloxy, N-(C₆-C₁₂)-Arylcarbamoyloxy, N-(C₇-C₁₆)-Aralkylcarbamoyloxy, N-(C₁-C₁₀)-Alkyl-N-(C₆-C₁₂)-arylcarbamoyloxy, N-(C₁-C₁₀)-Alkyl-N-(C₇-C₁₆)-Aralkylcarbamoyloxy, N-((C₁-C₁₀)-alkyl))carbamoyloxy, N-((C₆-C₁₂)-Aryloxy-(C₁-C₁₀)-alkyl)carbamoyloxy, N-((C₇-C₁₆)-Aralkyloxy-(C₁-C₁₀)-alkyl)carbamoyloxy, N-(C₁-C₁₀)-Alkyl-N-((C₁-C₁₀)-alkoxy-(C₁-C₁₀)-alkyl)carbamoyloxy, N-(C₁-C₁₀)-Alkyl-N-((C₆-C₁₂)-aryloxy-(C₁-C₁₀)-alkyl)carbamoyloxy, N-(C₁-C₁₀)-Alkyl-N-((C₇-C₁₆)-aralkyloxy-(C₁-C₁₀)-alkyl)carbamoyloxy,
Amino, (C₁-C₁₂)-Alkylamino, Di-(C₁-C₁₂)alkylamino, (C₃-C₈)-Cycloalkylamino, (C₃-C₁₂)-Alkenylamino, (C₃-C₁₂) Alkinylamino, N-(C₆-C₁₂)-Arylamino, N-(C₇-C₁₁)-Aralkylamino, N-Alkyl-Aralkylamino, N-Alkyl-Arylamino, (C₁-C₁₂)-Alkoxyamino, (C₁-C₁₂)-Alkoxy-N-(C₁-C₁₀)-alkylamino,
(C₁-C₁₂)-Alkanoylamino, (C₃-C₈)-Cycloalkanoylamino, (C₆-C₁₂)-Aroylamino, (C₇-C₁₆)-Aralkanoylamino, (C₁-C₁₂)-Alkanoyl-N-(C₁-C₁₀)-alkylamino, (C₃-C₈)-Cycloalkanoyl-N-(C₁-C₁₀)-alkylamino, (C₆-C₁₂)-Aroyl-N-(C₁-C₁₀)alkylamino, (C₇-C₁₁)-Aralkanoyl-N-(C₁-C₁₀)-alkylamino,
(C₁-C₁₂)-Alkanoylamino-(C₁-C₈)-alkyl, (C₃-C₈)-Cycloalkanoylamino-(C₁-C₈)alkyl, (C₆-C₁₂)-Aroylamino-(C₁-C₈)-alkyl, (C₇-C₁₆)-Aralkanoylamino-(C₁-C₈)-alkyl, Amino-(C₁-C₁₀)-alkyl, N-(C₁-C₁₀)-alkylamino-(C₁-C₁₀)alkyl, N,N-Di(C₁-C₁₀)alkylamino-(C₁-C₁₀)alkyl, (C₃-C₈)-Cycloalkylamino-(C₁-C₁₀)-alkyl, (C₁-C₁₂)-Alkylmercapto, (C₁-C₁₂)-Alkylsulfinyl, (C₁-C₁₂)-Alkylsulfonyl, (C₆-C₁₂)-Arylmercapto, (C₆-C₁₂)-Arylsulfinyl,
(C₆-C₁₂)-Arylsulfonyl, (C₇-C₁₆)-Aralkylmercapto, (C₇-C₁₆)-Aralkylsulfinyl, (C₇-C₁₆)-Aralkylsulfonyl,
Sulfamoyl, N-(C₁-C₁₀)-Alkylsulfamoyl, N,N-Di-(C₁-C₁₀)-alkylsulfamoyl, (C₃-C₈)-Cycloalkylsulfamoyl, N-(C₆-(C₁₂)-Arylsulfamoyl, N-(C₇-C₁₆)-Aralkylsulfamoyl, N-(C₁-C₁₀)-Alkyl-N-(C₆-C₁₂)-arylsulfamoyl, N-(C₁-C₁₀)-Alkyl-N-(C₇-C₁₆)-aralkylsulfamoyl, (C₁-C₁₀)-Alkyl-sulfonamido, N-((C₁-C₁₀)-alkyl)-(C₁-C₁₀)-alkylsulfonamido, (C₇-C₁₆)-Aralkylsulfonamido, oder mit N-((C₁-C₁₀)-alkyl-(C₇-C₁₆)-aralkylsulfonamido substituiert sind, wobei die Reste, die einen Arylrest enthalten ihrerseits am Aryl substituiert sein können durch 1 bis 5 gleiche oder verschiedene Reste aus der Reihe:
Hydroxy, Halogen, Cyano, Trifluormethyl, Nitro, Carboxy, (C₁-C₁₂)-Alkyl, (C₃-C₈)-Cycloalkyl, (C₆-C₁₂)-Aryl, (C₇-C₁₆)-Aralkyl, (C₁-C₁₂)-Alkoxy, (C₁-C₁₂)-Alkoxy-(C₁-C₁₂)alkyl, (C₁-C₁₂)-Alkoxy-(C₁-C₁₂)alkoxy, (C₆-C₁₂)-Aryloxy, (C₇-C₁₆)-Aralkyloxy, (C₁-C₈)-Hydroxyalkyl,
(C₁-C₁₂)-Alkylcarbonyl, (C₃-C₈)-Cycloalkylcarbonyl, (C₆-C₁₂)-Arylcarbonyl, (C₇-C₁₆)-Aralkylcarbonyl,
(C₁-C₁₂)-Alkoxycarbonyl, (C₁-C₁₂)-Alkoxy-(C₁-C₁₂)-alkoxycarbonyl, (C₆-C₁₂)- Aryloxycarbonyl, (C₇-C₁₆)-Aralkoxycarbonyl, (C₃-C₈)-Cycloalkoxycarbonyl, (C₂-C₁₂)-Alkenyloxycarbonyl, (C₂-C₁₂)-Alkinyloxycarbonyl,
(C₁-C₁₂)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy, (C₂-C₁₂)-Alkenylcarbonyloxy, (C₂-C₁₂)-Alkinylcarbonyloxy,
(C₁-C₁₂)-Alkoxycarbonyloxy, (C₁-C₁₂)-Alkoxy-(C₁-C₁₂)-alkoxycarbonyloxy, (C₆-C₁₂)-Aryloxycarbonyloxy, (C₇-C₁₆)-Aralkyloxycarbonyloxy, (C₃-C₈)-Cycloalkoxycarbonyloxy, (C₂-C₁₂)-Alkenyloxycarbonyloxy, (C₂-C₁₂)-Alkinyloxycarbonyloxy,
Carbamoyl, N-(C₁-C₁₂)-Alkylcarbamoyl, N, N-Di-(C₁-C₁₂)-alkylcarbamoyl, N-(C₃-C₈)-Cycloalkylcarbamoyl, N,N-Dicyclo-(C₃-C₈)-alkylcarbamoyl, N-(C₁-C₁₀)-Alkyl-N-(C₃-C₈)-cycloalkylcarbamoyl, N-((C₃-C₈)-Cycloalkyl-(C₁-C₆)-alkyl)carbamoyl, N-(C₁-C₆)-Alkyl-N-((C₃-C₈)-cycloalkyl-(C₁-C₆)-alkyl)carbamoyl, N-(+)-Dehydroabietylcarbamoyl, N-(C₁-C₆)-Alkyl-N-(+)-dehydroabietylcarbamoyl, N-(C₆-C₁₂)-Arylcarbamoyl, N-(C₇-C₁₆)-Aralkylcarbamoyl, N-(C₁-C₁₀)-Alkyl-N-(C₆-C₁₆)-arylcarbamoyl, N-(C₁-C₁₀)-Alkyl-N-(C₇-C₁₆)-aralkylcarbamoyl, N-((C₁-C₁₀)-Alkoxy-(C₁-C₁₀)-alkyl)carbamoyl, N-((C₆-C₁₆)-Aryloxy-(C₁-C₁₀)-alkyl)carbamoyl, N-((C₇-C₁₆)-Aralkyloxy-(C₁-C₁₀)-alkyl)carbamoyl, N-(C₁-C₁₀)-Alkyl-N-((C₁-C₁₀)-alkoxy-(C₁-C₁₀)-alkyl)carbamoyl, N-(C₁-C₁₀)-Alkyl-N-((C₆-C₁₂)-aryloxy-(C₁-C₁₀)-alkyl)carbamoyl, N-(C₁-C₁₀)-Alkyl-N-((C₇-C₁₆)-aralkyloxy-(C₁-C₁₀)-alkyl)carbamoyl, CON(CH₂)ₕ worin eine CH₂-Gruppe durch O, S, N-(C₁-C₈)Alkylimino, N-(C₃-C₈)-Cycloalkylimino, N-(C₃-C₈)-Cycloalkyl-(C₁-C₄)alkylimino, N-(C₆-C₁₂)-Arylimino, N-(C₇-C₁₆)-Aralkylimino oder N-(C₁-C₄)-Alkoxy-(C₁-C₆)-alkylimino ersetzt ein kann und h 3 bis 7 bedeutet,
Carbamoyloxy, N-(C₁-C₁₂)-Alkylcarbamoyloxy, N,N-Di-(C₁-C₁₂)-alkylcarbamoyloxy, N-(C₃-C₈)-Cycloalkylcarbamoyloxy, N-(C₆-C₁₆)-Arylcarbamoyloxy, N-(C₇-C₁₆)-Aralkylcarbamoyloxy, N-(C₁-C₁₀)-Alkyl-N-(C₆-C₁₂)-arylcarbamoyloxy, N-(C₁-C₁₀)-Alkyl-N-(C₇-C₁₆)-Aralkylcarbamoyloxy, N-((C₁-C₁₀)-alkyl))carbamoyloxy, N-((C₆-C₁₂)-Aryloxy-(C₁-C₁₀)-alkyl)-carbamoyloxy, N-((C₇-C₁₆)-Aralkyloxy-(C₁-C₁₀)-alkyl)carbamoyloxy, N-(C₁-C₁₀)-Alkyl-N-((C₁-C₁₀)-alkoxy-(C₁-C₁₀)-alkyl)carbamoyloxy, N-(C₁-C₁₀)-Alkyl-N-((C₆-C₁₂)-aryloxy-(C₁-C₁₀)-alkyl)carbamoyloxy, N-(C₁-C₁₀)-Alkyl-N-((C₇-C₁₆)-aralkyloxy-(C₁-C₁₀)-alkyl)carbamoyloxy,
Amino, (C₁-C₁₂)-Alkylamino, Di-(C₁-C₁₂)alkylamino, (C₃-C₈)-Cycloalkylamino, (C₃-C₁₂)-Alkenylamino, (C₃-C₁₂) Alkinylamino, N-(C₆-C₁₂)-Arylamino, N-(C₇-C₁₁)-Aralkylamino, N-Alkyl-Aralkylamino, N-Alkyl-Arylamino, (C₁-C₁₂)-Alkoxyamino, (C₁-C₁₂)-Alkoxy-N-(C₁-C₁₀)-alkylamino,
(C₁-C₁₂)-Alkanoylamino, (C₃-C₈)-Cycloalkanoylamino, (C₆-C₁₂)-Aroylamino, (C₇-C₁₆)-Aralkanoylamino, (C₁-C₁₂)-Alkanoyl-N-(C₁-C₁₀)-alkylamino, (C₃-C₈)-Cycloalkanoyl-N-(C₁-C₁₀)-alkylamino, (C₆-C₁₂)-Aroyl-N-(C₁-C₁₀)-alkylamino, (C₇-C₁₁)-Aralkanoyl-N-(C₁-C₁₀)-alkylamino,
(C₁-C₁₂)-Alkanoylamino-(C₁-C₈)-alkyl, (C₃-C₈)-Cycloalkanoylamino-(C₁-C₈)-alkyl, (C₆-C₁₂)-Aroylamino-(C₁-C₈)-alkyl, (C₇-C₁₆)-Aralkanoylamino-(C₁-C₈)-alkyl, Amino-(C₁-C₁₀)-alkyl, N-(C₁-C₁₀)alkylamino-(C₁-C₁₀)-alkyl, N,N-Di-(C₁-C₁₀)-alkylamino-(C₁-C₁₀)-alkyl, (C₃-C₈)-Cycloalkylamino-(C₁-C₁₀)-alkyl,
(C₁-C₁₂)-Alkylmercapto, (C₁-C₁₂)-Alkylsulfinyl, (C₁-C₁₂)-Alkylsulfonyl, (C₆-C₁₆)-Arylmercapto, (C₆-C₁₆)-Arylsulfinyl, (C₆-C₁₆)-Arylsulfonyl, (C₇-C₁₆)-Aralkylmercapto, (C₇-C₁₆)-Aralkylsulfinyl, (C₇-C₁₆)-Aralkylsulfonyl,
R¹ und R³ gleich oder verschieden sind und Wasserstoff, (C₁-C₈)-Alkyl, (C₁-C₈)-Alkoxy, Halogen, Nitril, Hydroxy, Amino, ggf. mono-oder disubstituiert mit (C₁-C₆)-Alkyl, oder Hydroxy-(C₁-C₆)-alkyl, (C₁-C₆)-Alkylcarbonyloxy bedeutet,
R² eine carbonsäureisostere Gruppe oder einen Rest -[CH₂]ₚ-CO-NR⁵R⁶ bedeutet, wobei
p = 0, 1, 2, 3 oder 4,
R⁵ Wasserstoff, (C₁-C₆)-Alkyl oder eine N-Schutzgruppe wie (C₁-C₈)-Alkanoyl, (C₁-C₆)-Alkylcarbamoyl, (C₁-C₆)-Alkoxycarbonyl, Benzyloxycarbonyl, (C₁-C₁₀)-Acyloxy-(C₁-C₆)-alkyl, Benzoyloxy-(C₁-C₆)-alkyl, Benzyloxycarbonyloxy-(C₁-C₆)-alkyl oder (C₁-C₆)-Alkoxycarbonyloxy-(C₁-C₆)-alkyl, ein 1, 2, 3 oder 4-wertiges physiologisch verwendbares Kation, ggf. 1-3fach substituiert mit (C₁-C₈)-Hydroxyalkyl, (C₁-C₄)-Alkoxy-(C₁-C₈)-alkyl, Phenyl, Benzyl oder (C₁-C₈)-Alkyl, welches seinerseits 1-3fach substituiert sein kann mit Hydroxy oder (C₁-C₄)-Alkoxy oder ein Kation eines basischen Aminosäurederivates bedeutet,
R⁶ einen Rest der Formel I, ausgenommen -SO₂H, bedeutet
-G-K-[C-U]ᵣ-D-W (I)
in welchem
G -SO, -SO₂- oder -CO- bedeutet,
K eine Bindung oder -NR⁷- bedeutet,
C eine Bindung oder
einen verzweigten oder unverzweigten aliphatischen (C₁-C₁₆)-Alkandiyl- oder cycloaliphatischen (C₃-C₁₀)-Alkandiylrest, einen verzweigten oder unverzweigten (C₂-C₁₆)-Alkendiyl- oder Cycloalkendiyl-Rest, einen (C₂-C₁₆)-Alkindiyl-Rest oder einen (C₂-C₁₆)-Alkenindiyl-Rest bedeutet, der jeweils eine oder mehrere C-C-Mehrfachbindungen enthalten kann,
U eine Bindung, Wasserstoff oder
einen Rest aus der Reihe folgender Heteroatomgruppierungen bedeutet
-CO-, -O(CO)-, -(CO)-O-, -(CO)NR-, -NR(CO)-, -O-, - SO-, -SO₂-, -NR, worin R (C₁-C₃)-Alkyl oder Wasserstoff bedeutet,
r 1, 2, 3 oder 4 ist,
D eine Bindung, Wasserstoff oder
einen verzweigten oder unverzweigten aliphatischen (C₁-C₁₀)-Alkandiyl-Rest,
einen verzweigten oder unverzweigten (C₁-C₁₀)-Alkendiyl-Rest, einen (C₂-C₁₀)-Alkindiyl-Rest oder einen (C₂-C₁₀)-Alkenindiyl-Rest bedeutet, der jeweils eine oder mehrere C-C-Mehrfachbindungen enthalten kann,
W eine Bindung, Wasserstoff oder
einen (C₃-C₁₀)-cycloaliphatischen Alkyl-, Alkenyl-, Alkinyl- oder Alkeninyl-Rest,
einen (C₆-C₁₆)-Arylrest oder einen 5- oder 6-gliedrigen Heteroarylrest bedeutet, wobei mindestens eine der Variablen C, D oder W nicht eine Bindung bedeuten und U nur dann in der Bedeutung einer Heteratomgruppierung steht, wenn C nicht eine Bindung bedeutet oder wenn D und/oder W nicht eine Bindung bedeuten und
C,D und/oder W, sofern diese keine Bindung oder Wasserstoff bedeuten, vorzugsweise ihrerseits substituiert sind durch eine Kombination von bis zu 5 gleichen oder verschiedenen Substituenten, die den Substituenten von V entsprechen,
R⁷ Wasserstoff, (C₁-C₈)-Alkyl, (C₆-C₁₂)-Aryl, (C₇-C₁₁)-Aralkyl, Heteroaryl, bedeutet,
wobei die vorstehenden Reste 1, 2 oder 3 gleiche oder verschiedene Substituenten aus der Reihe
Fluor, Chlor, Trifluormethyl, Nitril, (C₁-C₆)-Alkyl, (C₁-C₆)-Alkoxy, (C₁-C₆)-Alkylcarbamoyl, Di-(C₁-C₆)-alkylcarbamoyl, (C₃-C₆)-Cycloalkylcarbamoyl, (C₁-C₆)-Alkoxycarbonyl tragen können,
R⁴ einen verzweigten oder unverzweigten (C₇-C₂₀)-Alkylrest, einen (C₆-C₁₆)-Arylrest, einen (C₇-C₁₆)-Aralkylrest, einen Heteroarylrest oder einen Heteroaralkylrest bedeutet,
wobei diese Reste substituiert sind mit einem oder mehreren Resten, die den Substituenten von V entsprechen, oder
R¹ und R⁴ eine Kette [CH₂]ₒ bilden können, in welcher eine oder zwei CH₂-Gruppen der gesättigten oder mit einer C=C-Doppelbindung ungesättigten Kette gegebenenfalls durch O, S, SO, SO₂ oder NR' ersetzt sind, o = 3, 4 oder 5 bedeutet und
R^{Y} Wasserstoff, (C₆-C₁₂)-Aryl, (C₅-C₈)-Alkyl, (C₁-C₈)-Alkoxy-(C₁-C₈)alkyl, (C₇-C₁₂)-Aralkoxy-(C₁-C₈)alkyl, (C₆-C₁₂)Aryloxy-(C₁-C₈)-alkyl, (C₁-C₁₀)-Alkanoyl, ggf. substituiertes (C₇-C₁₆)-Aralkanoyl, ggf. substituiertes (C₆-C₁₂)-Aroyl bedeuten, und
f = 1 bis 8,
g = 0,1 bis (2f+1),
x = 0 bis 3,
h = 3 bis 6 bedeuten.

2. Verbindungen der Formel 1 nach Anspruch 1, in der
Q = O,
X = O,
Y = N oder CR³,
m = 0,
A (C₁-C₃)-Alkylen, das gegebenenfalls einfach oder zweifach substituiert ist mit Halogen, Cyano, Trifluormethyl, (C₁-C₆)-Alkyl, (C₁-C₆)-Hydroxyalkyl, (C₁-C₆)-Alkoxy, -O-[CH₂]ₓ-C_{f}H_{(2f+1-g)}F_{g} oder
A -CHR^{x}- bedeutet, wobei R^{x} einen der Substituenten des α-C-Atoms einer α-Aminosäure bedeutet, insbesondere einer natürlichen L-Aminosäure und ihres D-Isomeren,
B -CO₂V bedeutet, wobei
V den Rest eines Alkohols VOH bedeutet, worin V
(C₁-C₁₀)-Acyloxy-(C₁-C₆)-alkyl, vorzugsweise (C₁-C₁₀)-Alkanoyloxy-(C₁-C₆)-alkyl, Benzoyloxy-(C₁-C₆)-alkyl, Benzyloxycarbonyloxy-(C₁-C₆)-alkyl oder (C₁-C₆)-Alkoxycarbonyloxy-(C₁-C₆)-alkyl, oder
einen verzweigten, unverzweigten oder cyclischen aliphatischen (C₁-C₁₀)-Alkyl-Rest, oder
einen verzweigten oder unverzweigten (C₂-C₁₀)-Alkenyl-Rest oder einen (C₂-C₁₂)-Alkinyl-Rest, der jeweils eine oder mehrere C-C-Mehrfachbindungen enthalten kann, oder
einen (C₆-C₁₂)-Aryl-Rest, einen (C₇-C₁₁)-Aralkyl-Rest oder einen Heteroaryl- oder Heteroaralkyl-Rest bedeutet,
wobei die vorstehenden Reste einen oder zwei Substituenten aus der Reihe (C₁-C₈)-Alkyl, (C₃-C₈)-Cycloalkyl, Fluor, Chlor, Hydroxy, (C₁-C₆)-Alkoxy, (C₁-C₆)-Alkoxy-(C₁-C₆)alkoxy, (C₆-C₁₂)-Aryloxy, (C₇-C₁₂)-Aralkyloxy,
(C₁-C₈)-Alkylcarbonyl, (C₃-C₈)-Cycloalkylcarbonyl, (C₆-C₁₂)-Arylcarbonyl, (C₇-C₁₂)-Aralkylcarbonyl,
(C₁-C₈)-Alkoxycarbonyl, (C₁-C₆)-Alkoxy-(C₁-C₆)-alkoxycarbonyl, (C₆-C₁₂)-Aryloxycarbonyl, (C₇-C₁₂)-Aralkoxycarbonyl, (C₃-C₈)-Cycloalkoxycarbonyl,
(C₁-C₈)-Alkylcarbonyloxy, (C₃-C₈)-Cycloalkylcarbonyloxy, (C₆-C₁₂)-Arylcarbonyloxy, (C₇-C₁₂)-Aralkylcarbonyloxy,
(C₁-C₈)-Alkoxycarbonyloxy, (C₁-C₆)-Alkoxy-(C₁-C₆)-alkoxycarbonyloxy, (C₆-C₁₂)-Aryloxycarbonyloxy, (C₇-C₁₂)-Aralkyloxycarbonyloxy, (C₃-C₈)-Cycloalkoxycarbonyloxy,
Carbamoyl, N-(C₁-C₈)-Alkylcarbamoyl, N,N-Di-(C₁-C₈)-alkylcarbamoyl, N-(C₃-C₈)-Cycloalkylcarbamoyl,
N-((C₁-C₆)-Alkoxy-(C₁-C₆)-alkyl)carbamoyl,
Amino, (C₁-C₆)-Alkylamino, Di-(C₁-C₆)-alkylamino, (C₃-C₈)-Cycloalkylamino,N-(C₆-C₁₂)-Arylamino, N-(C₇-C₁₁)-Aralkylamino, N-(C₁-C₅)-Alkyl-(C₆-C₁₂)-arylamino,
(C₁-C₈)-Alkanoylamino, (C₃-C₈)-Cycloalkanoylamino, (C₆-C₁₂)-Aroylamino, (C₇-C₁₂)-Aralkanoylamino, (C₁-C₈)-Alkanoyl-N-(C₁-C₆)-alkylamino, (C₃-C₈)-Cycloalkanoyl-N-(C₁-C₆)-alkylamino, (C₆-C₁₂)-Aroyl-N-(C₁-C₆)-alkylamino, (C₇-C₁₁)-Aralkanoyl-N-(C₁-C₆)-alkylamino tragen können, und
wobei die Reste, die einen Arylrest enthalten, insbesondere substituiert sind mit bis zu 3 Substituenten aus der Reihe
Hydroxy, Fluor, Chlor, Cyano, Trifluormethyl, (C₁-C₆)-Alkyl, (C₃-C₈)-Cycloalkyl, (C₁-C₈)-Alkoxy,
(C₁-C₆)-Alkylcarbonyl, (C₃-C₈)-Cycloalkylcarbonyl,
(C₁-C₆)-Alkoxycarbonyl, (C₃-C₈)-Cycloalkoxycarbonyl,
(C₁-C₆)-Alkylcarbonyloxy, (C₃-C₈)-Cycloalkylcarbonyloxy,
(C₁-C₆)-Alkoxycarbonyloxy, (C₃-C₈)-Cycloalkoxycarbonyloxy,
Carbamoyl, N-(C₁-C₆)-Alkylcarbamoyl, N,N-Di-(C₁-C₆)-alkylcarbamoyl, N-(C₃-C₈)-Cycloalkylcarbamoyl,
N-((C₁-C₆)-Alkoxy-(C₁-C₆)-alkyl)carbamoyl,
N-(C₁-C₆)-Alkyl-N-((C₁-C₆)-alkoxy-(C₁-C₆)-alkyl)carbamoyl,
Carbamoyloxy, N-(C₁-C₆)-Alkylcarbamoyloxy, N,N-Di-(C₁-C₆)-alkylcarbamoyloxy, N-(C₃-C₈)-Cycloalkylcarbamoyloxy,
(C₁-C₆)-Alkanoylamino, (C₃-C₈)-Cycloalkanoylamino,
(C₁-C₆)Alkylmercapto, (C₁-C₆)-Alkylsulfinyl, (C₁-C₆)-Alkylsulfonyl und
R¹ und R³ Wasserstoff bedeuten,
R² einen Rest -CO-NR⁵R⁶ bedeutet, wobei
R⁵ Wasserstoff, (C₁-C₆)-Alkyl oder eine N-Schutzgruppe wie (C₁-C₈)-Alkanoyl, (C₁-C₆)-Alkylcarbamoyl, (C₁-C₆)-Alkoxycarbonyl, Benzyloxycarbonyl, (C₁-C₁₀)-Acyloxy-(C₁-C₆)-alkyl, vorzugsweise (C₁-C₁₀)-Alkanoyloxy-(C₁-C₆)-alkyl, Benzoyloxy-(C₁-C₆)-alkyl, Benzyloxycarbonyloxy-(C₁-C₆)-alkyl oder (C₁-C₆)-Alkoxycarbonyloxy-(C₁-C₆)-alkyl, ein 1, 2, 3 oder 4-wertiges physiologisch verwendbares Kation, insbesondere Na⊕, K⊕, Mg²⊕, Ca²⊕, Al³⊕ oder ein Ammoniumion, ggf. 1-3fach substituiert mit (C₁-C₈)-Hydroxyalkyl, (C₁-C₄)-Alkoxy-(C₁-C₈)-alkyl, Phenyl, Benzyl oder (C₁-C₈)-Alkyl, welches seinerseits 1-3fach substituiert sein kann mit Hydroxy oder (C₁-C₄)-Alkoxy, oder ein Kation eines basischen Aminosäurederivates bedeutet,
R⁶ einen Rest der Formel I, ausgenommen -SO₂H, bedeutet
-G-K-[C-U]ᵣ-D-W (I)
in welchem
G -SO₂- oder -CO- bedeutet,
K eine Bindung,
C eine Bindung oder
einen verzweigten oder unverzweigten aliphatischen (C₁-C₁₂)-Alkandiyl-rest, oder
einen verzweigten oder unverzweigten (C₂-C₁₂)-Alkendiyl-Rest, einen (C₂-C₁₂)-Alkindiyl-Rest oder einen (C₂-C₁₂)-Alkenindiyl-Rest bedeutet, der eine oder mehrere C-C-Mehrfachbindungen enthalten kann,
U eine Bindung, Wasserstoff oder einen Rest aus der Reihe folgender Heteroatomgruppierungen bedeutet
-(CO)NR-, -NR(CO)-, -O-, -SO-, -SO₂-, worin R (C₂-C₃)-Alkyl oder Wasserstoff bedeutet,
r 1 oder 2 ist,
D eine Bindung, Wasserstoff oder
einen verzweigten oder unverzweigten aliphatischen (C₁-C₈)-Alkandiyl-Rest,
einen verzweigten oder unverzweigten (C₂-C₈)-Alkendiyl-Rest oder (C₂-C₈)-Alkindiyl-Rest bedeutet und
W eine Bindung, Wasserstoff oder
einen (C₃-C₁₀) cycloaliphatischen Alkyl-, Alkenyl-, Alkinyl- oder Alkeninyl-Rest,
einen (C₆-C₁₆)-Arylrest oder einen 5- oder 6-gliedrigen Heteroarylrest bedeutet, wobei mindestens eine der Variablen C, D oder W nicht eine Bindung bedeuten und U nur dann in der Bedeutung einer Heteratomgruppierung steht, wenn C nicht eine Bindung bedeutet oder wenn D und/oder W nicht eine Bindung bedeuten und
C, D und/oder W, sofern diese keine Bindung oder Wasserstoff bedeuten, vorzugsweise ihrerseits substituiert sind durch eine Kombination von bis zu 5 gleichen oder verschiedenen Substituenten aus der Reihe
Hydroxy, Halogen, Cyano, Trifluormethyl, Nitro, Carboxy, (C₁-C₁₂)-Alkyl, (C₃-C₈)-Cycloalkyl, (C₃-C₈)-Cycloalkyl-(C₁-C₁₂)-alkyl, (C₃-C₈)-Cycloalkoxy, (C₃-C₈)-Cycloalkyl-(C₁-C₁₂)-alkoxy, (C₃-C₈)-Cycloalkyloxy-(C₁-C₁₂)-alkyl, (C₃-C₈)-Cycloalkyloxy-(C₁-C₁₂)-alkoxy, (C₃-C₈)-Cycloalkyl-(C₁-C₈)-alkyl-(C₁-C₆)-alkoxy, (C₃-C₈)-Cycloalkyl-(C₁-C₈)-alkoxy-(C₁-C₆)-alkyl, (C₃-C₈)-Cycloalkyloxy-(C₁-C₈)-alkoxy-(C₁-C₆)-alkyl, (C₃-C₈)-Cycloalkoxy-(C₁-C₈)-alkoxy-(C₁-C₈)-alkoxy, (C₆-C₁₂)-Aryl, (C₇-C₁₆)-Aralkyl, (C₂-C₁₂)-Alkenyl, (C₂-C₁₂)-Alkinyl, (C₁-C₁₂)-Alkoxy, (C₁-C₁₂)-Alkoxy-(C₁-C₁₂)alkyl, (C₁-C₁₂)-Alkoxy-(C₁-C₁₂)alkoxy, (C₁-C₁₂)-Alkoxy-(C₁-C₈)-alkoxy-(C₁-C₈)-alkyl, (C₆-C₁₂)-Aryloxy, (C₇-C₁₆)-Aralkyloxy, (C₆-C₁₂)-Aryloxy-(C₁-C₆)-alkoxy, (C₇-C₁₆)-Aralkoxy-(C₁-C₆)-alkoxy, (C₁-C₈)-Hydroxyalkyl, (C₆-C₁₆)-Aryloxy-(C₁-C₈)-alkyl, (C₇-C₁₆)-Aralkoxy-(C₁-C₈)-alkyl, (C₆-C₁₂)-Aryloxy-(C₁-C₈)-alkoxy-(C₁-C₆)-alkyl, (C₇-C₁₂)-Aralkyloxy-(C₁-C₈)-alkoxy-(C₁-C₆)-alkyl, -O-[CH₂₋]ₓC_{f}H_{(2f+1-g)}F_{g}, -OCF₂Cl, -OCF₂-CHFCl,
(C₁-C₁₂)-Alkylcarbonyl, (C₃-C₈)-Cycloalkylcarbonyl, (C₆-C₁₂)-Arylcarbonyl, (C₇-C₁₆)-Aralkylcarbonyl, Cinnamoyl, (C₂-C₁₂) Alkenylcarbonyl, (C₂-C₁₂)-Alkinylcarbonyl,
(C₁-C₁₂)-Alkoxycarbonyl, (C₁-C₁₂)-Alkoxy-(C₁-C₁₂)-alkoxycarbonyl, (C₆-C₁₂)- Aryloxycarbonyl, (C₇-C₁₆)-Aralkoxycarbonyl, (C₃-C₁₀)-Cycloalkoxycarbonyl, (C₂-C₁₂)-Alkenyloxycarbonyl, (C₂-C₁₂)-Alkinyloxycarbonyl,
(C₁-C₁₂)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy, (C₂-C₁₂)-Alkenylcarbonyloxy, (C₂-C₁₂)-Alkinylcarbonyloxy,
Carbamoyl, N-(C₁-C₁₂)-Alkylcarbamoyl, N, N-Di-(C₁-C₁₂)-alkylcarbamoyl, N-(C₃-C₈)-Cycloalkylcarbamoyl, N,N-Dicyclo-(C₃-C₈)-alkylcarbamoyl, N-(C₁-C₁₀)-Alkyl-N-(C₃-C₈)-cycloalkylcarbamoyl, N-((C₃-C₈)-Cycloalkyl-(C₁-C₆)-alkyl)carbamoyl, N-(C₁-C₆)-Alkyl-N-((C₃-C₈)-cycloalkyl-(C₁-C₆)-alkyl)carbamoyl, N-(+)-Dehydroabietylcarbamoyl, N-(C₁-C₆)-Alkyl-N-(+)-dehydroabietylcarbamoyl, N-(C₆-C₁₂)-Arylcarbamoyl, N-(C₇-C₁₆)-Aralkylcarbamoyl, N-(C₁-C₁₀)-Alkyl-N-(C₆-C₁₆)-arylcarbamoyl, N-(C₁-C₁₀)-Alkyl-N-(C₇-C₁₆)-aralkylcarbamoyl, N-((C₁-C₁₀)-Alkoxy-(C₁-C₁₀)alkyl)carbamoyl, N-((C₆-C₁₆)-Aryloxy-(C₁-₁₀)-alkyl)carbamoyl, N-((C₇-C₁₆)-Aralkyloxy-(C₁-C₁₀)-alkyl)carbamoyl, N-(C₁-C₁₀)-Alkyl-N-((C₁-C₁₀)-alkoxy-(C₁-C₁₀)-alkyl)carbamoyl, N-(C₁-C₁₀)-Alkyl-N-((C₆-C₁₂)-aryloxy-(C₁-C₁₀)-alkyl)carbamoyl, N-(C₁-C₁₀)-Alkyl-N-((C₇-C₁₆)-aralkyloxy-(C₁-C₁₀)alkyl)carbamoyl, CON(CH₂)ₕ, worin eine CH₂-Gruppe durch O, S, N-(C₁-C₈)Alkylimino, N-(C₃-C₈)-Cycloalkylimino, N-(C₃-C₈)-Cycloalkyl-(C₁-C₄)alkylimino, N-(C₆-C₁₂)-Arylimino, N-(C₇-C₁₆)-Aralkylimino oder N-(C₁-C₄)-Alkoxy-(C₁-C₆)-alkylimino ersetzt ein kann und h 3 bis 6 bedeutet, oder mit
einem Carbamoyl-Rest der allgemeinen Formel II 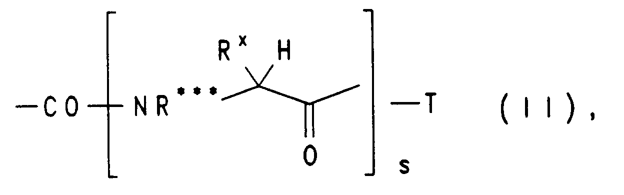 worin
R^{x} der Substituent einer α-Aminosäure bedeutet, zu denen die L-und D-Aminosäuren zählen,
s 1, 2, 3, 4 oder 5 und
T OH, OR oder NR*R** bedeutet, wobei
R*** Wasserstoff oder (C₁-C₄)-Alkyl,
R* und R** gleich oder verschieden sind und Wasserstoff, (C₆-C₁₂)-Aryl, (C₇-C₁₁)-Aralkyl, (C₁-C₈)-Alkyl, (C₃-C₈)-Cycloalkyl, (+)-Dehydroabietyl, (C₁-C₈)-Alkoxy-(C₁-C₈)alkyl, (C₇-C₁₂)-Aralkoxy-(C₁-C₈)alkyl, (C₆-C₁₂)-Aryloxy-(C₁-C₈)-alkyl, (C₁-C₁₀)-Alkanoyl, ggf. substituiertes (C₇-C₁₆)-Aralkanoyl, ggf. substituiertes (C₆-C₁₂)-Aroyl bedeuten, oder
R* und R** gemeinsam für -[CH₂]ₕ stehen, worin eine CH₂ Gruppe durch O, S, SO, SO₂, N-Acylimino, N-(C₁-C₁₀)-Alkoxycarbonylimino, N-(C₁-C₈)-Alkylimino, N-(C₃-C₈)-Cycloalkylimino, N-(C₃-C₈)-Cycloalkyl-(C₁-C₄)alkylimino, N-(C₆-C₁₂)-Arylimino, N-(C₇-C₁₆)-Aralkylimino oder N-(C₁-C₄)-Alkoxy-(C₁-C₆)-alkylimino ersetzt sein kann und h 3 bis 7 bedeutet, oder mit
Carbamoyloxy, N-(C₁-C₁₂)-Alkylcarbamoyloxy, N,N-Di-(C₁-C₁₂)-alkylcarbamoyloxy, N-(C₃-C₈)-Cycloalkylcarbamoyloxy, N-(C₆-C₁₂)-Arylcarbamoyloxy, N-(C₇-C₁₆)-Aralkylcarbamoyloxy, N-(C₁-C₁₀)-Alkyl-N-(C₆-C₁₂)arylcarbamoyloxy, N-(C₁-C₁₀)-Alkyl-N-(C₇-C₁₆)-Aralkylcarbamoyloxy,N-((C₁-C₁₀)-alkyl))carbamoyloxy, N-((C₆-C₁₂)-Aryloxy-(C₁-C₁₀)alkyl)carbamoyloxy,
N-((C₇-C₁₆)-Aralkyloxy-(C₁-C₁₀)alkyl)carbamoyloxy,
N-(C₁-C₁₀)-Alky-N-((C₁-C₁₀)-alkoxy-(C₁-C₁₀)-alkyl)carbamoyloxy, N-(C₁-C₁₀)-Alkyl-N-((C₆-C₁₂)-aryloxy-(C₁-C₁₀)-alkyl)carbamoyoxy, N-(C₁-C₁₀)-Alkyl-N-((C₇-C₁₆)-aralkyloxy-(C₁-C₁₀)alkyl)carbamoyloxy,
Amino, (C₁-C₁₂)-Alkylamino, Di-(C₁-C₁₂)alkylamino, (C₃-C₈)-Cycloalkylamino, (C₃-C₁₂)-Alkenylamino, (C₃-C₁₂)-Alkinylamino, N-(C₆-C₁₂)-Arylamino, N-(C₇-C₁₁)-Aralkylamino, N-Alkyl-Aralkylamino, N-Alkyl-Arylamino, (C₁-C₁₂)-Alkoxyamino, (C₁-C₁₂)-Alkoxy-N-(C₁-C₁₀)-alkylamino,
(C₁-C₁₂)-Alkanoylamino, (C₃-C₈)-Cycloalkanoylamino, (C₆-C₁₂)-Aroylamino, (C₇-C₁₆)-Aralkanoylamino, (C₁-C₁₂)-Alkanoyl-N-(C₁-C₁₀)-alkylamino, (C₃-C₈)-Cycloalkanoyl-N-(C₁-C₁₀)-alkylamino, (C₆-C₁₂)-Aroyl-N-(C₁-C₁₀)alkylamino, (C₇-C₁₁)-Aralkanoyl-N-(C₁-C₁₀)-alkylamino,
(C₁-C₁₂)-Alkanoylamino-(C₁-C₈)-alkyl, (C₃-C₈)-Cycloalkanoylamino-(C₁-C₈)-alkyl, (C₆-C₁₂)-Aroylamino-(C₁-C₈)-alkyl, (C₇-C₁₆)-Aralkanoylamino-(C₁-C₈)-alkyl, Amino-(C₁-C₁₀)-alkyl, N-(C₁-C₁₀)-alkylamino-(C₁-C₁₀)-alkyl, N,N-Di(C₁-C₁₀)-alkylamino-(C₁-C₁₀)alkyl, (C₃-C₈)-Cycloalkylamino-(C₁-C₁₀)-alkyl, (C₁-C₁₂)-Alkylmercapto, (C₁-C₁₂)-Alkylsulfinyl, (C₁-C₁₂)-Alkylsulfonyl, (C₆-C₁₂)-Arylmercapto, (C₆-C₁₂)-Arylsulfinyl, (C₆-C₁₂)-Arylsulfonyl, (C₇-C₁₆)-Aralkylmercapto, (C₇-C₁₆)-Aralkylsulfinyl, (C₇-C₁₆)-Aralkylsulfonyl, bedeuten,
wobei die Reste, die einen Arylrest enthalten ihrerseits am Aryl substituiert sein können durch 1 bis 5 gleiche oder verschiedene Reste aus der Reihe:
Hydroxy, Halogen, Cyano, Trifluormethyl, Nitro, Carboxy, (C₁-C₁₂)-Alkyl, (C₃-C₈)-Cycloalkyl, (C₆-C₁₂)-Aryl, (C₇-C₁₆)-Aralkyl, (C₁-C₁₂)-Alkoxy, (C₁-C₁₂)-Alkoxy-(C₁-C₁₂)alkyl, (C₁-C₁₂)-Alkoxy-(C₁-C₁₂)alkoxy, (C₆-C₁₂)-Aryloxy, (C₇-C₁₆)-Aralkyloxy, (C₁-C₈)-Hydroxyalkyl,
-O-[CH₂]ₓC_{f}H_{(2f+1-g)}F_{g}, OCF₂Cl, OCF₂-CHFCl,
(C₁-C₁₂)-Alkylcarbonyl, (C₃-C₈)-Cycloalkylcarbonyl, (C₆-C₁₂)-Arylcarbonyl, (C₇-C₁₆)-Aralkylcarbonyl,
(C₁-C₁₂)-Alkoxycarbonyl, (C₁-C₁₂)-Alkoxy-(C₁-C₁₂)-alkoxycarbonyl, (C₆-C₁₂)- Aryloxycarbonyl, (C₇-C₁₆)-Aralkoxycarbonyl, (C₃-C₈)-Cycloalkoxycarbonyl, (C₂-C₁₂)-Alkenyloxycarbonyl, (C₂-C₁₂)-Alkinyloxycarbonyl,
(C₁-C₁₂)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy, (C₂-C₁₂)-Alkenylcarbonyloxy, (C₂-C₁₂)-Alkinylcarbonyloxy,
Carbamoyl, N-(C₁-C₁₂)-Alkylcarbamoyl, N, N-Di-(C₁-C₁₂)-alkylcarbamoyl, N-(C₃-C₈)-Cycloalkylcarbamoyl, N,N-Dicyclo-(C₃-C₈)-alkylcarbamoyl, N-(C₁-C₁₀)-Alkyl-N-(C₃-C₈)-cycloalkylcarbamoyl, N-((C₃-C₈)-Cycloalkyl-(C₁-C₆)-alkyl)carbamoyl, N-(C₁-C₆)-Alkyl-N-((C₃-C₈)-cycloalkyl-(C₁-C₆)-alkyl)carbamoyl, N-(+)-Dehydroabietylcarbamoyl, N-(C₁-C₆)-Alkyl-N-(+)-dehydroabietylcarbamoyl, N-(C₆-C₁₂)-Arylcarbamoyl, N-(C₇-C₁₆)-Aralkylcarbamoyl, N-(C₁-C₁₀)-Alkyl-N-(C₆-C₁₆)-arylcarbamoyl, N-(C₁-C₁₀)-Alkyl-N-(C₇-C₁₆)-aralkylcarbamoyl, N-((C₁-C₁₀)-Alkoxy-(C₁-C₁₀)-alkyl)carbamoyl, N-((C₆-C₁₆)-Aryloxy-(C₁-C₁₀)-alkyl)carbamoyl, N-((C₇-C₁₆)-Aralkyloxy-(C₁-C₁₀)alkyl)carbamoyl,
N-(C₁-C₁₀)-Alkyl-N-((C₁-C₁₀)-alkoxy-(C₁-C₁₀)-alkyl)carbamoyl, N-(C₁-C₁₀)-Alkyl-N-((C₆-C₁₂)-aryloxy-(C₁-C₁₀)-alkyl)carbamoyl, N-(C₁-C₁₀)-Alky-N-((C₇-C₁₆)-aralkyloxy-(C₁-C₁₀)-alkyl)carbamoyl, CON(CH₂)ₕ worin eine CH₂-Gruppe durch O, S, N-(C₁-C₈)-Alkylimino, N-(C₃-C₈)-Cycloalkylimino, N-(C₃-C₈)-Cycloalkyl-(C₁-C₄)-alkylimino, N-(C₆-C₁₂)-Arylimino, N-(C₇-C₁₆)-Aralkylimino oder N-(C₁-C₄)-Alkoxy-(C₁-C₆)-alkylimino ersetzt ein kann und h 3 bis 6 bedeutet,
Amino, (C₁-C₁₂)-Alkylamino, Di-(C₁-C₁₂)alkylamino, (C₃-C₈)-Cycloalkylamino, (C₃-C₁₂)-Alkenylamino, (C₃-C₁₂) Alkinylamino, N-(C₆-C₁₂)-Arylamino, N-(C₇-C₁₁)-Aralkylamino, N-Alkyl-Aralkylamino, N-Alkyl-Arylamino, (C₁-C₁₂)-Alkoxyamino, (C₁-C₁₂)-Alkoxy-N-(C₁-C₁₀)-alkylamino,
(C₁-C₁₂)-Alkanoylamino, (C₃-C₈)-Cycloalkanoylamino,
(C₆-C₁₂)-Aroylamino, (C₇-C₁₆)-Aralkanoylamino, (C₁-C₁₂)-Alkanoyl-N-(C₁-C₁₀)-alkylamino, (C₃-C₈)-Cycloalkanoyl-N-(C₁-C₁₀)-alkylamino, (C₆-C₁₂)-Aroyl-N-(C₁-C₁₀)alkylamino, (C₇-C₁₁)-Aralkanoyl-N-(C₁-C₁₀)-alkylamino,
(C₁-C₁₂)-Alkanoylamino-(C₁-C₈)-alkyl, (C₃-C₈)-Cycloalkanoylamino-(C₁-C₈)-alkyl, (C₆-C₁₂)-Aroylamino-(C₁-C₈)-alkyl, (C₇-C₁₆)-Aralkanoylamino-(C₁-C₈)-alkyl, Amino-(C₁-C₁₀)-alkyl, N-(C₁-C₁₀)-alkylamino-(C₁-C₁₀)alkyl, N,N-Di-(C₁-C₁₀)-alkylamino-(C₁-C₁₀)alkyl, (C₃-C₈)-Cycloalkylamino-(C₁-C₁₀)alkyl,
(C₁-C₁₂)-Alkylmercapto, (C₁-C₁₂)-Alkylsulfinyl, (C₁-C₁₂)-Alkylsulfonyl, (C₆-C₁₆)-Arylmercapto, (C₆-C₁₆)-Arylsulfinyl, (C₆-C₁₆)-Arylsulfonyl, (C₇-C₁₆)-Aralkylmercapto, (C₇-C₁₆)-Aralkylsulfinyl, (C₇-C₁₆)-Aralkylsulfonyl,
R⁴ einen verzweigten oder unverzweigten (C₇-C₂₀)-Alkylrest, der bis zu 3 C-C-Mehrfachbindungen enthalten kann, oder (C₁-C₁₂)-Alkoxy-(C₁-C₈)-alkyl, (C₁-C₁₂)-Alkoxy-(C₁-C₈)-alkoxy-(C₁-C₈)-alkyl, [CH₂]ₓ C_{f}H_{(2f+1-g)}F_{g}, oder (C₆-C₁₂)-Aryl, (C₇-C₁₁)-Aralkyl, Heteroaryl, Heteroaralkyl, (C₃-C₈)-Cycloalkyl, (C₃-C₈)-Cycloalkyl-(C₁-C₆)-alkyl, (C₆-C₁₄)-Aryloxy-(C₁-C₈)-alkyl, Heteroaryloxy-(C₁-C₈)-alkyl, (C₃-C₈)-Cycloalkyloxy-(C₁-C₈)-alkyl,(C₇-C₁₆)-Aralkyloxy-(C₁-C₈)-alkyl, Heteroaralkyloxy-(C₁-C₈)-alkyl,
(C₆-C₁₄)-Aryloxy-(C₁-C₈)-alkoxy-(C₁-C₈)-alkyl, Heteroalkyl-(C₁-C₈)-alkoxy-(C₁-C₈)-alkyl, (C₃-C₈)-Cycloalkoxy-(C₁-C₈)-alkoxy-(C₁-C₆)-alkyl,
(C₇-C₁₆)-Aralkyloxy-(C₁-C₈)-alkoxy-(C₁-C₈)alkyl, Heteroaralkyloxy-(C₁-C₈)-alkoxy-(C₁-C₈)-alkyl, (C₃-C₈)-Cycloalkyl-(C₁-C₆)-alkoxy-(C₁-C₆)-alkyl bedeutet, oder
einen Rest der Formel Z bedeutet,
-[CH₂]ᵥ-[O]_{w}-[CH₂]ₜ-E (Z),
worin E einen substituierten Phenylrest der Formel F 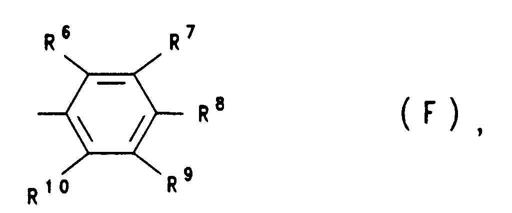 oder einen substituierten Heteroaryl-Rest, oder einen substituierten (C₃-C₈)-Cycloalkylrest bedeutet und
wobei
v = 0, 1, 2, 3, 4, 5, 6, w = 0, 1 und t = 0, 1, 2, 3, mit der Einschränkung, daß v ungleich 0 ist, falls w = 1 ist, bedeutet und R⁶, R⁷, R⁸, R⁹ und R¹⁰ gleich oder verschieden sind und Wasserstoff, Halogen, Cyano, Nitro, Trifluormethyl, (C₁-C₆)-Alkyl, (C₃-C₈-Cycloalkyl, (C₁-C₆)-Alkoxy, -O-[CH₂₋]ₓC_{f}H_{(2f+1-g)}F_{g}, -OCF₂Cl, -O-CF₂-CHFCl, (C₁-C₆)-Alkylmercapto, (C₁-C₆)-Hydroxyalkyl, (C₁-C₆)-Alkoxy-(C₁-C₆)-alkoxy, (C₁-C₆)-Alkoxy-(C₁-C₆)-alkyl, (C₁-C₆)-Alkylsulfinyl, (C₁-C₆)-Alkylsulfonyl, (C₁-C₆)-Alkylcarbonyl, (C₁-C₈)-Alkoxycarbonyl, Carbamoyl, N-(C₁-C₈)-Alkylcarbamoyl, N,N-Di-(C₁-C₈)-alkylcarbamoyl, gegebenenfalls mit Fluor, Chlor, Brom, Trifluormethyl und (C₁-C₆)-Alkoxy-substituiertes (C₇-C₁₁)-Aralkylcarbamoyl, N-(C₃-C₈)-Cycloalkylcarbamoyl, N-(C₃-C₈)-Cycloalkyl-(C₁-C₄)-alkylcarbamoyl, (C₁-C₆)-Alkylcarbonyloxy, Phenyl, Benzyl, Phenoxy, Benzyloxy, NR'R'', wie Amino, Anilino, N-Methylanilino, Phenylmercapto, Phenylsulfonyl, Phenylsulfinyl, Sulfamoyl, N-(C₁-C₈)-Alkylsulfamoyl oder N,N-Di-(C₁-C₈)-alkylsulfamoyl bedeuten, oder zwei benachbarte Substituenten gemeinsam eine Kette -[CH₂₋]ₙ oder -CH=CH-CH=CH- bedeuten, wobei eine CH₂-Gruppe der Kette gegebenenfalls durch O, S, SO, SO₂ oder NR' ersetzt ist, wobei
R' und R'' gemeinsam für -[CH₂]ₕ₋ stehen, worin eine CH₂-Gruppe durch O, S, N-(C₁-C₄)-Alkanoylimino oder N-(C₁-C₄)-Alkoxycarbonylimino ersetzt sein kann, und
f = 1 bis 8,
g = 0, 1 bis (2f+1),
h = 3 bis 6,
x = 0 bis 3, und
n = 3 oder 4 ist.

3. Verbindungen der Formel 1 nach den Ansprüchen 1 oder 2, in der
Q = O,
X = O,
Y = CR³,
m = O,
A -CHR^{x}-, wobei R^{x} den Substituenten des α-C-Atoms einer α-Aminosäure bedeutet, insbesondere einer natürlichen L-Aminosäure oder ihr D-Isomeres,
B = CO₂V bedeutet, wobei
V den Rest eines Alkohols VOH bedeutet und für einen verzweigten, unverzweigten oder cyclischen aliphatischen (C₁-C₉)-Alkyl-Rest, einen (C₃-C₈)-Cycloalkyl-(C₁-C₄)-alkylrest,
einen verzweigten oder unverzweigten (C₂-C₈)-Alkenylrest oder (C₂-C₈)-Alkinylrest, einen Phenyl-, Benzyl-, Phenethyl-, Phenylpropyl-oder Phenylbutylrest steht,
wobei die vorstehenden Reste einen Substituenten aus der Reihe Fluor, Chlor, Hydroxy, (C₁-C₄)-Alkoxy, (C₁-C₆)-Alkylcarbonyloxy, (C₃-C₈)-Cycloalkylcarbonyloxy, Benzoyloxy oder Phenylalkylcarbonyloxy enthalten,
R¹ und R³ Wasserstoff bedeuten,
R² einen Rest -CONR⁵R⁶ bedeutet, wobei
R⁵ Wasserstoff, (C₁-C₃)-Alkyl, (C₁-C₄)-Alkanoyl oder ein 1, 2 oder 3-wertiges physiologisch verwendbares Kation, insbesondere Na⊕, K⊕, Mg²⊕, Ca²⊕ oder ein Ammoniumion bedeutet, ggf. 1-3fach substituiert mit (C₁-C₈)-Hydroxyalkyl, (C₁-C₄)-Alkoxy-(C₁-C₈)-alkyl, Phenyl, Benzyl oder (C₁-C₈)-Alkyl, welches seinerseits 1-3fach substituiert sein kann mit Hydroxy und/oder (C₁-C₄)-Alkoxy,
R⁶ einen Rest der Formel I, ausgenommen - SO₂H, bedeutet,
-G-K-[C-U]ᵣ-D-W (I)
in welchem
G -SO₂- bedeutet,
K eine Bindung,
C eine Bindung oder
einen (C₁-C₆)-Alkandiyl-Rest bedeutet,
U eine Bindung, Wasserstoff oder -O- bedeutet,
r 1 ist,
D eine Bindung, Wasserstoff oder
einen unverzweigten aliphatischen (C₁-C₈)-Alkandiyl-Rest bedeutet, und
W eine Bindung, Wasserstoff, einen (C₆-C₁₂)-Arylrest oder einen 5- oder 6-gliedrigen Heteroarylrest bedeutet, wobei mindestens eine der Variablen C oder D oder W nicht eine Bindung bedeuten und U nur dann in der Bedeutung einer Heteratomgruppierung steht, wenn C nicht eine Bindung bedeutet oder wenn D und/oder W nicht eine Bindung bedeuten und
C, D und/oder W, sofern diese keine Bindung oder Wasserstoff bedeuten, vorzugsweise ihrerseits substituiert sind mit bis zu 3 gleichen oder verschiedenen Substituenten aus der Reihe
Hydroxy, Halogen, Cyano, Trifluormethyl, Nitro, Carboxy, (C₁-C₁₂)-Alkyl, (C₃-C₈)-Cycloalkyl, (C₃-C₈)-Cycloalkyl-(C₁-C₁₂)-alkyl, (C₃-C₈)-Cycloalkoxy, (C₃-C₈)-Cycloalkyl-(C₁-C₁₂)-alkoxy, (C₃-C₈)-Cycloalkyloxy-(C₁-C₁₂)-alkyl, (C₃-C₈)-Cycloalkyloxy-(C₁-C₁₂)-alkoxy, (C₃-C₈)-Cycloalkyl-(C₁-C₈)-alkyl-(C₁-C₆)-alkoxy, (C₃-C₈)-Cycloalkyl-(C₁-C₈)-alkoxy-(C₁-C₆)-alkyl, (C₃-C₈)-Cycloalkyloxy-(C₁-C₈)-alkoxy-(C₁-C₆)-alkyl, (C₃-C₈)-Cycloalkoxy-(C₁-C₈)-alkoxy-(C₁-C₈)-alkoxy, (C₆-C₁₂)-Aryl, (C₇-C₁₆)-Aralkyl, (C₂-C₁₂)-Alkenyl, (C₂-C₁₂)-Alkinyl, (C₁-C₁₂)-Alkoxy, (C₁-C₁₂)-Alkoxy-(C₁-C₁₂)alkyl, (C₁-C₁₂)-Alkoxy-(C₁-C₁₂)alkoxy, (C₁-C₁₂)-Alkoxy-(C₁-C₈)-alkoxy-(C₁-C₈)-alkyl, (C₆-C₁₂)-Aryloxy, (C₇-C₁₆)-Aralkyloxy, (C₆-C₁₂)-Aryloxy-(C₁-C₆)-alkoxy, (C₇-C₁₆)-Aralkoxy-(C₁-C₆)-alkoxy, (C₁-C₈)-Hydroxyalkyl, (C₆-C₁₆)-Aryloxy-(C₁-C₈)-alkyl, (C₇-C₁₆)-Aralkoxy-(C₁-C₈)-alkyl, (C₆-C₁₂)-Aryloxy-(C₁-C₈)-alkoxy-(C₁-C₆)-alkyl, (C₇-C₁₂)-Aralkyloxy-(C₁-C₈)-alkoxy-(C₁-C₆)-alkyl, -O-[CH₂₋]ₓC_{f}H_{(2f+1-g)}F_{g},
(C₁-C₁₂)-Alkoxycarbonyl, (C₁-C₁₂)-Alkoxy-(C₁-C₁₂)-alkoxycarbonyl, (C₆-C₁₂)- Aryloxycarbonyl, (C₇-C₁₆)-Aralkoxycarbonyl, (C₃-C₈)-Cycloalkoxycarbonyl,
(C₁-C₁₂)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy,
Carbamoyl, N-(C₁-C₁₂)-Alkylcarbamoyl, N, N-Di-(C₁-C₁₂)-alkylcarbamoyl, N-(C₃-C₈)-Cycloalkylcarbamoyl, N,N-Dicyclo-(C₃-C₈)-alkylcarbamoyl, N-(C₁-C₁₀)-Alkyl-N-(C₃-C₈)-cycloalkylcarbamoyl, N-((C₃-C₈)-Cycloalkyl-(C₁-C₆)-alkyl)carbamoyl, N-(C₁-C₆)-Alkyl-N-((C₃-C₈)-cycloalkyl-(C₁-C₆)-alkyl)carbamoyl, N-(+)-Dehydroabietylcarbamoyl, N-(C₁-C₆)-Alkyl-N-(+)-dehydroabietylcarbamoyl, N-(C₆-C₁₂)-Arylcarbamoyl, N-(C₇-C₁₆)-Aralkylcarbamoyl, N-(C₁-C₁₀)-Alkyl-N-(C₆-C₁₆)-arylcarbamoyl, N-(C₁-C₁₀)-Alkyl-N-(C₇-C₁₆)-aralkylcarbamoyl, N-((C₁-C₁₀)-Alkoxy-(C₁-C₁₀)alkyl)carbamoyl, N-((C₆-C₁₆)-Aryloxy-(C₁-C₁₀)alkyl)carbamoyl, N-((C₇-C₁₆)-Aralkyloxy-(C₁-C₁₀)-alkyl)carbamoyl, N-(C₁-C₁₀)-Alkyl-N-((C₁-C₁₀)-alkoxy-(C₁-C₁₀)-alkyl)carbamoyl, N-(C₁-C₁₀)-Alkyl-N-((C₆-C₁₂)-aryloxy-(C₁-C₁₀)-alkyl)carbamoyl, N-(C₁-C₁₀)-Alkyl-N-((C₇-C₁₆)-aralkyloxy-(C₁-C₁₀)-alkyl)carbamoyl, CON(CH₂)ₕ, worin eine CH₂-Gruppe durch O, S, N-(C₁-C₈)Alkylimino, N-(C₃-C₈)-Cycloalkylimino, N-(C₃-C₈)-Cycloalkyl-(C₁-C₄)-alkylimino, N-(C₆-C₁₂)-Arylimino, N-(C₇-C₁₆)-Aralkylimino oder N-(C₁-C₄)-Alkoxy-(C₁-C₆)-alkylimino ersetzt ein kann und h 3 bis 7 bedeutet,
einem Carbamoyl-Rest der allgemeinen Formel II 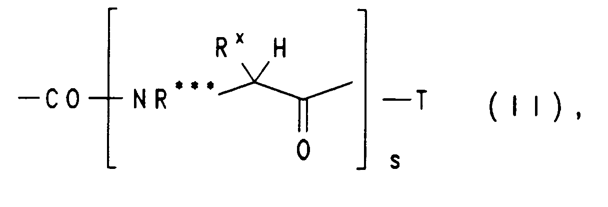 worin
R^{x} der Substituent einer α-Aminosäure bedeutet, zu denen die L-und D-Aminosäuren zählen,
s 1, 2, 3, 4 oder 5 und
T OH, OR oder NR*R** bedeutet, wobei
R*** Wasserstoff oder (C₁-C₄)-Alkyl,
R* und R** gleich oder verschieden sind und Wasserstoff, Phenyl, Benzyl Phenethyl, (C₁-C₈)-Alkyl, (C₃-C₆)-Cycloalkyl, (+)-Dehydroabietyl, (C₁-C₈)-Alkoxy-(C₁-C₈)-alkyl, Phenyl-(C₁-C₆)-alkoxy-(C₁-C₈)-alkyl, (C₆-C₁₂)-Phenoxy-(C₁-C₈)-alkyl bedeuten, oder
R* und R** gemeinsam für -[CH₂]ₕ stehen, worin eine CH₂ Gruppe durch O, S, SO, SO₂, N-Acylimino, N-(C₁-C₁₀)-Alkoxycarbonylimino, N-(C₁-C₈)-Alkylimino, N-(C₃-C₈)-Cycloalkylimino, N-(C₃-C₈)-Cycloalkyl-(C₁-C₄)-alkylimino, N-Phenylimino, N-Benzylimino oder N-(C₁-C₄)-Alkoxy-(C₁-C₆)-alkylimino ersetzt sein kann und h 3 bis 7 bedeutet, oder mit
Amino, (C₁-C₁₂)-Alkylamino, Di-(C₁-C₁₂)-alkylamino, (C₃-C₈)-Cycloalkylamino, N-(C₆-C₁₂)-Arylamino, N-(C₇-C₁₁)-Aralkylamino,
(C₁-C₁₂)-Alkanoylamino, (C₃-C₈)-Cycloalkanoylamino, (C₆-C₁₂)-Aroylamino, (C₇-C₁₆)-Aralkanoylamino, (C₁-C₁₂)-Alkanoyl-N-(C₁-C₁₀)-alkylamino, (C₃-C₈)-Cycloalkanoyl-N-(C₁-C₁₀)-alkylamino, (C₆-C₁₂)-Aroyl-N-(C₁-C₁₀)-alkylamino, (C₇-C₁₁)-Aralkanoyl-N-(C₁-C₁₀)-alkylamino,
(C₁-C₁₂)-Alkanoylamino-(C₁-C₆)-alkyl, (C₃-C₈)-Cycloalkanoylamino-(C₁-C₈)-alkyl, (C₆-C₁₂)-Aroylamino-(C₁-C₆)-alkyl, (C₇-C₁₆)-Aralkanoylamino-(C₁-C₆)-alkyl, Amino-(C₁-C₁₀)-alkyl,
N-(C₁-C₁₀)-alkylamino-(C₁-C₁₀)alkyl, N,N-Di(C₁-C₁₀)-alkylamino-(C₁-C₁₀)-alkyl, (C₃-C₈)-Cycloalkylamino-(C₁-C₁₀)-alkyl,
(C₁-C₁₂)-Alkylmercapto, (C₁-C₁₂)-Alkylsulfinyl, (C₁-C₁₂)-Alkylsulfonyl, (C₆-C₁₂)-Arylmercapto, (C₆-C₁₂)-Arylsulfinyl,
(C₆-C₁₂)-Arylsulfonyl, (C₇-C₁₂)-Aralkylmercapto, (C₇-C₁₂)-Aralkylsulfinyl, (C₇-C₁₂)-Aralkylsulfonyl,
wobei die Reste, die einen Arylrest enthalten ihrerseits am Aryl substituiert sein können durch 1 bis 5 gleiche oder verschiedene Reste aus der Reihe:
Hydroxy, Halogen, Cyano, Trifluormethyl, Nitro, Carboxyl, (C₁-C₈)-Alkyl, (C₃-C₈)-Cycloalkyl, (C₆-C₁₂)-Aryl, (C₇-C₁₆)-Aralkyl, (C₂-C₁₂)-Alkenyl, (C₂-C₁₂)-Alkinyl, (C₁-C₈)-Alkoxy, (C₁-C₆)-Alkoxy-(C₁-C₆)-alkyl, (C₆-C₁₂)-Aryloxy, (C₇-C₁₆)-Aralkyloxy, -O-[CH₂₋]_{X}C_{f}H_{(2f+1-g)}F_{g},
(C₁-C₁₂)-Alkoxycarbonyl, (C₃-C₈)-Cycloalkoxycarbonyl,
(C₁-C₁₂)-Alkylcarbonyloxy, (C₃-C₈)-Cycloalkylcarbonyloxy,
Carbamoyl, N-(C₁-C₁₂)-Alkylcarbamoyl, N, N-Di-(C₁-C₁₂)-alkylcarbamoyl, N-(C₃-C₈)-Cycloalkylcarbamoyl, N,N-Dicyclo-(C₃-C₈)-alkylcarbamoyl, N-(C₁-C₁₀)-Alkyl-N-(C₃-C₈)-cycloalkylcarbamoyl, N-((C₃-C₈)-Cycloalkyl-(C₁-C₆)-alkyl)carbamoyl, N-(C₁-C₆)-Alkyl-N-((C₃-C₈)-cycloalkyl-(C₁-C₆)-alkyl)carbamoyl, N-(+)-Dehydroabietylcarbamoyl, N-(C₁-C₆)-Alkyl-N-(+)-dehydroabietylcarbamoyl, N-(C₆-C₁₂)-Arylcarbamoyl, N-(C₇-C₁₆)-Aralkylcarbamoyl, N-(C₁-C₁₀)-Alkyl-N-(C₆-C₁₆)-arylcarbamoyl, N-(C₁-C₁₀)-Alkyl-N-(C₇-C₁₆)-aralkylcarbamoyl, N-((C₁-C₁₀)-Alkoxy-(C₁-C₁₀)-alkyl)carbamoyl, N-((C₆-C₁₆)-Aryloxy-(C₁-C₁₀)-alkyl)carbamoyl, N-((C₇-C₁₆)-Aralkyloxy-(C₁-C₁₀)-alkyl)carbamoyl,
N-(C₁-C₁₀)-Alkyl-N-((C₁-C₁₀)-alkoxy-(C₁-C₁₀)-alkyl)carbamoyl, N-(C₁-C₁₀)-Alkyl-N-((C₆-C₁₂)-aryloxy-(C₁-C₁₀)-alkyl)carbamoyl, N-(C₁-C₁₀)-Alkyl-N-((C₇-C₁₆)-aralkyloxy-(C₁-C₁₀)-alkyl)carbamoyl, CON(CH₂)ₕ worin eine CH₂-Gruppe durch O, S, N-(C₁-C₈)-Alkylimino, N-(C₃-C₈)-Cycloalkylimino, N-(C₃-C₈)-Cycloalkyl-(C₁-C₄)-alkylimino, N-(C₆-C₁₂)-Arylimino, N-(C₇-C₁₆)-Aralkylimino oder N-(C₁-C₄)-Alkoxy-(C₁-C₆)-alkylimino ersetzt ein kann und h 3 bis 7 bedeutet,
Amino, (C₁-C₆)-Alkylamino, Di-(C₁-C₆)-alkylamino, (C₃-C₈)-Cycloalkylamino, N-(C₆-C₁₂)-Arylamino, N-(C₇-C₁₁)-Aralkylamino, N-(C₁-C₃)-Alkyl-(C₇-C₁₁)-aralkylamino, N-(C₁-C₃)-Alkyl-(C₆-C₁₂)-arylamino, (C₁-C₈)-Alkoxyamino,
(C₁-C₈)-Alkanoylamino, (C₃-C₈)-Cycloalkanoylamino, (C₆-C₁₂)-Aroylamino, (C₇-C₁₂)-Aralkanoylamino, (C₁-C₈)-Alkanoyl-N-(C₁-C₆)-alkylamino, (C₃-C₈)-Cycloalkanoyl-N-(C₁-C₆)-alkylamino, (C₆-C₁₂)-Aroyl-N-(C₁-C₁₀)-alkylamino, (C₇-C₁₁)-Aralkanoyl-N-(C₁-C₆)-alkylamino,
(C₁-C₈)-Alkanoylamino-(C₁-C₄)-alkyl, (C₃-C₈)-Cycloalkanoylamino-(C₁-C₄)-alkyl, (C₆-C₁₂)-Aroylamino-(C₁-C₄)-alkyl und (C₇-C₁₂)-Aralkanoylamino-(C₁-C₄)-alkyl,
R⁴ einen verzweigten oder unverzweigten (C₇-C₂₀)-Alkylrest, der eine oder zwei C-C-Mehrfachbindungen enthalten kann, oder
(C₁-C₈)-Alkoxy-(C₁-C₆)-alkyl, (C₁-C₆)-Alkoxy-(C₁-C₄)-alkoxy-(C₁-C₄)-alkyl oder einen Rest der Formel Z bedeutet,
-[CH₂]ᵥ-[O]_{w}-[CH₂]ₜ-E (Z),
wobei E einen substituierten Phenylrest der Formel F 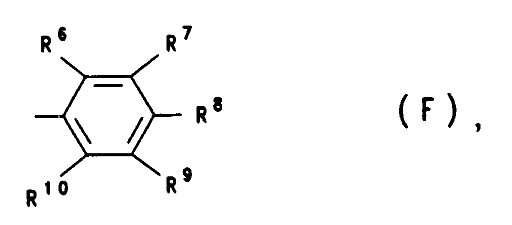 oder einen (C₃-C₈)-Cycloalkylrest bedeutet, wobei
v = 0, 1, 2, 3, w = 0, 1 und t = 0, 1 sein kann, mit der Einschränkung, daß v ungleich 0 ist, falls w = 1 ist, und
worin R⁶, R⁷, R⁸, R⁹ und R¹⁰ gleich oder verschieden sind und Wasserstoff, Fluor, Chlor, Cyano, Trifluormethyl, (C₁-C₆)-Alkyl, (C₁-C₆)-Alkoxy, -O-[CH₂₋]ₓC_{f}H_{(2f+1-g)}F_{g}, N-(C₁-C₈)-Alkylcarbamoyl, N,N-Di-(C₁-C₈)-alkylcarbamoyl, N-(C₃-C₈)-Cycloalkylcarbamoyl, gegebenenfalls mit Fluor, Chlor, Trifluormethyl und (C₁-C₆)-Alkoxy substituiertes (C₇-C₁₁)-Phenylalkylcarbamoyl bedeuten.

4. Verbindungen der Formel 1 nach einem oder mehreren der Ansprüche 1 bis 3, in der
Q = O,
X = O,
Y = CR³,
m = O,
A -CHR^{x}-, wobei R^{x} den Substituenten des α-C-Atoms einer α-Aminosäure bedeutet, insbesondere Wasserstoff
B = CO₂V bedeutet, wobei
V den Rest eines Alkohols VOH bedeutet und für einen verzweigten oder unverzweigten oder cyclischen aliphatischen (C₁-C₉)-Alkyl-Rest, einen (C₅-C₆)-Cycloalkyl-(C₁-C₄)-alkylrest,
einen verzweigten oder unverzweigten (C₂-C₈)-Alkenylrest oder einen Phenyl-, Benzyl-, Phenethyl-, Phenylpropyl-oder Phenylbutylrest steht,
wobei die vorstehenden Reste einen Substituenten aus der Reihe Hydroxy, (C₁-C₄)-Alkoxy, (C₁-C₆)-Alkylcarbonyloxy, (C₅-C₆)-Cycloalkylcarbonyloxy, Benzoyloxy, Phenylalkylcarbonyloxy enthalten,
R¹ und R³ Wasserstoff,
R² einen Rest -CONR⁵R⁶ bedeutet, wobei
R⁵ Wasserstoff oder ein 1, 2 oder 3-wertiges physiologisch verwendbares Kation bedeutet, insbesondere Na⊕, K⊕, Mg²⊕, Ca²⊕ oder H₃N⊕C(CH₂OH)₃ (Tris-Salz),
R⁶ einen Rest der Formel I, ausgenommen - SO₂H, bedeutet
-G-K-[C-U]ᵣ-D-W (I)
in welchem
G -SO₂- bedeutet,
K eine Bindung,
C eine Bindung,
U eine Bindung,
r 1 ist,
D eine Bindung oder
(C₁-C₄)-Alkandiyl,
W Wasserstoff oder
einen Phenylrest bedeutet,
wobei dieser durch 1, 2, 3, 4 oder 5 Substituenten aus der Reihe Fluor, Chlor, Nitril, Trifluormethyl, (C₁-C₆)-Alkyl, Phenyl, (C₁-C₆)-Alkoxy, Phenoxy, -O-[CH₂₋]_{X}C_{f}H_{(2f+1-g)}F_{g},
Carbamoyl, N-(C₁-C₈)-Alkylcarbamoyl, N, N-Di-(C₁-C₈)-alkylcarbamoyl, N-(C₃-C₆)-Cycloalkylcarbamoyl, N-(C₁-C₄)-Alkyl-N-(C₃-C₆)-cycloalkylcarbamoyl, N-((C₃-C₆)-Cycloalkyl-(C₁-C₄)-alkyl)carbamoyl, N-(C₁-C₆)-Alkyl-N-((C₃-C₆)-cycloalkyl-(C₁-C₄)-alkyl)carbamoyl, N-(+)-Dehydroabietylcarbamoyl, N-(C₆-C₁₂)-Phenylcarbamoyl, N-Phenyl-(C₁-C₄)-alkylcarbamoyl, N-(C₁-C₆)-Alkyl-N-phenylcarbamoyl, N-(C₁-C₆)-Alkyl-N-phenyl-(C₁-C₄)-alkylcarbamoyl, N-((C₁-C₆)-Alkoxy-(C₁-C₆)-alkyl)carbamoyl, N-Phenoxy-(C₁-C₆)-alkyl)carbamoyl, CON(CH₂)ₕ, worin eine CH₂-Gruppe durch O, N-(C₁-C₆)-Alkylimino, ersetzt ein kann und h 3 bis 5 bedeutet, oder
einen Carbamoyl-Rest der allgemeinen Formel II 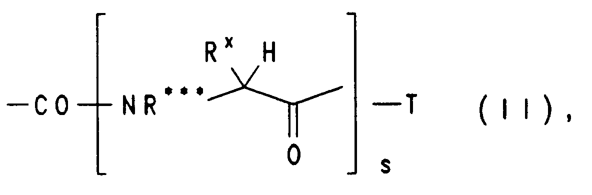 worin
R^{x} der Substituent einer α-Aminosäure bedeutet, zu denen die L-und D-Aminosäuren zählen,
s 1, 2, 3, 4 oder 5 und
T OH, OR oder NR*R** bedeutet, wobei
R*** Wasserstoff oder (C₁-C₄)-Alkyl,
R* und R** gleich oder verschieden sind und Wasserstoff, Phenyl, Benzyl Phenethyl, (C₁-C₈)-Alkyl, (C₃-C₆)-Cycloalkyl, (+)-Dehydroabietyl, (C₁-C₈)-Alkoxy-(C₁-C₈)-alkyl, (C₇-C₁₂)-Phenylalkoxy-(C₁-C₈)-alkyl, (C₆-C₁₂)-Phenoxy-(C₁-C₈)-alkyl bedeuten, oder
R* und R** gemeinsam für -[CH₂]ₕ stehen, worin eine CH₂ Gruppe durch O, S, SO, SO₂, N-Acylimino, N-(C₁-C₁₀)-Alkoxycarbonylimino, N-(C₁-C₈)-Alkylimino, N-(C₃-C₆)-Cycloalkylimino, N-(C₃-C₆)-Cycloalkyl-(C₁-C₄)-alkylimino, N-Phenylimino, N-Benzylimino oder N-(C₁-C₄)-Alkoxy-(C₁-C₆)-alkylimino ersetzt sein kann und h 3 bis 5 bedeutet,
(C₁-C₈)-Alkanoylamino, (C₃-C₈)-Cycloalkanoylamino, (C₆-C₁₂)-Phenyl-amino, (C₇-C₁₁)-Phenylalkanoylamino, (C₁-C₈)-Alkanoyl-N-(C₁-C₁₀)-alkylamino, (C₃-C₆)-Cycloalkanoyl-N-(C₁-C₆)-alkylamino, Benzoyl-N-(C₁-C₁₀)-alkylamino, (C₇-C₁₁)-Phenylalkanoyl-N-(C₁-C₆)-alkylamino,
(C₁-C₁₀)-Alkanoylamino-(C₁-C₂)-alkyl, (C₃-C₈)-Cycloalkanoylamino-(C₁-C₂)-alkyl, Benzoylamino-(C₁-C₂)-alkyl, (C₇-C₁₄)-Phenylalkanoylamino-(C₁-C₂)-alkyl substituiert ist und wobei die Reste, die einen Arylrest enthalten, ihrerseits mit einem Substituenten aus der Reihe Wasserstoff, Hydroxy, Fluor, Chlor, Trifluormethyl, (C₁-C₆)-Alkyl, (C₁-C₈)-Alkoxy substituiert sein können und
R⁴ einen verzweigten oder unverzweigten (C₇-C₁₂)-Alkylrest, der eine oder zwei C-C-Mehrfachbindungen enthalten kann, oder
(C₁-C₄)-Alkoxy-(C₁-C₆)-alkyl, (C₁-C₄)-Alkoxy-(C₁-C₄)-alkoxy-(C₁-C₄)alkyl oder einen Rest der Formel Z bedeutet,
-[CH₂]ᵥ-[O]_{w}-[CH₂]ₜ-E (Z),
wobei E einen substituierten Phenylrest der Formel F 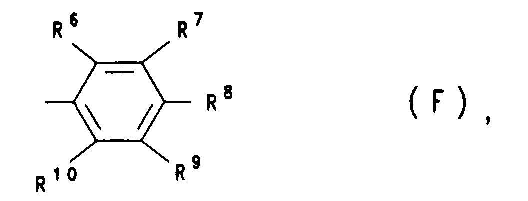 oder einen (C₃-C₈)-Cycloalkylrest bedeutet, wobei
v = 0, 1, 2 oder 3, w = 0 und t = 0 ist, und worin R⁶, R⁷, R⁸, R⁹ und R¹⁰ gleich oder verschieden sind und Wasserstoff, Fluor, Chlor, Cyano, Trifluormethyl, (C₁-C₆)-Alkyl, (C₁-C₆)-Alkoxy, -O-[CH₂₋]ₓC_{f}H_{(2f+1-g)}F_{g}, N-(C₁-C₈)-Alkylcarbamoyl, N,N-Di-(C₁-C₈)-alkylcarbamoyl, N-(C₃-C₈)-Cycloalkylcarbamoyl, gegebenenfalls mit Fluor, Chlor, Trifluormethyl und (C₁-C₆)-Alkoxy substituiertes (C₇-C₁₁)-Phenylalkylcarbamoyl bedeuten und
n 0,
f 1 bis 5,
g 0, 1 bis (2f+1) und
x 0 oder 1 bedeuten.

5. Verbindungen gemäß einem oder mehreren der Ansprüche 1 bis 4 zur Herstellung eines Arzneimittels gegen fibrotische Erkrankungen und/oder als Inhibitoren der Kollagenbiosynthese.

6. Verbindung gemäß einem oder mehreren der Ansprüche 1 bis 4 als Inhibitoren der Prolylhydroxylase.

7. Verbindungen gemaß einem oder mehreren der Ansprüche 1 bis 4 zur Verwendung als Arzneimittel.

8. Arzneimittel, enthaltend eine oder mehrere Verbindungen der allgemeinen Formel 1 gemäß einem oder mehreren der Ansprüche 1 bis 4.

9. Verfahren zur Herstellung von Verbindungen nach Formel I gemäß den Ansprüchen 1 bis 4, in der A einen substituierten Alkylen-Teil, B = CO₂H, Y = CR³ und m = 0 und 1 bedeuten, indem
i1.) Pyridin-2-carbonsäuren der Formel 9 (R¹¹ = H) mit den Aminoestern der Formel 10 zu den Amidestern der Formel 1 umgesetzt werden, oder
i2.) Pyridin-2-carbonsäureester der Formel 9 (R¹¹ = niedrig Alkyl) unter den Bedingungen der Aminolyse zu den Verbindungen der Formel 1 umgesetzt werden; 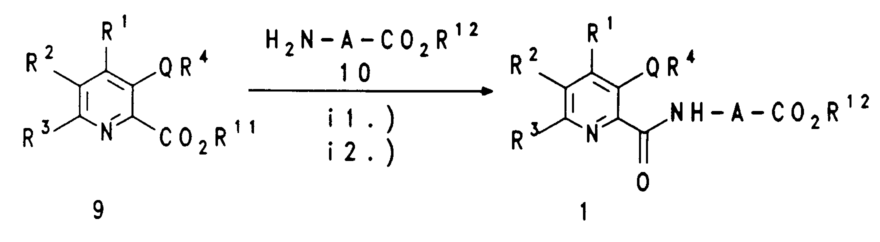 und
ii) die Verbindungen der Formel 1 durch Alkylierung von Verbindungen der Formel 11 mit R⁴X hergestellt werden 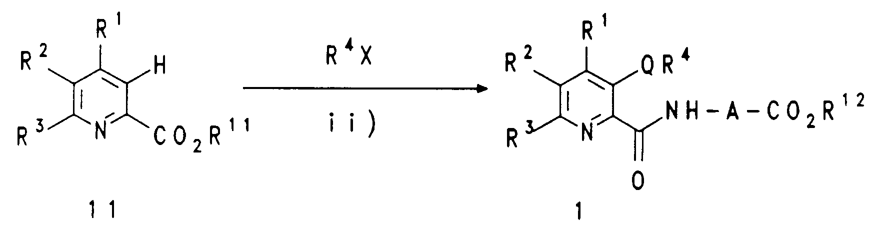 wobei X für eine Abgangsgruppe, insbesondere für Halogen, OSO₂Me oder OSO₂ Phenyl, steht und ggf.
iii) die Verbindungen der Formel 1, sofern Q = O und NR^{y} gilt, in ihre Pyridin-N-oxide (1') 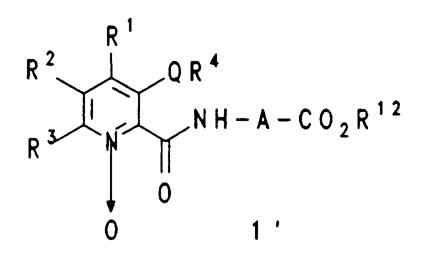 übergeführt werden.
